# EUROPEAN PATENT APPLICATION

(11) **EP 3 881 866 A1**
(43) Date of publication of application: **22.09.2021**
(21) Application number: 20163257.7
(22) Date of filing: 16.03.2020
(51) Int. Cl.: A61K 39/395

(54) **A TARGETING MODULE COMPRISING PD-L1 AND/OR PD-L2 FOR USE IN A METHOD FOR STIMULATING A CHIMERIC ANTIGEN RECEPTOR MEDIATED RESPONSE IN A MAMMAL**

(71) Applicant: GEMoaB GmbH, 01307 Dresden (DE)
(72) Inventor: Ehninger, Armin, 01307 Dresden (DE)
(74) Representative: Kailuweit & Uhlemann Patentanwälte Partnerschaft mbB

(57) **Abstract**

The present invention relates to a targeting module comprising at least one PD-L1 and/or PD-L2 binding domain, and a tag-binding domain or a tag for use in a method for stimulating a chimeric antigen receptor mediated immune response in a mammal, a nucleic acid, a vector or a cell comprising a nucleotide sequence encoding the targeting module, a pharmaceutical composition and a kit comprising the targeting module and a vector or a cell comprising a nucleotide sequence encoding a switchable chimeric antigen receptor.

## Description

The present invention relates to a targeting module comprising at least one PD-L1 and/or PD-L2 binding domain, and a tag-binding domain or a tag for use in a method for stimulating a chimeric antigen receptor mediated immune response in a mammal, a nucleic acid, a vector or a cell comprising a nucleotide sequence encoding the targeting module, a pharmaceutical composition and a kit comprising the targeting module and a vector or a cell comprising a nucleotide sequence encoding a switchable chimeric antigen receptor.

Chimeric antigen receptors (CARs) are artificial receptors consisting of a binding moiety, which provides the antigen-specificity and one or several signaling chains derived from immune receptors (Cartellieri et al. 2010). These two principal CAR domains are connected by a linking peptide chain including a transmembrane domain, which anchors the CAR in the cellular plasma membrane. Immune cells, in particular T and NK cells, can be genetically modified to express CARs inserted into their plasma membrane. If such a CAR-modified immune cell encounters other cells or tissue structures expressing or being decorated with the appropriate target of the CAR binding moiety, upon binding of the CAR binding moiety to the target antigen the CAR modified immune cell is cross-linked to the target. Cross-linking leads to an induction of signal pathways via the CAR signaling chains, which will change the biologic properties of the CAR-engrafted immune cell. For example, CAR triggering in effector CD4+ and CD8+ T cells will activate typical effector functions like secretion of lytic compounds and cytokines, which will eventually lead to the killing of the respective target cell. In contrast, CAR activation in gene-modified regulatory T cells (Tregs) leads to an activation of Treg-specific immunomodulatory and suppressive mechanism like interleukin (IL)-10 or tumor growth factor beta (TGF-β) secretion. The adoptive transfer of immune cells engineered with chimeric antigen receptors (CARs) is currently considered as a highly promising therapeutic option for treatment of otherwise incurable malignant, infectious or autoimmune diseases. Until today, two CAR-T cell therapeutics have gained market approval for treatment of B cell derived malignancies, proving the clinical feasibility of this approach.

However, the conventional CAR technology comes along with a number of critical issues, which need to be solved before this treatment modality can be widely applied for clinical treatments. First of all, several safety issues have to be addressed. So far, immune responses of T cells engineered with conventional CARs are difficult to control after infusion into the patient. Serious adverse event rates are high (Titov et al. 2018). Especially unexpected target gene expression on normal tissue may provoke a rapid and rigorous immune reaction of engineered T cells against normal cells, which can cause severe side effects (Lamers et al. 2006, Morgan et al. 2010). Moreover, as CAR-T cells are a new class of self-amplifying cell drugs, infused T cells can undergo a vigorous expansion in the presence of heavy tumor burden leading to tumor lysis syndrome, cytokine release syndrome and macrophage activation syndrome (Brudno and Kochenderfer 2016, Maude et al. 2014). Another drawback of conventional CAR technology is the restriction of engineered T cell retargeting to a single antigen. Such a monotherapeutic approach implies the risk for development of tumor escape variants, which have lost the target antigen during treatment. The emergence of tumor escape variants under conventional CAR T cell therapy after several months was already observed in clinical trials (Grupp et al. 2013, Sotillo et al. 2015). Taken together, these obstacles restrict the application of CAR-T cells to very few indications. In fact, examples of clinical effectiveness have been restricted to CD19- and BCMA-targeting CAR-T cells until now.

Modular "universal" CAR-T (UniCAR) approaches can overcome these limitations by separating antigen-recognition and activating domain of a CAR into two separate operational units. T cells are engineered to express a CAR with a universal binding domain recognizing a tag (Cartellieri et al. 2016). Antigen-specificity is provided by soluble adapter proteins, which consist of an antigen-binding domain fused to the tag recognized by the universal CAR.

WO 2012/082841 A2 discloses universal anti-tag chimeric antigen receptor-expressing T cells and methods of treating cell-related disorders, e.g. cancer. In addition, WO 2013/044225 A1 discloses a universal immune receptor expressed by T cells for the targeting of diverse and multiple antigens. Both methods describe the use of modified T cells expressing universal anti-tag immune receptors. These T cells can be redirected to disease-related cell surface antigens by additionally applying soluble modules binding these surface antigens and carrying the respective tag. WO 2016/030414 A1 provides a genetically modified immune cell that allows a redirection against diverse disorders in a safe and efficient manner using endogenous tags based on nuclear proteins.

Despite remarkable therapeutic success using chimeric antigen reœptor modified T cells (CAR-T) in hematologic malignancies, targeting solid tumors still remains challenging, given their heterogeneity and immunosuppressive milieu, including the expression of inhibitory immune checkpoints such as PD-L1 or PD-L2 by tumor cells. Preclinical models demonstrate that combining CAR-T therapy with checkpoint inhibitors targeting the PD-1/PD-L1 axis could be a promising strategy to enhance anti-cancer activity of CAR-T in solid tumors and on-going phase I trials are clinically evaluating this strategy (Grosser et al. 2019). However, primary and acquired resistance to checkpoint blockade is frequently observed limiting its broad applicability and efficacy. In addition, lack of specificity results in frequent occurrence of immune-related adverse events affecting several organs.

The object of the present invention is to provide an approach, which overcomes the disadvantages of the state of the art.

The object is solved by a targeting module comprising
i) at least one PD-L1 and/or PD-L2 binding domain,
ii) a tag-binding domain or a tag,
for use in a method for stimulating a chimeric antigen receptor mediated immune response in a mammal, preferably for use in the treatment of cancer, infectious disease or autoimmune disease; wherein the targeting module is administered in combination with a vector or a cell comprising a nucleotide sequence encoding a switchable chimeric antigen receptor,
wherein the switchable chimeric antigen receptor comprises three domains,
wherein
- the first domain is a tag-binding domain or a tag,
- the second domain is an extracellular hinge and a transmembrane domain and
- the third domain is a signal transduction domain,
wherein the tag-binding domain of the targeting module binds to the tag of the switchable chimeric antigen receptor or the tag of the targeting module binds to the tag-binding domain of the switchable chimeric antigen receptor.

Advantageously, the targeting module according to the invention used in combination with a switchable chimeric antigen receptor actively targets PD-L1 and/or PD-L2 expressed by tumor cells and is capable of inducing a significant anti-tumor response. Further advantageously, the pharmacokinetic and pharmacodynamic half-life of the targeting module according to the invention is short, preferably in the range of 0.4 to 5 h, providing a rapid and reversible switch off mechanism of the mediated immune response. Advantageously, the toxicity of the targeting module according to the invention is low, which is a result of the rapid switch off mechanism, as can be demonstrated by sparing PD-L1 low expressing cells when targeting module is not replenished in an in vitro experiment.

As used herein, the term "targeting module" refers to an isolated protein with at least two different domains, wherein each domain is specific for a target or a uniform group of targets, respectively, wherein at least one domain is specific for a target cell, in particular the PD-L1 and/or PD-L2 binding domain; and one domain is specific for a switchable chimeric antigen receptor, in particular the tag-binding domain or tag.

According to the invention, the targeting module comprises at least one PD-L1 binding domain or at least one PD-L2 binding domain or at least one PD-L1 binding domain and at least one PD-L2 binding domain.

As used herein "PD-L1" (Programmed death ligand 1) refers to a transmembrane protein according to SEQ ID No. 1 that in humans is encoded by the CD274 or PDCD1LG1 gene and is a ligand of PD-1. As used herein "PD-L2" (Programmed death 1 ligand 2) refers to a transmembrane protein according to SEQ ID No. 2 that in humans is encoded by the CD273 or PDCD1LG2 gene and is a ligand of PD-1.

As used herein, the term "domain" refers to a part of a protein sequence, which can exist and function independently from the rest of the protein.

As used herein, the term "specific" refers to the ability of an antibody or antibody fragment or a protein, peptide or low molecular weight organic ligand to recognize and bind with a binding partner (e.g. a tumor antigen) protein present in a sample, but not substantially recognize or bind other molecules in the sample.

As used herein, the term "binds" or "binding" refers to a non-covalent binding, in particular ionic bonds, hydrogen bonds, Van der Waals forces and/or hydrophobic interactions.

As used herein, the term "administered in combination" refers to a treatment, wherein the targeting module is administered prior to, simultaneously with and/or after the administration of the vector or cell comprising a nucleotide sequence encoding a switchable chimeric antigen receptor.

In embodiments, the targeting module is administered on its own, preferably one hour to 2 days, more preferably 4 to 24 hours, prior to the administration of the vector or cell comprising a nucleotide sequence encoding a switchable chimeric antigen receptor. Advantageously, the administration of the targeting module prior to the administration of the vector or cell comprising a nucleotide sequence encoding a switchable chimeric antigen receptor stimulates the switchable chimeric antigen receptors and increases the expansion of the respective effector cells and their accumulation at the target site.

In further embodiments, the targeting module is administered simultaneously with the vector or cell comprising a nucleotide sequence encoding a switchable chimeric antigen receptor.

In further embodiments, the targeting module is administered, preferably in the range of 3 days to 30 days, after the administration of the vector or cell comprising a nucleotide sequence encoding a switchable chimeric antigen receptor.

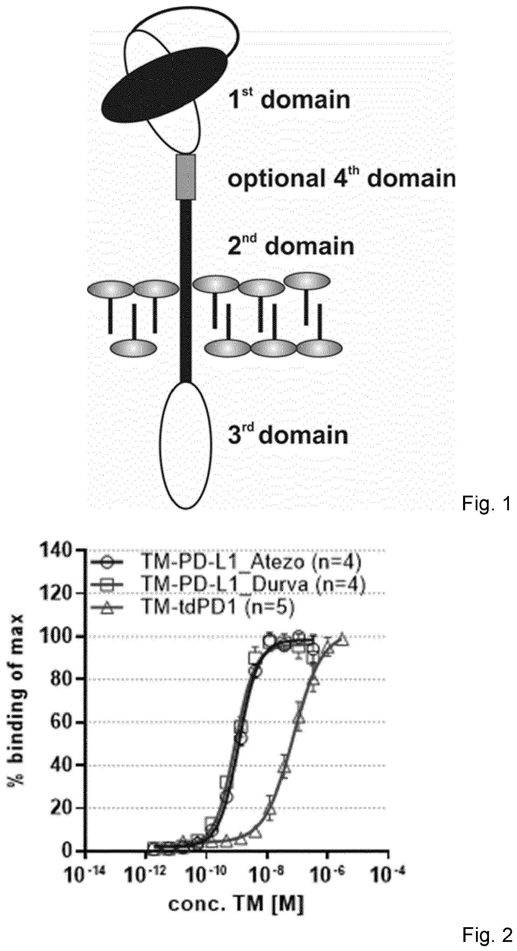

As used herein, the term "switchable chimeric antigen receptor" refers to an artificial chimeric fusion protein, in particular a receptor comprising a tag-binding domain or a tag, an extracellular hinge and a transmembrane domain and a signal transduction domain (Fig. 1). The domains can be derived from different sources and therefore, the receptor is called chimeric. Advantageously, the receptor can bind with the tag-binding domain or tag to different targeting modules.

Advantageously, the cell comprising a nucleotide sequence encoding a switchable chimeric antigen receptor (CAR) expresses the switchable CAR, which has binding specificity for the tag-binding domain or tag of the targeting module, which in turn binds to PD-L1 and/or PD-L2 on a target cell.

The term "autoimmune disorder" refers to an abnormal immune response of the body against substances and tissues normally present in the body (autoimmunity).

In embodiments, the targeting module is in monomeric or dimeric form, preferably in monomeric form.

In further embodiments, the targeting module is monovalent or bivalent.

In some embodiments, the targeting module according to the invention is bivalent and comprises one PD-L1 and one PD-L2 binding domain.

Preferably, the PD-L1 binding domain is an antibody or antibody fragment.
Preferably, the PD-L2 binding domain is an antibody or antibody fragment.

As used herein, the term "antibody" refers to a protein, which binds antigens via the Fab's variable region. The fragment antigen-binding (Fab) fragment is a region on an antibody that binds to antigens. It is composed of one constant and one variable domain of each of the heavy (V_{H}) and the light chain (V_{L}). As used herein, the term "antibody fragment" refers to a protein comprising at least the variable domain of a light or heavy chain of an antibody. In an embodiment, antibody fragments are selected from single-chain variable fragments (scFv), single chain antibodies, F(ab')2 fragments, Fab fragments, and fragments produced by a Fab expression library or single-domain antibodies (nanobodies).

As used herein, the term "single chain variable fragment (scFv)" refers to an artificial antibody fragment comprising a variable domain of a light and a heavy chain of an antibody covalently linked. In an embodiment, the variable domain of a light (V_{L}) and a heavy chain (V_{H}) of an antibody are covalently linked by a short peptide of ten to 25 amino acids. In a further embodiment, the short peptide links the N-terminus of the V_{H} with the C-terminus of the V_{L}, or vice versa.

In embodiments, the antibody is obtained from an animal species, preferably from a mammal such as human, simian, mouse, rat, rabbit, guinea pig, horse, cow, sheep, goat, pig, dog or cat. Preferably, the antibody or antibody fragment is a human, humanized or deimmunized antibody. Humanized antibodies can be prepared in various ways, for example, by resurfacing and CDR grafting. In case of resurfacing, a combination of molecular modeling, statistical analyses, and mutagenesis is used to modify all non-CDR regions on the surface of the antibody to become similar to the surface of antibodies of the target organism. In CDR grafting, the CDR regions according to the invention are introduced into known human framework regions, which are similar in sequence to the original ones. Deimmunized antibodies can be obtained by specifically mutating residues that confer immunogenicity hotspots as predicted based *on in silico* peptide-MHC affinity prediction.

In embodiments, the antibody or antibody fragment is a polyclonal, a monoclonal, or a chimeric antibody, wherein an antigen binding region of a non-human antibody is transferred into the framework of a human antibody by recombinant DNA techniques including *in silico* design.

In embodiments, antibodies to a selected tag or antigen may be produced by immunization of various hosts including, but not limited to, goats, rabbits, rats, mice, humans, through injection with cells expressing a particular protein, DNA or RNA encoding for the protein, the protein itself or any portion, fragment or oligopeptide that retain immunogenic properties of the protein.

In embodiments, the PD-L1 binding domain is selected from PD-1, BMS-936559, Atezolizumab, Durvalumab, Avelumab, 3F2.1 and fragments thereof. Preferably, the PD-L1 binding domain is an scFv fragment of BMS-936559, Atezolizumab or Durvalumab.

As used herein, the term "fragments" refers to proteins consisting of at least 90 %, preferably at least 95 %, more preferably at least 98 % of the amino acid sequence of the PD-L1 binding domains.

As used herein, the term "PD-1" (Programmed cell death protein 1) refers to a protein on the surface of cells that has a role in regulating the immune system's response to tie cells of the human body by down-regulating the immune system and promoting self-tolerance by suppressing T cell inflammatory activity. Preferably, the PD-L1 binding domain comprises at least one sequence with a sequence identity of at least 90 %, preferably at least 95 %, more preferably at least 98 % to SEQ ID No. 3 or a fragment thereof, wherein the at least one sequence binds the antigen PD-L1 and PD-L2. In embodiments, the fragment of SEQ ID No. 3 comprises the extracellular domain of PD-1.

As used herein, the term "BMS-936559" refers to an antibody that specifically binds PD-L1. Preferably, the PD-L1 binding domain comprises at least one sequence each with a sequence identity of at least 90 %, preferably at least 95 %, more preferably at least 98 % to SEQ ID No.4 and SEQ ID No. 5, wherein the at least one sequence binds the antigen PD-L1.

As used herein, the term "Atezolizumab" refers to an antibody that specifically binds PD-L1. Preferably, the PD-L1 binding domain comprises at least one sequence each with a sequence identity of at least 90 %, preferably at least 95 %, more preferably at least 98 % to SEQ ID No. 6 and SEQ ID No. 7, wherein the at least one sequence binds the antigen PD-L1.

As used herein, the term "Durvalumab" refers to an antibody that specifically binds PD-L1. Preferably, the PD-L1 binding domain comprises at least one sequence each with a sequence identity of at least 90 %, preferably at least 95 %, more preferably at least 98 % to SEQ ID No. 8 and SEQ ID No. 9, wherein the at least one sequence binds the antigen PD-L1.

As used herein, the term "Avelumab" refers to an antibody that specifically binds PD-L1. Preferably, the PD-L1 binding domain comprises at least one sequence each with a sequence identity of at least 90 %, preferably at least 95 %, more preferably at least 98 % to SEQ ID No. 61 and SEQ ID No. 62, wherein the at least one sequence binds the antigen PD-L1.

As used herein, the term "3F2.1" refers to an antibody that specifically binds PD-L1. Preferably, the PD-L1 binding domain comprises at least one sequence each with a sequence identity of at least 90 %, preferably at least 95 %, more preferably at least 98 % to SEQ ID No. 63 and SEQ ID No. 64, wherein the at least one sequence binds the antigen PD-L1.

As used herein, the term "tag" refers to a peptide sequence attached to peptides or proteins to enable them to bind to specific atoms, ions or molecules, in particular the tag-binding domain.

In embodiments, the PD-L2 binding domain is selected from an antibody or fragment thereof, which specifically binds PD-L2.

In embodiments, the tag is a peptide epitope tag. In further embodiments, the tag comprises 10 to 20 amino acids.

In embodiments, the peptide epitope tag is a myc-tag, a His-tag, a short linear peptide sequence from yeast transcription factor GCN4, preferably according to SEQ ID No. 10, SEQ ID No. 11 or mutants thereof; a leucine zipper sequence, preferably SYNZIP 1 to SYNZIP 48, BATF, FOS, ATF4, ATF3, BACH1, JUND, NFE2L3, HEPTAD (Reinke et al. 2010), a sequence according to SEQ ID No. 12 or SEQ ID No. 13 or mutants thereof; or a short linear peptide sequence from a human nuclear protein, preferably from the human La protein. Preferably, the peptide epitope tag from the human La protein is the human La epitope E5B9 according to SEQ ID No. 14 or E7B6 according to SEQ ID No. 15 or SEQ ID No. 16, most preferably the human La epitope E5B9 according to SEQ ID No. 14 or E7B6 according to SEQ ID No. 16.

As used herein, the term "mutants" refers to peptides or proteins having at least 85 % sequence identity to the named peptides or proteins, preferably at least 95 % sequence identity. Advantageously, the mutants are capable of having one or more activities of the named peptides or proteins, in particular bind the identical tag-binding domains as the peptide epitope tag.

In embodiments, mutants are truncated versions of peptides or proteins. As used herein, the term "truncated versions" refers to shortened peptides or proteins having at least 85 % sequence identity to the named peptides or proteins, preferably at least 95 % sequence identity, more preferably having a chain length of at least 85 % and a sequence identity of 100 %, most preferably a chain length of at least 95 % and a sequence identity of 100 %. Advantageously, the truncated version has at least 80 %, preferably of at least 90 %, more preferably of at least 95 %; of the activity of the named peptide or protein.

As used herein, the term "nuclear protein" refers to a protein found in the cell nucleus.

In embodiments, the His-tag is an amino acid sequence consisting of histidine residues, preferably in the range of six to fourteen histidine residues.

Advantageously, tags, which are peptide sequences from nuclear antigens, cannot be accessed and bound by the corresponding tag-binding domain in the context of the native protein under physiological conditions. Further advantageously, the tag is not immunogenic. This leads to minimization of risk of uncontrolled on-target off-site toxicities by CAR expressing immune cells like release of toxic levels of cytokines, referred to variously as cytokine storms or cytokine release syndrome (CRS).

In preferred embodiments, the peptide epitope tag is a myc-tag, a His-tag, a short linear peptide sequence from yeast transcription factor GCN4, preferably according to SEQ ID No. 10, SEQ ID No. 11; a leucine zipper sequence, preferably SYNZIP 1 to SYNZIP 48, BATF, FOS, ATF4, ATF3, BACH1, JUND, NFE2L3, HEPTAD (Reinke et al. 2010), a sequence according toSEQ ID No. 12 or SEQ ID No. 13; or a short linear peptide sequence from a human nuclear protein, preferably from the human La protein.

In further embodiments, the tag-binding domain is an antibody or an antibody fragment.

In embodiments, the tag-binding domain binds to a tag from the human nuclear La protein, preferably the tag-binding domain is an antibody or an antigen-binding fragment, wherein the tag-binding domain constitutes an anti-La epitope scFv, more preferably an anti-La epitope scFv comprising a V_{L} according to the following sequence: 
DIVMTQSPDSLAVSLGERATINCX¹SSQSLLNSRTX²KNYLAWYQQKPGQPPKLLIYWASTRX³S
GVPDRFSGSGSGTDFTLTISSLQAEDVAVYYCKQSYNLX⁴TFGGGTKVEIK (SEQ ID No. 17),
 wherein X¹ to X⁴ are variable amino acid residues, preferably selected from a proteinogenic alpha amino acid residue; or a sequence having at least 85 % sequence identity, preferably at least 95 % sequence identity; to one of the sequences SEQ ID No. 19 or SEQ ID No. 21.

In some embodiments, the variable amino acid residues X¹ to X⁴ are selected as follows:
X¹ is selected from polar and/or positive charged residues, such as Serine, Threonine, Asparagine, Glutamine, Histidine, Lysine and Arginine; preferably Lysine or Arginine;
X² is preferably selected from Lysine and Proline;
X³ is selected from polar and charged residues, such as Asparagine, Aspartic Acid, Glutamine, Glutamic acid, Histidine, Lysine and Arginine, preferably Glutamic acid and Lysine;
X⁴ is selected from hydrophobic residues, such as Isoleucine, Leucine, Valine, Alanine, Methionine, Phenylalanine, Proline and Tryptophan; preferably Leucine or Proline.

In further embodiments, the tag-binding domain is an antibody or an antigen-binding fragment, wherein the tag-binding domain constitutes an anti-La epitope scFv comprising a sequence having at least 85 % sequence identity, preferably at least 95 % sequence identity; to one of the sequences SEQ ID No. 18 or SEQ ID No. 20.

Preferably, the tag-binding domain constitutes an anti-La epitope scFv comprising a sequence having each at least 85 % sequence identity, preferably at least 95 % sequence identity; to the sequences according to SEQ ID NO. 18 (V_{H}) and SEQ ID NO. 19 (V_{L}) or the sequences according to SEQ ID NO. 20 (V_{H}) and SEQ ID NO. 21 (V_{L}).

Most preferably, the tag-binding domain constitutes the anti-La 5B9 scFv according to SEQ ID NO. 18 (V_{H}) and SEQ ID NO. 19 (V_{L}) or the anti-La 7B6 scFv according to SEQ ID NO. 20 (V_{H}) and SEQ ID NO. 21 (V_{L}).

In further embodiments, the targeting module according to the invention further comprises an effector cell binding domain, wherein the effector cell binding domain specifically binds an epitope on a human CD3 chain, a human T cell receptor (TCR) or human CD16.

As used herein, the term "effector cell binding domain" refers to a peptide or protein, which specifically binds a protein or protein complex (antigen) on an effector cell. As used herein, the term "protein complex" refers to a group of two or more associated protein chains.

As used herein, the term "effector cell" refers to an immune cell, which executes immune reactions, in particular an immune cell with cytolytic, phagocytic and/or immunosuppressive activity. Preferably, the effector cell is a T cell, a natural killer (NK) cell or a macrophage. Advantageously, the targeting module activates the effector cell by binding to the domain and exceeding a certain threshold regarding required number of such interactions.

Preferably, the effector cell binding domain is an antibody or antibody fragment.

In an embodiment, the effector cell binding domain specifically binds an epitope on human CD3 with a dissociation constant K_{d} up to 10⁻⁸ M. Commonly, the affinity of the effector cell binding domain, in particular the dissociation constant K_{d}, is determined with surface plasmon resonance measurements or flow-cytometry-based cellular binding assay.

Preferably, the effector cell binding domain specifically binds an epitope on human CD3 with a low affinity, preferably with a dissociation constant K_{d} in the range of 10⁻⁴ M to 10⁻⁸ M.

In embodiments, the effector cell binding domain comprises at least one of the following sequences:
i) a V_{L} according to SEQ ID No. 22 or 24 and/or
ii) a V_{H} according to SEQ ID No. 23 or 25.

In embodiments, the effector cell binding domain comprises at least one sequence with a sequence identity of at least 90 %, preferably at least 95 %, more preferably at least 98 % to SEQ ID No. 22 or 24 and/or SEQ ID No. 23 or 25, wherein the at least one sequence binds the antigen CD3.

Preferably, the humanized anti-human CD3 effector cell binding domain comprises a variable region of the heavy chain (V_{H}) according to SEQ ID No. 25 and a variable region of the light chain (V_{L}) according to SEQ ID No. 24, e.g. in the form of an scFv. Preferably, V_{H} and V_{L} are connected via glycine serine linker.

In some embodiments, the effector cell binding domain is a humanized anti-TCR domain and preferably comprises at least one of the following sequences:
i) a V_{L} according to SEQ ID No. 26 and/or
ii) a V_{H} according to SEQ ID No. 27.

In embodiments, the effector cell binding domain comprises at least one sequence with a sequence identity of at least 90 %, preferably at least 95 %, more preferably at least 98 % to the sequences according to SEQ ID No. 26 or SEQ ID No. 27, wherein the at least one sequence binds the antigen TCR.

In some embodiments, the effector cell binding domain is a humanized anti-CD16A domain and preferably comprises at least one of the following sequences:
i) a V_{L} according to SEQ ID No. 28 and/or
ii) a V_{H} according to SEQ ID No. 29,
preferably, a V_{L} according to SEQ ID No. 28 and a V_{H} according to SEQ ID No. 29.

In an embodiment, the effector cell binding domain comprises at least one sequence with a sequence identity of at least 90 %, preferably at least 95 %, more preferably at least 98 % to the sequences according to SEQ ID No. 28 or SEQ ID No. 29, wherein the at least one sequence binds the antigen CD16A.

In embodiments, the variable regions of the at least one PD-L1 and/or PD-L2 binding domain and/or the effector cell binding domain comprise a humanized amino acid sequence.

In embodiments, the different domains of the targeting module according to the invention are linked with each other by a linker. The linker comprises a short sequence of preferably 10 to 20 amino acid residues. In embodiments, the targeting module comprises a flexible peptide sequence that is selected such that the domains have a three-dimensional folding that allows them to exhibit the specificity for effector cell and target cell binding. Preferred linkers are glycine-serine linkers with the structure (GₓS_{y}) with x and y selected from 1 to 10, preferably 1 to 5. Mostly preferred are 1 to 10 repeats of the sequence G₄S₁. Moreover, linkers are preferred that are constituted of a peptide sequence that can increase the protease resistance of the antibody derivatives.

Preferably, the targeting module according to the invention is expressed as a fusion protein.

In embodiments, the targeting module according to the invention comprises a further domain selected from the group comprising co-stimulatory ligands, radionuclides, cell death inducing chemical compounds and half-life increasing domains, preferably IgG1 Fc, IgG2 Fc, IgG3 Fc, IgG4 Fc, HSA or mutants thereof. As used herein, the term "mutants" refers to proteins having at least 85 % sequence identity to the half-life increasing domain, preferably at least 95 % sequence identity. Advantageously, the mutant is capable of having one or more activities of the named peptides or proteins, in particular the mutant increases the half-life like the half-life increasing domain.

In preferred embodiments, the targeting module according to the invention comprises a further domain selected from the group comprising co-stimulatory ligands, radionuclides, cell death inducing chemical compounds and half-life increasing domains, preferably IgG1 Fc, IgG2 Fc, IgG3 Fc, IgG4 Fc or HSA.

In embodiments, the length of the targeting module according to the invention is in the range of 20 to 1600 amino acids.

In some embodiments, the targeting module according to the invention is used in a method for stimulating a chimeric antigen receptor mediated immune response in a mammal, wherein the targeting module is administered in combination with a vector or a cell comprising a nucleotide sequence encoding a switchable chimeric antigen receptor and at least one further targeting module, wherein the at least one further targeting module comprises at least one target cell binding domain and a tag-binding domain or a tag, wherein the at least one target cell binding domain is an antibody, an antibody fragment, a protein, a peptide or a low molecular weight organic ligand that binds to surface antigens selected from the group comprising CD2, CD3, CD4, CD8, CD10, CD19, CD20, CD22, CD23, CD25, CD30, CD33, CD38, CD44, CD44v6 CD52, CD90, CD99, CD123, CD133, CD150 CD181, CD182, CD184, CD223, CD229, CD269 (BCMA), CD273, CD274, CD276, CD279, CD319, CD366 and CD371, interleukin receptors, especially preferred IL-8Ra (CXCR1), IL-8Rβ (CXCR2), IL-11Ra, IL-11Rβ, IL-13Ra1 and 2, CXCR4, c-Met, mesothelin, members of the epidermal growth factor receptor family and mutants thereof, especially preferred ErbB1, ErbB2, ErbB3, ErbB4 or mutants thereof, members of the tumor necrosis factor receptor superfamily, ephrins, ephrin receptors, especially preferred EphA1-10, EphA5 or EphB1-6, prostate specific antigens prostate stem cell antigen (PSCA) and prostate specific membrane antigen (PSMA), embryonic antigens carcinoembryonic antigen (CEA) and fetal acethylcholine receptor, members of the vascular endothelia growth factor family, epithelia cell adhesion molecule EpCAM, alphafetoprotein AFP, members of the intercellular adhesion molecule family, members of the mucin protein family, follicle stimulating hormone receptor (FSHR), the human high molecular weight-melanoma-associated antigen (HMW-MAA), folate binding protein FBP, folate receptors, somatostatin receptors, ligands of the NKG2D receptor, cytokine receptors, members of the epithelia glycoprotein family, diasialogangliosides, glypicans, G protein-coupled receptors, members of the carbonic anhydrase family, members of the carbohydrate antigen family, Notch ligands, melanoma-associated chondroitin sulfate proteoglycan (MCSP), glycoprotein A33, guanylate cyclase 2C and tumor-specific glycans, including mutants and analogues of the named antibodies, antibody fragments, proteins, peptides or low molecular weight organic ligands, wherein the targeting module according to the invention and the at least one further targeting module comprise different target cell binding domains, and identical tag-binding domains or a tags.

As used herein, the term "target cell binding domain" refers to a peptide, protein, or low molecular weight organic ligand, which specifically binds a protein or protein complex (antigen) on the surface of a target cell, preferably a cancer cell, T cell, infected cell or pathogens or parasites.

As used herein, the terms "peptide", "polypeptide" and "protein" are used interchangeably, and refer to a compound comprised of amino acid residues covalently linked by peptide bonds. A protein or peptide must contain at least two amino acids, and no limitation is placed on the maximum number of amino acids that can comprise a protein's or peptide's sequence.

As used herein, the term "low molecular weight organic ligand" refers to an organic molecule with a molecular weight of maximal 10 kilodalton, preferably of maximal 3 kilodalton, which specifically binds a protein or protein complex (antigen) on the surface of a target cell, preferably a cancer cell, T cell, infected cell or pathogens or parasites.

The term "target cell binding domain" also comprises soluble T cell receptors, which are composed of the alpha and beta or the gamma and delta chains of a T cell receptor (TCR), fragments or mutants thereof. Such TCR-derived binding moieties recognize and bind to peptides presented by human leukocyte antigen class (HLA) I and II protein complexes. Examples are, but are not limited to, TCRs specific for peptides derived from proteins like EGFR family, survivin, sry-like high motility group box (SOX) protein family, melanoma-associated antigens (e.g. autoimmunogenic cancer/testis antigen NY-ESO-1, members of the melanoma antigen family A MAGEA, the preferentially expressed antigen in melanoma PRAME), and leukemia-associated antigens (e.g. Wilms tumor gene 1 WT1).

As used herein, the term "mutants" refers to peptides or proteins having at least 85 % sequence identity to the named antibodies, antibody fragments, proteins or peptides, preferably at least 95 % sequence identity. Advantageously, the mutants bind the identical antigens as the named antibodies, antibody fragments, proteins or peptides.

As used herein, the term "analogues" refers to molecules having a high degree of structural identity to the named antibodies, antibody fragments, proteins, peptides or low molecular weight organic ligands, preferably at least one atom, group of atoms, functional group or substructure is replaced with another group of atoms, e.g. a hydroxy group. In embodiments, an analogue of somatostatin (SRIF14) is octreotide or pasireotide. Advantageously, the analogues bind the identical antigens as the named antibodies, antibody fragments, proteins, peptides or low molecular weight organic ligands.

In embodiments, the analogues of the named antibodies, antibody fragments, proteins or peptides comprise modifications selected from the group comprising D amino acids, pseudo peptide bonds, aminoalcohols, non-proteinogenic amino acids, unnatural amino acids, amino acids with modified side chains and/or circular proteins. Advantageously, these analogues reveal increased stability.

In further embodiments, the target cell binding domain is soluble T cell receptor consisting of the alpha and beta or the gamma and delta chain of a T cell receptor (TCR).

In preferred embodiments, the at least one further targeting module comprises at least one target cell binding domain and a tag-binding domain or a tag, wherein the at least one target cell binding domain is an antibody or an antibody fragment that binds to surface antigens selected from the group comprising CD2, CD3, CD4, CD8, CD10, CD19, CD20, CD22, CD23, CD25, CD30, CD33, CD38, CD44, CD44v6 CD52, CD90, CD99, CD123, CD133, CD150 CD181, CD182, CD184, CD223, CD229, CD269 (BCMA), CD273, CD274, CD276, CD279, CD319, CD366 and CD371, interleukin receptors, especially preferred IL-8Rα (CXCR1), IL-8Rβ (CXCR2), IL-11Rα, IL-11Rβ, IL-13Ra1 and 2, CXCR4, c-Met, mesothelin, members of the epidermal growth factor receptor family, especially preferred ErbB1, ErbB2, ErbB3 or ErbB4; members of the tumor necrosis factor receptor superfamily, ephrins, ephrin receptors, especially preferred EphA1-10, EphA5 or EphB1-6, prostate specific antigens prostate stem cell antigen (PSCA) and prostate specific membrane antigen (PSMA), embryonic antigens carcinoembryonic antigen (CEA) and fetal acethylcholine receptor, members of the vascular endothelia growth factor family, epithelia cell adhesion molecule EpCAM, alphafetoprotein AFP, members of the intercellular adhesion molecule family, members of the mucin protein family, follicle stimulating hormone receptor (FSHR), tie human high molecular weight-melanoma-associated antigen (HMW-MAA), folate binding protein FBP, folate receptors, somatostatin receptors, ligands of the NKG2D receptor, cytokine receptors, members of the epithelia glycoprotein family, diasialogangliosides, glypicans, G protein-coupled receptors, members of the carbonic anhydrase family, members of the carbohydrate antigen family, Notch ligands, melanoma-associated chondroitin sulfate proteoglycan (MCSP), glycoprotein A33, guanylate cyclase 2C and tumor-specific glycans, wherein the targeting module according to the invention and the at least one further targeting module comprise different target cell binding domains, and identical tag-binding domains or a tags.

In further embodiments, the at least one further targeting module comprises two target cell binding domains binding to surface antigens selected from PSCA and PSMA, CD19 and CD20, CD19 and CD22, CD19, CD20 and CD22, CD19 and CD123, CD33 and CD123, CD33 and CD99, CD33 and CD366, CD123 and CD366, CD123 and CD371, CD366 and CD371, ErbB-1 and ErbB-2, PSCA and ErbB-2, PSMA and CEA, IL-13Ra2 and ErbB-2, CD38 and CD269, mesothelin and mucin 16.

Preferably, the targeting module according to the invention comprises one of the sequences according to SEQ ID No. 57 to 59, more preferably the targeting module according to the invention consists of one of the sequences according to SEQ ID No. 57 to 59.

In preferred embodiments, the targeting module according to the invention is used in a method for stimulating a chimeric antigen receptor mediated immune response in a mammal, wherein the targeting module is administered, preferably in the range of 3 days to 30 days, after the administration of the vector or cell comprising a nucleotide sequence encoding a switchable chimeric antigen receptor and at least one further targeting module. Advantageously, by using the targeting module in combination with a further targeting module, wherein the targeting module is administered after the administration of the vector or cell comprising a nucleotide sequence encoding a switchable chimeric antigen receptor and the further targeting module, adverse systemic effects of the PD-L1 therapy are reduced.

More preferably, the targeting module according to the invention is used in a method for stimulating a chimeric antigen receptor mediated immune response in a mammal, wherein the at least one further targeting module is administered on its own, preferably one hour to 2 days, more preferably 4 to 24 hours, prior to the administration of the vector or cell comprising a nucleotide sequence encoding a switchable chimeric antigen receptor and the targeting module according to the invention is administered, preferably in the range of 3 days to 30 days, after the administration of the vector or cell comprising a nucleotide sequence encoding a switchable chimeric antigen receptor.

The present invention further comprises a nucleic acid, a vector or a cell comprising a nucleotide sequence encoding a targeting module according to the invention for use in a method for stimulating a chimeric antigen receptor mediated immune response in a mammal, preferably for use in the treatment of cancer, infectious disease or autoimmune disease;
wherein the nucleic acid, vector or cell is administered in combination with a vector or a cell comprising a nucleotide sequence encoding a switchable chimeric antigen receptor, wherein the switchable chimeric antigen receptor comprises three domains, wherein
- the first domain is a tag-binding domain or a tag,
- the second domain is an extracellular hinge and a transmembrane domain and
- the third domain is a signal transduction domain,
wherein the tag-binding domain of the targeting module binds to the tag of the switchable chimeric antigen receptor or the tag of the targeting module binds to the tag-binding domain of the switchable chimeric antigen receptor.

According to the invention, the nucleic acid, vector and/or cell are isolated. As used herein, the term "isolated" means altered or removed from the natural state.

In embodiments, the nucleic acid is a cDNA. As used herein, the term "cDNA" (complementary DNA) refers to double-stranded DNA synthesized from a single stranded RNA, e.g. mRNA, in a reaction catalyzed by the enzyme reverse transcriptase. In embodiments, cDNA is of synthetic origin. In further embodiments, cDNA is derived from mRNA, therefore containing only exons but no introns, as opposed to genomic DNA.

The vector is preferably a plasmid, an artificial chromosome, linearized DNA or RNA, a virus particle or another vector that contains an expression cassette that is incorporated stably into the genome of a host cell or host organism.

In embodiments, the cell is selected from immune cells, preferably with cytolytic, phagocytic or immunosuppressive activity, such as T cells, Natural Killer (NK) cells and macrophages. In preferred embodiments, the cell is selected from T cells, including alpha/beta and gamma/delta T cells or subpopulations of T cells like stem-cell memory T cells or central memory T cells, cytotoxic T cells; or NK cells.

In embodiments, the nucleic acid, vector or cell further comprises an inducible expression system. In some embodiments, the inducible expression system is based on a prokaryotic operon, including, but not limited to, the lac operon, transposon Tn10 or tetracycline operon. In other embodiments, the inducible expression system is based on components of a eukaryotic signaling pathway, including, but not limited to, expression systems based on a steroid receptor, an estrogen receptor, progesterone or metallothionein.

In preferred embodiments, the inducible expression system is selected from Tet-On® (also rtTA; TET Systems, Takara Bio), Tet-On® Advanced (also rtTA2^{s}-M2 or rtTA2-syn1; TET Systems, Takara Bio), Tet-On® 3G (also rtTA-V16; TET Systems, Takara Bio), T-REx® (Life Technologies) or a promoter responsive to nuclear factor of activated T cells (NFAT).

As used herein, the term "Tet-On" refers to an inducible expression system comprising at least a tetracycline response element (TRE) and reverse tetracycline transactivator (rtTA) protein or variant thereof. As used herein, the term "reverse tetracycline transactivator (rtTA) protein" refers to a protein, which is capable of binding the operator (tetO) on the TRE, if bound by a tetracycline or a derivative of tetracycline, e.g. doxycycline. Thus, the introduction of tetracycline or a derivative of tetracycline to the system initiates the transcription of the genetic product. In embodiments, the inducible expression system induces the transcription of the nucleotide sequence encoding a switchable chimeric antigen receptor and/or a nucleotide sequence encoding a targeting module according to the invention, preferably the inducible expression system induces the transcription of the nucleotide sequence encoding a targeting module according to the invention.

As used herein, the term "variant" refers to a nucleotide sequence, peptide or protein, which is capable of performing one or more functions of the nucleotide or named amino acid sequence.

As used herein, the term "derivative" refers to a molecule having a high degree of structural identity to the molecule, preferably the same scaffold, wherein at least one atom, group of atoms, functional group or substructure is replaced with another atom, group of atoms functional group or substructure, e.g. a hydroxy group. Advantageously, the derivative is capable of having one or more activities of the named molecule.

Advantageously, the Tet-On system is preferred over Tet-Off for its faster responsiveness and its activation rather than repression by tetracycline or a derivative of tetracycline.

In embodiments, the rtTA protein is a fusion protein of rTetR (reverse tetracycline repressor), a mutated version of TetR (tetracycline repressor) from Escherichia coli, and the activation domain of virion protein 16 (VP16) from Herpes Simplex Virus. In preferred embodiments, rtTA comprises SEQ ID No. 30, SEQ ID No. 31 or SEQ ID No. 32.

As used herein, the term "tetracycline response element (TRE)" refers to at least one TetO sequence with a minimal promoter, responding to binding of the rtTA protein by increased expression of the gene or genes downstream of its promoter.

In embodiments, the TRE consists of 2 to 9 repeats, preferably 7 repeats, of the 19 bp bacterial TetO sequence according to SEQ ID No. 33, preferably separated by spacer sequences, more preferably by spacer sequences comprising SEQ ID No. 34.

In preferred embodiments, the TRE is SEQ ID No. 35.

As used herein, the term "Tet-On Advanced" refers to a Tet-On system with reduced basal expression and increased sensitivity to doxycycline, which is human codon optimized and comprises three minimal transcriptional activation domains, corresponding to the rtTA of SEQ ID No. 31.

As used herein, the term "Tet-On 3G" refers to a Tet-On system with further increased sensitivity to doxycycline, corresponding to the rtTA of SEQ ID No. 32.

As used herein, the term "T-REx" refers to a system, where the gene of interest is flanked by an upstream CMV promoter and two TetO2 sites. Binding of TetR homodimers to each TetO2 site represses gene expression in the absence of tetracycline or a derivative of tetracycline. In the presence of tetracycline or a derivative of tetracycline to the TetR homodimers, they dissociate from TetO2 resulting in expression of the gene of interest.

As used herein, the term "promoter responsive to nuclear factor of activated T cells (NFAT)" refers to a promoter naturally occurring in human cell nucleus, which is bound by nuclear factors of activated T cells (NFAT) initiating the transcription of the genetic product. As used herein, the term "nuclear factors of activated T cells (NFAT)" refers to transcription factors with one DNA binding domain for transcription and two calcineurin binding domains for the activation by calcineurin. The activation by calcineurin takes place by the activation of the serine/threonine phosphatase calcineurin by calmodulin, a calcium sensor protein. Activated calcineurin dephosphorylates the serine-rich region (SRR) and SP-repeats in the amino termini of NFAT proteins, resulting in a conformational change that exposes a nuclear localization signal, resulting in NFAT nuclear import.

In embodiments, the promoter responsive to NFAT consists of 2 to 12 repeats, preferably 6 repeats, of the 30 bp human NFAT binding sequence according to SEQ ID No. 36, optionally separated by spacer sequences, followed by a minimal promoter.

In embodiments, the minimal promoter is derived from the interleukin-2 (IL-2) promoter according to SEQ ID No. 37 or SEQ ID No. 38.

In embodiments, the promoter responsive to NFAT has SEQ ID No. 39 or SEQ ID No. 40.

In further embodiments, the nucleic acid, vector or cell according to the invention further comprises a nucleotide sequence encoding a switchable chimeric antigen receptor,
wherein the switchable chimeric antigen receptor comprises three domains,
wherein
- the first domain is a tag-binding domain or tag,
- the second domain is an extracellular hinge and a transmembrane domain and
- the third domain is a signal transduction domain,
wherein the tag-binding domain of the targeting module binds to the tag of the switchable chimeric antigen receptor or the tag of the targeting module binds to the tag-binding domain of the switchable chimeric antigen receptor.

Another aspect of the invention is a nucleic acid, a vector or a cell comprising
- an inducible expression system, preferably selected from Tet-On, Tet-On Advanced, Tet-On 3G, T-REx or a promoter responsive to NFAT, and
- a nucleotide sequence encoding a switchable chimeric antigen receptor,
   wherein the switchable chimeric antigen receptor comprises three domains,
   wherein
   - the first domain is a tag-binding domain or tag,
   - the second domain is an extracellular hinge and a transmembrane domain and
   - the third domain is a signal transduction domain, and
- a nucleotide sequence encoding a targeting module according to the invention,
   wherein the tag-binding domain of the targeting module binds to the tag of the switchable chimeric antigen receptor or the tag of the targeting module binds to the tag-binding domain of the switchable chimeric antigen receptor.

In embodiments, the inducible expression system induces the transcription of the nucleotide sequence encoding a switchable chimeric antigen receptor and/or a nucleotide sequence encoding a targeting module according to the invention, preferably the inducible expression system induces the transcription of the nucleotide sequence encoding a targeting module according to the invention.

The invention further comprises a pharmaceutical composition comprising the targeting module according to the invention, the nucleic acid, vector or cell according to the invention for use in a method for stimulating a chimeric antigen receptor mediated immune response in a mammal, preferably for use in the treatment of cancer, infectious disease or autoimmune disease;
wherein the pharmaceutical composition is administered in combination with a vector or a cell comprising a nucleotide sequence encoding a switchable chimeric antigen receptor, wherein the switchable chimeric antigen receptor comprises three domains,
wherein
- the first domain is a tag-binding domain or a tag,
- the second domain is an extracellular hinge and a transmembrane domain and
- the third domain is a signal transduction domain,
wherein the tag-binding domain of the targeting module binds to the tag of the switchable chimeric antigen receptor or the tag of the targeting module binds to the tag-binding domain of the switchable chimeric antigen receptor.

The pharmaceutical composition is preferably administered parenterally, particularly preferred intravenously. In an embodiment, the pharmaceutical composition is present in a form suitable for intravenous administration. Preferably, the pharmaceutical composition is a solution, emulsion or suspension.

In embodiments, the pharmaceutical composition is an injectable buffered solution comprising a concentration in the range of 1 ng/ml to 500 mg/ml of the targeting module, the nucleic acid, vector and/or cell according to the invention, preferably in the range of 5 µg/ml to 5 mg/ml.

In embodiments, the pharmaceutical composition further comprises a pharmaceutically acceptable dilution agent or carrier. In further embodiments, the carrier is selected from water, an aqueous buffer solution, 0.9 % saline solution, 5 % glucose, 5 % xylitol, 0.3 % glycine solution, ringer solutions, amino acid solutions and a similar solvent. In further embodiments, the aqueous buffer solution is selected from an aqueous histidine, sodium succinate, sodium citrate, sodium phosphate or potassium phosphate buffered solution with a pH value in the range of pH 5.0 to pH 7.0. In embodiments, the buffer solution has a buffer concentration in the range of 1 mmol/l (mM) to 500 mM, preferably in the range of 5 mM to 20 mM, especially preferred in the range of 5 mM to 10 mM. In further embodiments, the carrier comprises sodium chloride, preferably in a concentration in the range of 1 mM to 300 mM, especially preferred 150 mM. In embodiments, the pharmaceutical composition further comprises a stabilizer, preferably in a concentration in the range of 1 mM to 900 mM, especially preferred in the range of 50mM and 600 mM. In embodiments, the stabilizer is sucrose, trehalose or L-methionine.

In some embodiments, the pharmaceutical composition further comprises pharmaceutically acceptable excipients. The term "pharmaceutically acceptable excipients" refers to compounds, which provide approximately physiological conditions and/or increase the stability, such as agents for adjusting the pH value and buffering agents, agents for adjusting the toxicity and the like. In embodiments, pharmaceutically acceptable excipients are selected from sodium acetate, sodium chloride, potassium chloride, calcium chloride, sodium lactate and polysorbate-80, preferably polysorbate-80 in the range of 0.0001 % (w/v) to 1 % (w/v), especially preferred in the range of 0.001 % (w/v) to 0.1 % (w/v).

In a preferred embodiment, the pharmaceutical composition comprises the targeting module in a dosage quantity in the range of 25 µg/day to 100 mg/day, preferably dosage quantities in the range of 0.1 mg/day to 20 mg/day.

In further embodiments, the pharmaceutical composition is sterile. The pharmaceutical composition is sterilized by conventional well-known techniques including, but not limited to, sterile filtration.

In embodiments, the pharmaceutical composition is used for administration to a subject.

In embodiments, the pharmaceutical composition is lyophilized prior to storage or stored as solution at ambient temperature or below, including, but not limited to, frozen storage.

In embodiments, the pharmaceutical composition is reconstituted and/or diluted in an infusion and stabilizer solution prior to administration to a subject. The solutions used for reconstitution or infusion/stabilization may contain any of the components mentioned for the pharmaceutical composition or similar components.

Another aspect of the invention is a kit comprising
a) a targeting module according to the invention, the nucleic acid, vector or cell according to the invention and
b) a vector or a cell comprising a nucleotide sequence encoding a switchable chimeric antigen receptor,
   wherein the switchable chimeric antigen receptor comprises three domains,
   wherein
   - the first domain is a tag-binding domain or tag,
   - the second domain is an extracellular hinge and a transmembrane domain and
   - the third domain is a signal transduction domain,
   wherein the tag-binding domain of the targeting module binds to the tag of the switchable chimeric antigen receptor or the tag of the targeting module binds to the tag-binding domain of the switchable chimeric antigen receptor.

In embodiments, the tag is a peptide epitope tag. In further embodiments, the tag comprises 10 to 20 amino acids.

In embodiments, the peptide epitope tag is a myc-tag, a His-tag, a short linear peptide sequence from yeast transcription factor GCN4, preferably according to SEQ ID No. 10, SEQ ID No. 11 or mutants thereof; a leucine zipper sequence, preferably SYNZIP 1 to SYNZIP 48, BATF, FOS, ATF4, ATF3, BACH1, JUND, NFE2L3, HEPTAD (Reinke et al. 2010), a sequence according to SEQ ID No. 12 or SEQ ID No. 13 or mutants thereof, or a short linear peptide sequence from a human nuclear protein, preferably from the human La protein. Preferably, the peptide epitope tag from the human La protein is the human La epitope E5B9 according to SEQ ID No. 14 or E7B6 according to SEQ ID No. 15 or SEQ ID No. 16, most preferably the human La epitope E5B9 according to SEQ ID No. 14 or E7B6 according to SEQ ID No. 16.

In embodiments, the His-tag is an amino acid sequence consisting of histidine residues, preferably in the range of six to fourteen histidine residues.

In preferred embodiments, the peptide epitope tag is a myc-tag, a His-tag, a short linear peptide sequence from yeast transcription factor GCN4, preferably according to SEQ ID No. 10 or SEQ ID No. 11; a leucine zipper sequence, preferably SYNZIP 1 to SYNZIP 48, BATF, FOS, ATF4, ATF3, BACH1, JUND, NFE2L3, HEPTAD (Reinke et al. 2010), a sequence according to SEQ ID No. 12 or SEQ ID No. 13; or a short linear peptide sequence from a human nuclear protein, preferably from the human La protein.

In further embodiments, the tag-binding domain is an antibody or an antibody fragment.

In embodiments, the tag-binding domain is an antibody or an antibody fragment binding to a myc-tag, a His-tag, a short linear peptide sequence from yeast transcription factor GCN4, a leucine zipper sequence or a short linear peptide sequence from a human nuclear protein.

In embodiments, the tag-binding domain binds to a tag from the human nuclear La protein, preferably the tag-binding domain is an antibody or an antigen-binding fragment, wherein the tag-binding domain constitutes an anti-La epitope scFv, more preferably an anti-La epitope scFv comprising a V_{L} according to the following sequence: 
DIVMTQSPDSLAVSLGERATINCX¹SSQSLLNSRTX²KNYLAWYQQKPGQPPKLLIYWASTRX³S
GVPDRFSGSGSGTDFTLTISSLQAEDVAVYYCKQSYNLX⁴TFGGGTKVEIIK (SEQ ID No. 17),
 wherein X¹ to X⁴ are variable amino acid residues, preferably selected from a proteinogenic alpha amino acid residue; or a sequence having at least 85 % sequence identity, preferably at least 95 % sequence identity; to one of the sequences SEQ ID No. 19 or SEQ ID No. 21.

In some embodiments, the variable amino acid residues X¹ to X⁴ are selected as follows:
X¹ is selected from polar and/or positive charged residues, such as Serine, Threonine, Asparagine, Glutamine, Histidine, Lysine and Arginine; preferably X¹ is Lysine or Arginine;
X² is preferably selected from Lysine and Proline;
X³ is selected from polar and charged residues, such as Asparagine, Aspartic Acid, Glutamine, Glutamic acid, Histidine, Lysine and Arginine, preferably Glutamic acid and Lysine;
X⁴ is selected from hydrophobic residues, such as Isoleucine, Leucine, Valine, Alanine, Methionine, Phenylalanine, Proline and Tryptophan; preferably Z⁴ is selected from Leucine and Proline.

In further embodiments, the tag-binding domain is an antibody or an antigen-binding fragment, wherein the tag-binding domain constitutes an anti-La epitope scFv comprising a sequence having at least 85 % sequence identity, preferably at least 95 % sequence identity; to one of the sequences SEQ ID No. 18 or SEQ ID No. 20.

Preferably, the tag-binding domain constitutes an anti-La epitope scFv comprising a sequence having each at least 85 % sequence identity, preferably at least 95 % sequence identity; to the sequences according to SEQ ID NO. 18 (V_{H}) and SEQ ID NO. 19 (V_{L}) or the sequences according to SEQ ID NO. 20 (V_{H}) and SEQ ID NO. 21 (V_{L}).

Most preferably, the tag-binding domain constitutes the anti-La 5B9 scFv according to SEQ ID NO. 18 (V_{H}) and SEQ ID NO. 19 (V_{L}) or the anti-La 7B6 scFv according to SEQ ID NO. 20 (V_{H}) and SEQ ID NO. 21 (V_{L}).

As used herein, the term "extracellular hinge and a transmembrane domain" refers to a flexible peptide sequence connected to the tag-binding domain or tag, which anchors the switchable CAR into the cell membrane of the cell and protruding from the surface of the cell for optimal binding to its particular targeting module.

In embodiments, the extracellular hinge and transmembrane domain is selected from hinge and transmembrane domains of human CD28 molecule, CD8a chain, NK cell receptors, preferably natural killer group NKG2D; or parts of the constant region of an antibody and combinations thereof. As used herein, the term "combinations thereof' refers to combinations of different hinge and transmembrane domains.

Pinthus *et al.* 2003, Pinthus *et al.* 2004 and Cartellieri *et al.* 2016 describe the use of hinge and transmembrane domains of the human CD28 molecule in CARs (Pinthus *et al.* 2003, Pinthus *et al.* 2004, Cartellieri *et al.* 2016).

Carpentino *et al.,* Milone *et al.* and Zhao *et al.* describe the use of hinge and transmembrane domains of human CD8a molecule in CARs (Carpentino *et al.* 2009, Milone *et al.* 2009, Zhao *et* al. 2009).

Zhang *et al.* 2005 and Zhang *et al.* 2006 describe the use of hinge and transmembrane domains of NKG2D in CARs (Zhang *et al.* 2005, Zhang *et al.* 2006).

Hombach *et al.,* Frigault *et al.* and Wang *et al.* describe the use of hinge and transmembrane domains of parts of the constant region of immunoblobulin G1 (IgG) (Hombach *et al.* 2007, Frigault *et al.* 2015, Wang *et al.* 2007). Frigault *et al.* describes the use of hinge domains of the constant region of IgG4.

Examples of combinations of the extracellular hinge and transmembrane domain are, but are not limited to, CD28 extracellular hinge and transmembrane domain, CD8alpha extracellular hinge and transmembrane domain, IgG1 or IgG4 constant regions combined with CD28 or CD137 transmembrane domain.

As used herein, the term "signal transduction domain" refers to a peptide sequence which transmits a signal into the cell by cross-linkage of the cell expressing the switchable CAR (effector cell) to a human cell surface protein or protein complex (target cell). Cross-linkage between effector and target cell is mediated by the targeting module according to the invention.

In embodiments, the signal transduction domain is selected from cytoplasmic regions of CD28, CD137 (4-1BB), CD134 (OX40), CD278 (ICOS), DAP10 and CD27, programmed cell death-1 (PD-1), cytotoxic T-lymphocyte antigen 4 (CTLA-4), cytoplasmic regions of CD3 chains, DAP12, CD122 (interleukin-2 receptor β), CD132 (interleukin-2 receptor γ), CD127 (interleukin-7 receptor a), CD360 (interleukin-21 receptor) activating Fc receptors and mutants thereof.

As used herein, the term "mutants" refers to proteins having at least 85 % sequence identity to the signal transduction domains, preferably at least 95 % sequence identity. Advantageously, the mutant transmits a signal into the cell by cross-linkage of the cell expressing the switchable CAR (effector cell) to a human cell surface protein or protein complex (target cell) in the same way as the named signal transduction domains.

In embodiments, mutants are truncated versions. As used herein, the term "truncated versions" refers to shortened proteins having at least 85 % sequence identity to the signal transduction domains, preferably at least 95 % sequence identity, more preferably having a chain length of at least 85 % and a sequence identity of 100 %, most preferably a chain length of at least 95 % and a sequence identity of 100 %. Advantageously, the truncated version has an activity of at least 80 %, preferably of at least 90 %, more preferably of at least 95 %; of the named signal transduction domains.

Hombach *et al.,* Maher *et al.* and Cartellieri *et al.* describe the use of cytoplasmic regions of CD28 as signal transduction domain in CARs (Hombach *et al.* 2001, Maher *et al.* 2002, Cartellieri *et al.* 2016). Guedan *et al.* describes the use of a mutant of cytoplasmic regions of CD28 as signal transduction domain (Guedan et al. 2020).

Milone *et al.* and Finney *et al.* describe the use of cytoplasmic regions of CD137 (4-1 BB) as signal transduction domain (Finney *et al.* 2004, Milone *et al.* 2009).

Finney *et al.* and Hombach and Abken describe the use of cytoplasmic regions of CD134 (OX40) as signal transduction domain in CARs (Finney *et al.* 2004, Hombach and Abken 2011, Hombach and Abken 2013).

Guedan *et al.* describes the use of cytoplasmic regions of CD278 (ICOS) as signal transduction domain (Guedan *et al.* 2018).

Zhang *et al.* describes the use of DAP10 as signal transduction domain (Zhang *et al.* 2005).

Fedorov *et al.* describes the use of programmed cell death 1 (PD-1) and of cytotoxic T-lymphocyte antigen 4 (CTLA-4) as signal transduction domain in CARs (Fedorov *et al.* 2013).

Gong *et al.* and Gade *et al.* describe the use of cytoplasmic regions CD3 chains, in particular the CD3ζ chain, as signal transduction domain in CARs (Gong *et al.* 1999, Gade *et al.* 2005).

Töpfer *et al.* describes the use of DAP12 as signal transduction domain in CARs (Töpfer *et al.* 2015).

Kagoya *et al.* describes the use of signaling chains or motifs derived from interleukin receptors as signal transduction domain in CARs (Kagoya *et al.* 2018).

Lamers *et al.* and Kershaw *et al.* describe the use of activating Fc receptors, in particular the Fc epsilon receptor γ chain, as signal transduction domain (Lamers *et al.* 2004, Kershaw *et al.* 2006).

In preferred embodiments, the signal transduction domain is selected from cytoplasmic regions of CD28, CD137 (4-1BB), CD134 (OX40), CD278 (ICOS), DAP10 and CD27, programmed cell death-1 (PD-1), cytotoxic T-lymphocyte antigen 4 (CTLA-4), cytoplasmic regions of CD3 chains, DAP12, CD122 (interleukin-2 receptor β), CD132 (interleukin-2 receptor γ), CD127 (interleukin-7 receptor a) and CD360 (interleukin-21 receptor) activating Fc receptors.

In embodiments, the switchable chimeric antigen receptor comprises at least one signal transduction domain, preferably two, three, four or more signal transduction domains, especially preferably selected from cytoplasmic regions of CD28, CD137 (4-1BB), CD134 (OX40), CD278 (ICOS), DAP10 and CD27, programmed cell death-1 (PD-1), cytotoxic T-lymphocyte antigen 4 (CTLA-4), cytoplasmic regions of CD3 chains, DAP12, CD122 (interleukin-2 receptor β), CD132 (interleukin-2 receptor γ), CD127 (interleukin-7 receptor a), CD360 (interleukin-21 receptor), activating Fc receptors and mutants thereof.

In preferred embodiments, the switchable chimeric antigen receptor comprises at least one signal transduction domain, preferably two, three, four or more signal transduction domains, especially preferably selected from cytoplasmic regions of CD28, CD137 (4-1BB), CD134 (OX40), CD278 (ICOS), DAP10 and CD27, programmed cell death-1 (PD-1), cytotoxic T-lymphocyte antigen 4 (CTLA-4), cytoplasmic regions of CD3 chains, DAP12, CD122 (interleukin-2 receptor β), CD132 (interleukin-2 receptor γ), CD127 (interleukin-7 receptor a) and CD360 (interleukin-21 receptor), activating Fc receptors.

In further embodiments, the switchable chimeric antigen receptor comprises a fourth domain, wherein the fourth domain is a short peptide linker in the extracellular portion of the receptor that may serve to detect the chimeric antigen receptor on the cell surface or stimulate the chimeric antigen receptor T cell.

In further embodiments, the fourth domain forms a linear epitope for a monoclonal antibody (mab) specifically binding to the fourth domain. In some embodiments, the fourth domain comprises at least one linear epitope, preferably E7B6 according to SEQ ID No. 15 or SEQ ID No. 16.

In some embodiments, the fourth domain is located in between the tag-binding domain or the tag and the extracellular hinge domain or an integral part of the extracellular hinge domain.

Advantageously, the switchable CAR engrafted cells with the fourth domain can be specifically stimulated to proliferate preferentially and persist longer compared to non-engrafted cells either *in vitro* or *in vivo.* Further advantageously, the fourth domain may be also used to purify switchable CAR engrafted cells from mixed cell populations or to dampen switchable CAR engrafted cell mediated immune response and to eliminate switchable CAR engrafted cells *in vivo.*

In further embodiments, the switchable CAR comprises a signal peptide. Advantageously, the signal peptide allows for expression on the cell surface of an effector cell. In an embodiment, the signal peptide is at the N-terminus of the switchable CAR nucleotide sequence in front of the tag-binding domain or the tag. In some embodiments, the signal peptide targets proteins to the secretory pathway either co-translationally or post-translationally and is selected from leader peptides from proteins like CD28, CD8alpha, IL-2 or the heavy or light chains of antibodies of human origin to avoid immunogenic reactions.

In further embodiments, the nucleic acid is a cDNA.

In embodiments, the nucleic acid encodes a switchable CAR according one of the sequences SEQ ID NO. 41 to 48. Preferably, the nucleic acid is one of the sequences SEQ ID NO. 49 to 56.

In some embodiments, the cell comprising a nucleotide sequence encoding a switchable chimeric antigen receptor is selected from immune cells, preferably with cytolytic, phagocytic or immunosuppressive activity, such as T cells, Natural Killer (NK) cells and macrophages. In preferred embodiments, the cell is selected from T cells, including alpha/beta and gamma/delta T cells or subpopulations of T cells like stem-cell memory T cells or central memory T cells, cytotoxic T cells; or NK cells.

In embodiments, the kit further comprises at least one further targeting module, wherein the at least one further targeting module comprises at least one target cell binding domain and a tag-binding domain or a tag, wherein the at least one target cell binding domain is an antibody, an antibody fragment, a protein, a peptide or a low molecular weight organic ligand that binds to surface antigens selected from the group comprising CD2, CD3, CD4, CD8, CD10, CD19, CD20, CD22, CD23, CD25, CD30, CD33, CD38, CD44, CD44v6 CD52, CD90, CD99, CD123, CD133, CD150 CD181, CD182, CD184, CD223, CD229, CD269 (BCMA), CD273, CD274, CD276, CD279, CD319, CD366 and CD371, interleukin receptors, especially preferred IL-8Ra (CXCR1), IL-8Rβ (CXCR2), IL-11Ra, IL-11Rβ, IL-13Ra1 and 2, CXCR4, c-Met, mesothelin, members of the epidermal growth factor receptor family and mutants thereof, especially preferred ErbB1, ErbB2, ErbB3, ErbB4 or mutants thereof, members of the tumor necrosis factor receptor superfamily, ephrins, ephrin receptors, especially preferred EphA1-10, EphA5 or EphB1-6, prostate specific antigens prostate stem cell antigen (PSCA) and prostate specific membrane antigen (PSMA), embryonic antigens carcinoembryonic antigen (CEA) and fetal acethylcholine receptor, members of the vascular endothelia growth factor family, epithelia cell adhesion molecule EpCAM, alphafetoprotein AFP, members of the intercellular adhesion molecule family, members of the mucin protein family, follicle stimulating hormone receptor (FSHR), the human high molecular weight-melanoma-associated antigen (HMW-MAA), folate binding protein FBP, folate receptors, somatostatin receptors, ligands of the NKG2D receptor, cytokine receptors, members of the epithelia glycoprotein family, diasialogangliosides, glypicans, G protein-coupled receptors, members of the carbonic anhydrase family, members of the carbohydrate antigen family, Notch ligands, melanoma-associated chondroitin sulfate proteoglycan (MCSP), glycoprotein A33, guanylate cyclase 2C and tumor-specific glycans, including mutants and analogues of the named antibodies, antibody fragments, proteins, peptides or low molecular weight organic ligands, wherein the targeting module according to the invention and the at least one further targeting module comprise different target cell binding domains, and identical tag-binding domains or tags. Such kits are suitable to avoid tumor escape from the immune system.

The term "target cell binding domain" also comprises soluble T cell receptors, which are composed of the alpha and beta or the gamma and delta chains of a T cell receptor (TCR), fragments or mutants thereof.

In preferred embodiments, the kit further comprises at least one further targeting module, wherein the at least one further targeting module comprises at least one target cell binding domain and a tag-binding domain or a tag, wherein the at least one target cell binding domain is an antibody or an antibody fragment that binds to surface antigens selected from the group comprising CD2, CD3, CD4, CD8, CD10, CD19, CD20, CD22, CD23, CD25, CD30, CD33, CD38, CD44, CD44v6 CD52, CD90, CD99, CD123, CD133, CD150 CD181, CD182, CD184, CD223, CD229, CD269 (BCMA), CD273, CD274, CD276, CD279, CD319, CD366 and CD371, interleukin receptors, especially preferred IL-8Ra (CXCR1), IL-8Rβ (CXCR2), IL-11Rα, IL-11Rβ, IL-13Ra1 and 2, CXCR4, c-Met, mesothelin, members of the epidermal growth factor receptor family, especially preferred ErbB1, ErbB2, ErbB3 or ErbB4; members of the tumor necrosis factor receptor superfamily, ephrins, ephrin receptors, especially preferred EphA1-10, EphA5 or EphB1-6, prostate specific antigens prostate stem cell antigen (PSCA) and prostate specific membrane antigen (PSMA), embryonic antigens carcinoembryonic antigen (CEA) and fetal acethylcholine receptor, members of the vascular endothelia growth factor family, epithelia cell adhesion molecule EpCAM, alphafetoprotein AFP, members of the intercellular adhesion molecule family, members of the mucin protein family, follicle stimulating hormone receptor (FSHR), the human high molecular weight-melanoma-associated antigen (HMW-MAA), folate binding protein FBP, folate receptors, somatostatin receptors, ligands of the NKG2D receptor, cytokine receptors, members of the epithelia glycoprotein family, diasialogangliosides, glypicans, G protein-coupled receptors, members of the carbonic anhydrase family, members of the carbohydrate antigen family, Notch ligands, melanoma-associated chondroitin sulfate proteoglycan (MCSP), glycoprotein A33, guanylate cyclase 2C and tumor-specific glycans, wherein the targeting module according to the invention and the at least one further targeting module comprise differenttarget cell binding domains, and identical tag-binding domains or tags.

In embodiments, the kit comprises
- a nucleic acid, vector or cell according to the invention and
- a vector or a cell comprising a nucleotide sequence encoding a switchable chimeric antigen receptor,
   wherein the switchable chimeric antigen receptor comprises three domains,
   wherein
   - the first domain is a tag-binding domain or tag,
   - the second domain is an extracellular hinge and a transmembrane domain and
   - the third domain is a signal transduction domain,
- at least one further targeting module,
wherein the tag-binding domain of the targeting module binds to the tag of the switchable chimeric antigen receptor or the tag of the targeting module binds to the tag-binding domain of the switchable chimeric antigen receptor,
wherein the targeting module according to the invention and the at least one further targeting module comprise different target cell binding domains, and identical tag-binding domains or tags.

In embodiments, the kit comprises one to three targeting modules, preferably one targeting module according to the invention and one or two further targeting modules.

In embodiments, the tag-binding domain or tag is present at the amino terminal end of the polypeptide that comprises the switchable CAR. Advantageously, locating the tag-binding domain or tag at the amino terminus permits the tag-binding domain or tag unhampered access to the targeting module that is bound to the target cell.

In embodiments, the kit further comprises an inducer, preferably tetracycline or a derivative of tetracycline, e.g. doxycycline.

In embodiments of the kit, the targeting module, the vector and/or the cell comprising a nucleotide sequence encoding a switchable chimeric antigen receptor are in the form of a pharmaceutical composition.

The pharmaceutical composition is preferably administered parenterally, particulary preferred intravenously. In embodiments, the pharmaceutical composition is present in a form suitable for intravenous administration. Preferably, the pharmaceutical composition is a solution, emulsion or suspension.

In embodiments, the pharmaceutical composition is an injectable buffered solution comprising between 1 µg/ml to 500 mg/ml of the targeting module and/or the nucleic acid and/or the vector and/or the cell according to the invention, preferably between 50 µg/ml to 50 mg/ml.

In embodiments, the pharmaceutical composition is an injectable buffered solution comprising between 1,000 ml⁻¹ to 5.10⁸ ml⁻¹ of the cell comprising a nucleotide sequence encoding a switchable chimeric antigen receptor, preferably between 1.10⁵ ml⁻¹ to 2.5.10⁷ ml⁻¹.

In embodiments, the pharmaceutical composition further comprises a pharmaceutically acceptable dilution agent or carrier. In further embodiments, the carrier is selected from water, an aqueous buffer solution, 0.9 % saline solution, 5 % glucose, 5 % xylitol, 0.3 % glycine solution, ringer solutions, amino acid solutions and a similar solvent. In further embodiments, the aqueous buffer solution is selected from an aqueous histidine, sodium succinate, sodium citrate, sodium phosphate or potassium phosphate buffered solution with a pH value in the range of pH 5.0 to pH 8.0, preferably with a pH value in the range of pH 6.0 to pH 7.0. In embodiments, the buffer solution has a buffer concentration in the range of 1 mmol/l (mM) to 500 mM, preferably in the range of 5 mM to 20 mM, especially preferred in the range of 5 mM to 10 mM. In further embodiments, the carrier comprises sodium chloride, preferably in a concentration in the range of 0 mM to 300 mM, especially preferred 150 mM. In embodiments, the pharmaceutical composition further comprises a stabilizer, preferably in a concentration in the range of 1 mM to 900 mM, especially preferred in the range of 10 mM and 600 mM. In embodiments, the stabilizer is sucrose, trehalose or L-methionine.

In further embodiments, the pharmaceutical composition further comprises pharmaceutically acceptable excipients. The term "pharmaceutically acceptable excipients" refers to compounds, which provide approximately physiological conditions and/or to increase the stability, such as agents for adjusting the pH value and buffering agents, agents for adjusting the toxicity and the like or agents to enable frozen storage of the pharmaceutical composition In embodiments, pharmaceutically acceptable excipients are selected from sodium acetate, sodium chloride, potassium chloride, calcium chloride, sodium lactate and polysorbate-80, preferably polysorbate-80 in the range of 0.0001 % (w/v) to 1 % (w/v), especially preferred in the range of 0.001 % (w/v) to 0.1 % (w/v).

In preferred embodiments, the pharmaceutical composition comprises the targeting module in a dosage quantity in the range of 25 µg/day to 100 mg/day, preferably dosage quantities in the range of 0.1 mg/day to 10 mg/day.

In further embodiments, the pharmaceutical composition is sterile. The pharmaceutical composition is sterilized by conventional well-known techniques including, but not limited, to sterile filtration.

In embodiments, the pharmaceutical composition is used for administration to a subject.

In embodiments, the pharmaceutical composition is lyophilized prior to storage or stored as solution at ambient temperature or below, including, but not limited to, frozen storage.

In embodiments, the pharmaceutical composition is reconstituted and/or diluted in an infusion and stabilizer solution prior to administration to a subject. The solutions used for reconstitution or infusion/stabilization may contain any of the components mentioned for the pharmaceutical composition or similar components.

In preferred embodiments, the pharmaceutical composition comprises the cell comprising a nucleotide sequence encoding a switchable chimeric antigen receptor in a dosage quantity in the range of 1.10⁷ to 1.10⁹ per administration, preferably dosage quantities in the range of 1.10⁸ to 5.10⁸ per administration.

Another aspect of the invention is the kit according to invention for use in a method for stimulating a chimeric antigen receptor mediated immune response in a mammal, preferably for use in the treatment of cancer, infectious disease or autoimmune disease.

In embodiments, the targeting module according to the invention, the nucleic acid, vector or cell according to the invention or the kit according to the invention is used for preparing a medication for therapeutic and/or diagnostic use in case of cancer, an infection or an autoimmune disease.

A further aspect of the invention is a method for stimulating a chimeric antigen receptor mediated immune response in a mammal, preferably a human, having cancer, an infectious or an autoimmune disease by administration of a targeting module, a cell or a vector, a pharmaceutical composition or a kit according to the invention. For therapeutic applications, a sterile pharmaceutical composition according to the invention or a sterile kit according to the invention, comprising a pharmacologically effective quantity of targeting module according to the invention and a vector or a cell comprising a nucleotide sequence encoding a switchable chimeric antigen receptor, is administered to a subject in order to treat the aforementioned illnesses.

In embodiments, the method for stimulating a chimeric antigen receptor mediated immune response in a mammal; preferably the method for treatment of cancer, infectious or autoimmune disease, further comprises the administration of an inducer, preferably tetracycline or a derivative of tetracycline, e.g. doxycycline.

In embodiments, an inducer, preferably tetracycline or a derivative of tetracycline, e.g. doxycycline; is administered orally or intravenously following administration of the nucleic acid, vector or cell according the invention.

In some embodiments, the method for stimulating a chimeric antigen receptor mediated immune response in a mammal, preferably the method for treatment of cancer, infectious or autoimmune disease, comprises the following steps:
a) administering to a mammal an effective amount of a targeting module according to the invention and
b) administering to the mammal an effective amount of a vector or a cell comprising a nucleotide sequence encoding a switchable chimeric antigen receptor,
   wherein the switchable chimeric antigen receptor comprises three domains,
   wherein
   - the first domain is a tag-binding domain or tag,
   - the second domain is an extracellular hinge and a transmembrane domain and
   - the third domain is a signal transduction domain,
   wherein the tag-binding domain of the targeting module binds to the tag of the switchable chimeric antigen receptor or the tag of the targeting module binds to the tag-binding domain of the switchable chimeric antigen receptor,
wherein the targeting module is administered to a mammal prior to, concurrent with or after the administration of the vector or cell.

In further embodiments, the method for stimulating a chimeric antigen receptor mediated immune response in a mammal, preferably the method for treatment of cancer, infectious or autoimmune disease, comprises the following steps:
a) administering to a mammal an effective amount of a targeting module comprising at least one target cell binding domain and a tag-binding domain or a tag,
   wherein the at least one target cell binding domain is an antibody, antibody fragment, a protein, a peptide or a low molecular weight organic ligand that binds to surface antigens selected from the group comprising CD2, CD3, CD4, CD8, CD10, CD19, CD20, CD22, CD23, CD25, CD30, CD33, CD38, CD44, CD44v6 CD52, CD90, CD99, CD123, CD133, CD150 CD181, CD182, CD184, CD223, CD229, CD269 (BCMA), CD273, CD274, CD276, CD279, CD319, CD366 and CD371, interleukin receptors, especially preferred IL-8Rα (CXCR1), IL-8Rβ (CXCR2), IL-11Rα, IL-11Rβ, IL-13Ra1 and 2, CXCR4, c-Met, mesothelin, members of the epidermal growth factor receptor family and mutants thereof, especially preferred ErbB1, ErbB2, ErbB3, ErbB4 or mutants thereof, members of the tumor necrosis factor receptor superfamily, ephrins, ephrin receptors, especially preferred EphA1-10, EphA5 or EphB1-6, prostate specific antigens prostate stem cell antigen (PSCA) and prostate specific membrane antigen (PSMA), embryonic antigens carcinoembryonic antigen (CEA) and fetal acethylcholine receptor, members of the vascular endothelia growth factor family, epithelia cell adhesion molecule EpCAM, alphafetoprotein AFP, members of the intercellular adhesion molecule family, members of the mucin protein family, follicle stimulating hormone receptor (FSHR), the human high molecular weight-melanoma-associated antigen (HMW-MAA), folate binding protein FBP, folate receptors, somatostatin receptors, ligands of the NKG2D receptor, cytokine receptors, members of the epithelia glycoprotein family, diasialogangliosides, glypicans, G protein-coupled receptors, members of the carbonic anhydrase family, members of the carbohydrate antigen family, Notch ligands, melanoma-associated chondroitin sulfate proteoglycan (MCSP), glycoprotein A33, guanylate cyclase 2C and tumor-specific glycans, including mutants and analogues of the named antibodies, antibody fragments, proteins, peptides or low molecular weight organic ligands, and
b) administering to the mammal an effective amount of a vector or a cell comprising a nucleotide sequence encoding a switchable chimeric antigen receptor,
   wherein the switchable chimeric antigen receptor comprises three domains,
   wherein
   - the first domain is a tag-binding domain or tag,
   - the second domain is an extracellular hinge and a transmembrane domain and
   - the third domain is a signal transduction domain,
   wherein the tag-binding domain of the targeting module binds to the tag of the switchable chimeric antigen receptor or the tag of the targeting module binds to the tag-binding domain of the switchable chimeric antigen receptor, and
c) administering to a mammal an effective amount of a targeting module according to the invention,
wherein the targeting module according to the invention and the at least one further targeting module comprise different target cell binding domains, and identical tag-binding domains or tags, wherein the method is carried out in the order of the steps a), b) and c).

The term "target cell binding domain" also comprises soluble T cell receptors, which are composed of the alpha and beta or the gamma and delta chains of a T cell receptor (TCR), fragments or mutants thereof.

In preferred embodiments, the method for stimulating a chimeric antigen receptor mediated immune response in a mammal, preferably the method for treatment of cancer, infectious or autoimmune disease, comprises the following steps:
a) administering to a mammal an effective amount of a targeting module comprising at least one target cell binding domain and a tag-binding domain or a tag,
   wherein the at least one target cell binding domain is an antibody, antibody fragment, a protein, a peptide or a low molecular weight organic ligand that binds to surface antigens selected from the group comprising CD2, CD3, CD4, CD8, CD10, CD19, CD20, CD22, CD23, CD25, CD30, CD33, CD38, CD44, CD44v6 CD52, CD90, CD99, CD123, CD133, CD150 CD181, CD182, CD184, CD223, CD229, CD269 (BCMA), CD273, CD274, CD276, CD279, CD319, CD366 and CD371, interleukin receptors, especially preferred IL-8Rα (CXCR1), IL-8Rβ (CXCR2), IL-11Rα, IL-11Rβ, IL-13Ra1 and 2, CXCR4, c-Met, mesothelin, members of the epidermal growth factor receptor family, especially preferred ErbB1, ErbB2, ErbB3 or ErbB4; members of the tumor necrosis factor receptor superfamily, ephrins, ephrin receptors, especially preferred EphA1-10, EphA5 or EphB1-6, prostate specific antigens prostate stem cell antigen (PSCA) and prostate specific membrane antigen (PSMA), embryonic antigens carcinoembryonic antigen (CEA) and fetal acethylcholine receptor, members of the vascular endothelia growth factor family, epithelia cell adhesion molecule EpCAM, alphafetoprotein AFP, members of the intercellular adhesion molecule family, members of the mucin protein family, follicle stimulating hormone receptor (FSHR), the human high molecular weight-melanoma-associated antigen (HMW-MAA), folate binding protein FBP, folate receptors, somatostatin receptors, ligands of the NKG2D receptor, cytokine receptors, members of the epithelia glycoprotein family, diasialogangliosides, glypicans, G protein-coupled receptors, members of the carbonic anhydrase family, members of the carbohydrate antigen family, Notch ligands, melanoma-associated chondroitin sulfate proteoglycan (MCSP), glycoprotein A33, guanylate cyclase 2C and tumor-specific glycans, and
b) administering to the mammal an effective amount of a vector or a cell comprising a nucleotide sequence encoding a switchable chimeric antigen receptor,
   wherein the switchable chimeric antigen receptor comprises three domains,
   wherein
   - the first domain is a tag-binding domain or tag,
   - the second domain is an extracellular hinge and a transmembrane domain and
   - the third domain is a signal transduction domain,
   wherein the tag-binding domain of the targeting module binds to the tag of the switchable chimeric antigen receptor or the tag of the targeting module binds to the tag-binding domain of the switchable chimeric antigen receptor, and
c) administering to a mammal an effective amount of a targeting module according to the invention,
wherein the targeting module according to the invention and the at least one further targeting module comprise different target cell binding domains, and identical tag-binding domains or tags, wherein the method is carried out in the order of the steps a), b) and c).

In preferred embodiments, the targeting module is administered on its own, preferably one hour to 2 days, more preferably 4 to 24 hours, prior to the administration of the vector or cell comprising a nucleotide sequence encoding a switchable chimeric antigen receptor and the targeting module according to the invention is administered, preferably in the range of 3 days to 30 days, after the administration of the vector or cell comprising a nucleotide sequence encoding a switchable chimeric antigen receptor.

In further embodiments, the method for stimulating a chimeric antigen receptor mediated immune response in a mammal, preferably the method for treatment of cancer, infectious or autoimmune disease, comprises the following steps:
a) administering to a mammal an effective amount of a vector or a cell comprising an inducible expression system, preferably selected from Tet-On, Tet-On Advanced transactivator, Tet-On 3G or a promoter responsive to NFAT; and a nucleotide sequence encoding a switchable chimeric antigen receptor, wherein the switchable chimeric antigen receptor comprises three domains, wherein
   - the first domain is a tag-binding domain or tag,
   - the second domain is an extracellular hinge and a transmembrane domain and
   - the third domain is a signal transduction domain, and
   a nucleotide sequence encoding a targeting module according to the invention, wherein the tag-binding domain of the targeting module binds to the tag of the switchable chimeric antigen receptor or the tag of the targeting module binds to the tag-binding domain of the switchable chimeric antigen receptor, and
b) administering to a mammal an effective amount of an inducer, preferably tetracycline or a derivative of tetracycline, e.g. doxycycline.

In embodiments, the inducible expression system induces the transcription of the nucleotide sequence encoding a switchable chimeric antigen receptor and/or a nucleotide sequence encoding a targeting module according to the invention, preferably the inducible expression system induces the transcription of the nucleotide sequence encoding a targeting module according to the invention.

In preferred embodiments, the method for stimulating a chimeric antigen receptor mediated immune response in a mammal, preferably the method for treatment of cancer, infectious or autoimmune disease, comprises the following steps:
a) administering to a mammal an effective amount of a targeting module comprising at least one target cell binding domain and a tag-binding domain or a tag,
   wherein the at least one target cell binding domain is an antibody, antibody fragment, a protein, a peptide or a low molecular weight organic ligand that binds to surface antigens selected from the group comprising CD2, CD3, CD4, CD8, CD10, CD19, CD20, CD22, CD23, CD25, CD30, CD33, CD38, CD44, CD44v6 CD52, CD90, CD99, CD123, CD133, CD150 CD181, CD182, CD184, CD223, CD229, CD269 (BCMA), CD273, CD274, CD276, CD279, CD319, CD366 and CD371, interleukin receptors, especially preferred IL-8Rα (CXCR1), IL-8Rβ (CXCR2), IL-11Rα, IL-11Rβ, IL-13Ra1 and 2, CXCR4, c-Met, mesothelin, members of the epidermal growth factor receptor family, especially preferred ErbB1, ErbB2, ErbB3 or ErbB4; members of the tumor necrosis factor receptor superfamily, ephrins, ephrin receptors, especially preferred EphA1-10, EphA5 or EphB1-6, prostate specific antigens prostate stem cell antigen (PSCA) and prostate specific membrane antigen (PSMA), embryonic antigens carcinoembryonic antigen (CEA) and fetal acethylcholine receptor, members of the vascular endothelia growth factor family, epithelia cell adhesion molecule EpCAM, alphafetoprotein AFP, members of the intercellular adhesion molecule family, members of the mucin protein family, follicle stimulating hormone receptor (FSHR), the human high molecular weight-melanoma-associated antigen (HMW-MAA), folate binding protein FBP, folate receptors, somatostatin receptors, ligands of the NKG2D receptor, cytokine receptors, members of the epithelia glycoprotein family, diasialogangliosides, glypicans, G protein-coupled receptors, members of the carbonic anhydrase family, members of the carbohydrate antigen family, Notch ligands, melanoma-associated chondroitin sulfate proteoglycan (MCSP), glycoprotein A33, guanylate cyclase 2C and tumor-specific glycans, and
b) administering to a mammal an effective amount of a vector or a cell comprising an inducible expression system, preferably selected from Tet-On, Tet-On Advanced transactivator, Tet-On 3G or a promoter responsive to NFAT; and a nucleotide sequence encoding a switchable chimeric antigen receptor, wherein the switchable chimeric antigen receptor comprises three domains, wherein
   - the first domain is a tag-binding domain or tag,
   - the second domain is an extracellular hinge and a transmembrane domain and
   - the third domain is a signal transduction domain, and
   a nucleotide sequence encoding a targeting module according to the invention, wherein the tag-binding domain of the targeting module binds to the tag of the switchable chimeric antigen receptor or the tag of the targeting module binds to the tag-binding domain of the switchable chimeric antigen receptor and,
c) administering to a mammal an effective amount of an inducer, preferably tetracycline or a derivative of tetracycline, e.g. doxycycline,
wherein the targeting module according to the invention and the at least one further targeting module comprise different target cell binding domains, and identical tag-binding domains or tags, wherein the method is carried out preferably in the order of the steps a), b) and c).

In further embodiments, the preceding embodiments can be combined.

The present invention will now be further explained by the following non-limiting figures and examples.
**Fig. 1** shows a schema of a switchable chimeric antigen receptor (CAR) with three domains, wherein the first domain is a tag-binding domain or a tag (exemplified as scFv), the second domain is an extracellular hinge and a transmembrane domain and the third domain is a signal transduction domain, and the optional fourth domain is a short peptide linker in the extracellular portion of the receptor.
**Fig. 2** shows results of flow-cytometry based in vitro binding assays of three different PD-L1 and/or PD-L2 binding TMs to PD-L1 expressing cells. Data are presented as mean ± SD of at least four independent experiments.

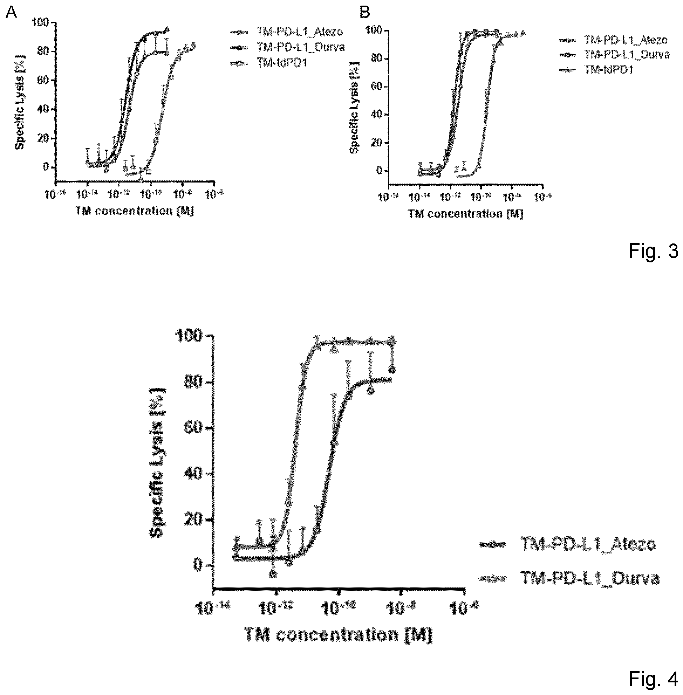

**Fig. 3** shows results of flow-cytometry based in vitro killing assays of switchable chimeric antigen receptor T cells redirected by three different PD-L1 and/or PD-L2 binding TMs to PD-L1 expressing cells. Target cell numbers were determined after incubation for A) 24 h and B) 48 h. Data are presented as mean ± SD of four independent experiments with individual T cell donors.
**Fig. 4** shows results of live-cell microscopy in vitro killing assays of switchable chimeric antigen receptor T cells redirected by two different anti-PD-L1 TMs to interferon-γ treated PC3-PSCA cells. Data are presented as mean ± SD of four independent experiments with individual T cell donors.

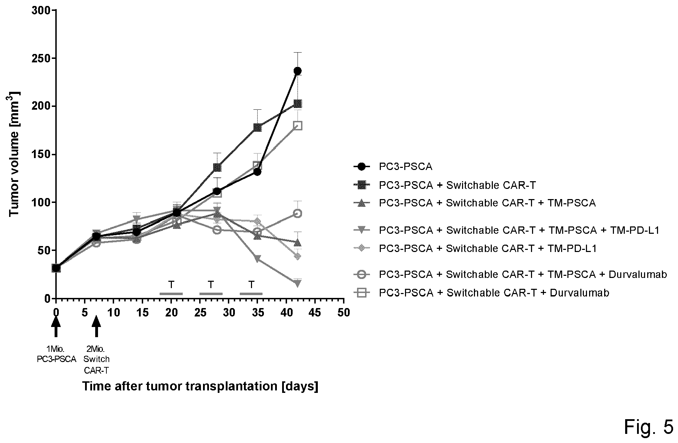

**Fig. 5** shows results of in vivo treatment of immunodeficient mice subcutaneously transplanted with PC3-PSCA tumors and treated with switchable chimeric antigen receptor T cells alone or in different combinations with an anti-PSCA TM, an anti-PD-L1 TM based on Durvalumab or Durvalumab. Data are presented as mean ± SEM of eleven mice per group.

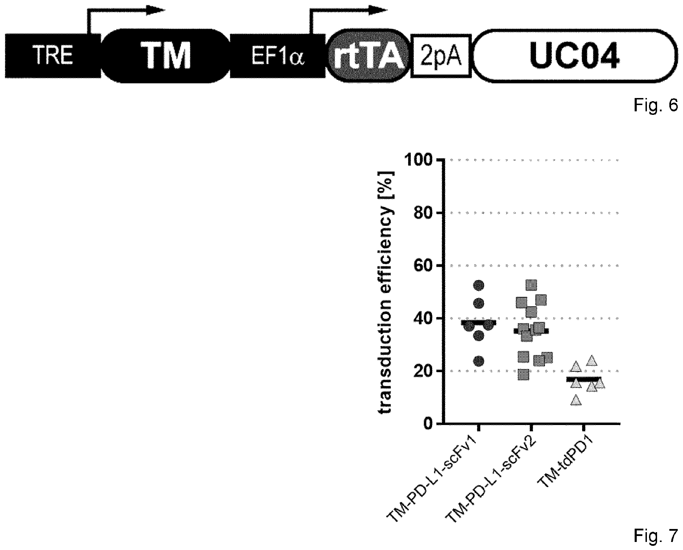

**Fig. 6** shows a schema of combined UniCAR and TetOn-inducible immune checkpoint specific targeting module expression cassette (TRE-tetracycline response element, EF1a-elongation factor 1a, rtTA-reverse tetracycline transactivator, iRFP-infrared fluorescent protein, eGFP-enhanced green fluorescent protein).
**Fig. 7** shows the frequency of gene modified primary human T cells obtained by lentiviral gene transfer: doxycycline- inducible gene expression of PD-L1 binding targeting modules under the control of a TetOn-cassette combined with a UniCAR. Data shown represent at least six batches obtained from individual human T cell donors.

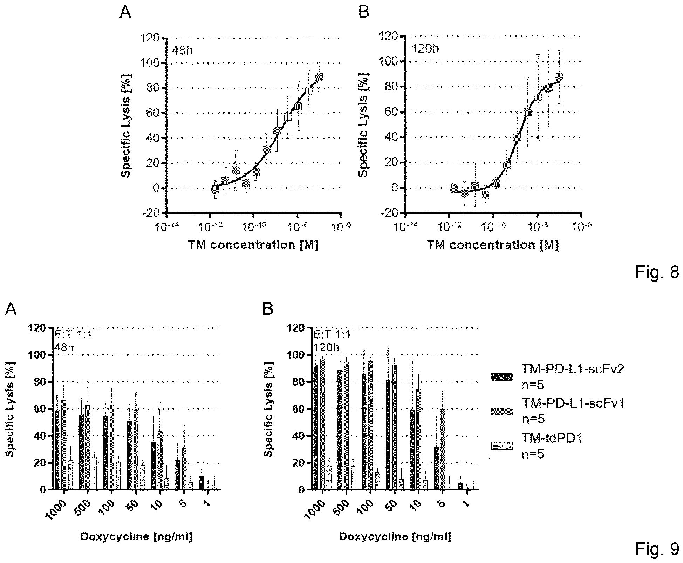

**Fig. 8** shows targeting module-dependent cytotoxic response of human T cells genetically engineered to express UniCAR and a PD-L1 and/or PD-L2 binding TM under control of a doxycycline-responsive TetOn-cassette **A**) after 48 h and **B**) after 120 h. Mean ± SD of results obtained with six independent human donors are shown.
**Fig. 9** shows flow cytometry-based cytotoxicity assay to assess lysis of PD-L1 positive Nalm-6 cells mediated by UniCAR-T modified to secrete PD-L1 and/or PD-L2 binding TM **A**) after 48 h and **B**) after 120 h. Data are presented as mean ± SD of at least four independent experiments with individual T cell donors, respectively.

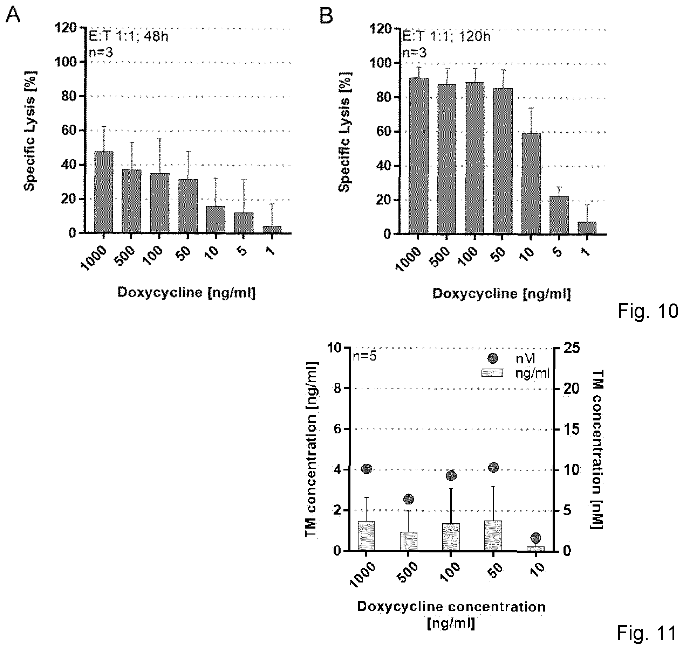

**Fig. 10** shows a cytotoxicity assay of human T cells expressing UniCAR and a PD-L1 and/or PD-L2 binding TM under control of doxycycline-responsive TetOn-cassette against intrinsically PD-L1 up-regulating PC3-PSCA cells **A**) after 48 h and **B**) after 120 h. Data are shown as mean ± SD of three repetitive experiments with individual T cell donors, respectively.
**Fig. 11** shows the quantification of TM-PD-L1-scFv1 concentrations secreted by gene-engineered human T cells upon doxycycline induction. Data are presented as mean ± SD of five repetitive experiments with five individual T cell donors.

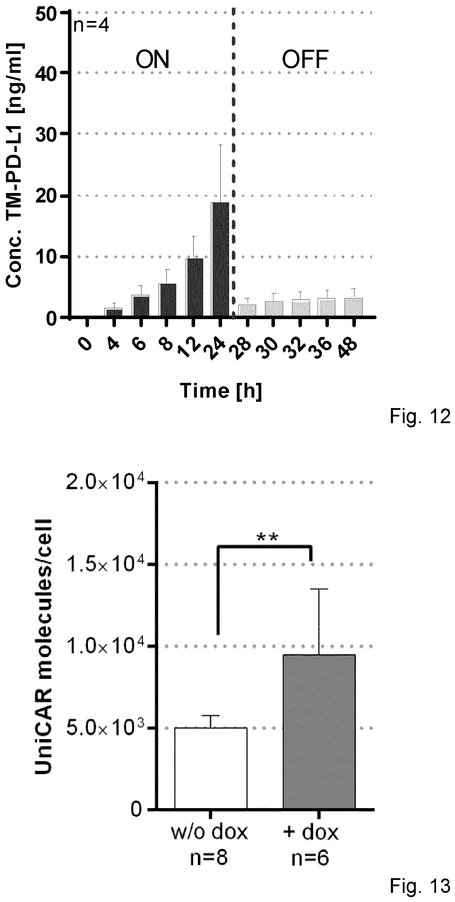

**Fig. 12** shows kinetics of On/Off switch of doxycycline-inducible TM-PD-L1-scFv1 secretion. Data are presented as mean ± SD of four individual T cell donors and experiments.
**Fig. 13** shows the quantification of UniCAR molecules on the surface in dependence on activation by doxycycline-induced expression of TM-PD-L1-scFv1. Mean ± SD of three individual T cell donors are shown, statistical significance was calculated by paired t-test, ** p < 0.01.

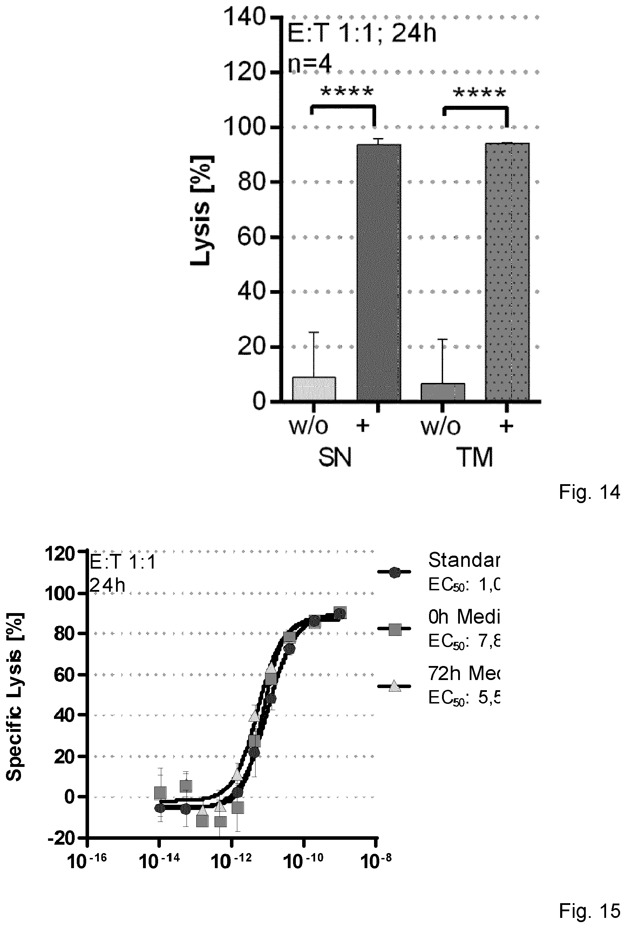

**Fig. 14** shows that basal expression of TM-PD-L1-scFv under control of Tet On-cassette is below threshold for cytotoxicity induction. Human T cells genetically modified to express a switchable chimeric antigen receptor and to secret TM-PD-L1-scFv in response to doxycycline were cultured in standard culture medium in absence (SN w/o) or presence of 1000 ng/ml doxycycline (SN +) for 72 hours. Supernatant was harvested, diluted 1:5, and added to Nalm-6-PD-L1 target cells and human T cells genetically modified to express a switchable chimeric antigen receptor alone. As additional controls, cell mix was incubated with 1 nM purified TM-PD-L1-scFv1 (+TM) or plain cell culture medium (w/o TM). After incubation for 24 h, lysis of target cells was analyzed using flow cytometry. Data presented as mean ± SD of four individual T cell donors. Statistical significance was calculated by One-way ANOVA; **** p<0.0001.
**Fig. 15** shows stability of TM-PD-L1-scFv1 in culture medium at 37 °C for 72 h followed by flow cytometry-based cytotoxicity assay.

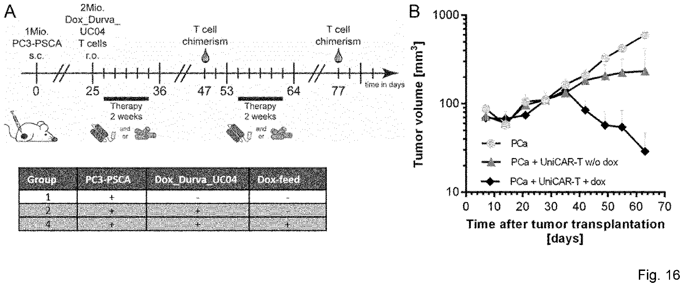

**Fig. 16** shows the in vivo efficacy of human T cells genetically engineered to express UniCAR and TM-PD-L1-scFv under control of TetOn-cassette in a xenotransplantation model of prostate cancer (PCa). **A**) Schematic illustration of the experimental set-up. **B**) Measured tumor volume plotted as a function of time as mean ± SEM of at least nine mice.

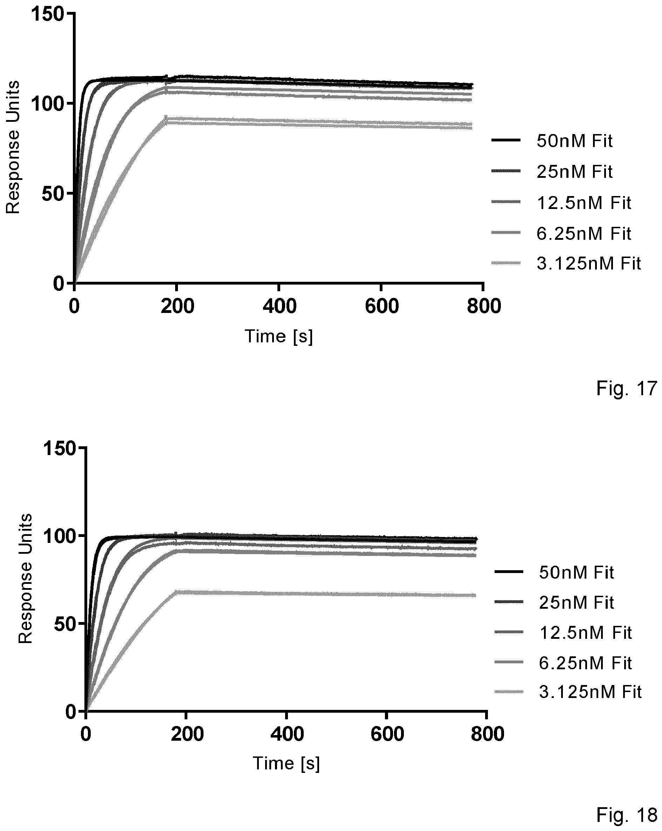

**Fig. 17** shows the surface plasmon resonance (SPR) measurements of the binding of TM-PD-L1-scFv1 to recombinant human Fc-tagged PD-L1.
**Fig. 18** shows the surface plasmon resonance (SPR) measurements of the binding of TM-PD-L1-scFv2 to recombinant human Fc-tagged PD-L1.

### Production of the switchable CAR cell

The immune cells can be genetically engineered to express switchable CARs and/or inducible TMs directed against PD-L1 and/or PD-L2 by various methods. A polynucleotide vector encoding the switchable CAR and all necessary elements to ensure its expression in the genetically engineered immune cell is transferred into the immune cell. The transfer of the vector can be performed by electroporation or transfection of nucleic acids or the help of viral vector systems like adeno-, adeno-associated, retro-, foamy- or lentiviral viral gene transfer.

The lentiviral gene transfer is applied for stable expression of switchable CARs in immune cells by first constructing a lentiviral vector encoding for a selected switchable CAR. The lentiviral vector is pLVX-EF1alpha UniCAR 28/ζ (Clontech, Takara Bio Group), in which the lentiviral parts of the vector are derived from the human immunodeficiency virus (HIV) and the MSC/IRES/ZxGreenl portion was replaced by the switchable CAR construct.

The lentiviral particles are produced by transient transfection of human embryonal kidney (HEK) 293T (ACC 635) cells with the switchable CAR encoding lentiviral vector plasmid and cotransfection with a group specific antigen (gag) and Polymerase (pol) encoding plasmid (psPAX2) plus a plasmid encoding for an envelope (pMD2.G). After transfection, the packaging plasmid expresses Gag and Pol protein of HIV-1. The plasmid MD2.G encodes the glycoprotein of the vesicular stomatitis virus (VSV-G). VSV-G protein is used to lentiviral vectors to transduce a broad range of mammalian cells. Various envelopes from different virus species can be utilized for this purpose. Lentiviral vectors can successfully pseudotype with the envelope glycoproteins (Env) of amphotropic murine leukemia virus (MLV) or the G protein of vesicular stomatitis virus (VSV-G), a modified envelope of the prototypic foamy virus (PFV) or chimeric envelope glycoprotein variants derived from gibbon ape leukemia virus (GaLV) and MLV.

Supernatants from transfected HEK293T cells are harvested 24 h to 96 h after transfection and virus particles are concentrated from the supernatant by ultracentrifugation or other methods. For lentiviral transduction of immune cells, *peripheral blood mononuclear cells* (PBMC) or isolated T cells are activated with mab specific for the CD3 complex, e.g. clone OKT3 or UCHT1, either given in solution or coated to plastic cell culture dishes or magnetic beads or a biodegradable polymer matrix. Activation of PBMC or isolated T cells is further enhanced by stimulating costimulatory pathways with mabs or ligands specific for CD27, CD28, CD134 or CD137 either alone or in combinations coated to plastic cell culture dishes or magnetic beads or a biodegradable polymer matrix and the supply with exogenous recombinant cytokines like interleukin (IL)-2, IL-7, IL-12, IL-15 and IL-21. Concentrated or non-concentrated virus particles are added to PBMC or T cell cultures 24 h to 96 h after initial administration of activating CD3 specific antibodies and/or antibodies specific for costimulatory receptors CD27, CD28, CD134 or CD137 and/or recombinant cytokines as single or multiple doses. T cell electroporation, transduction and expansion may be performed in open cell culture systems by manual handling or in closed partially or fully automated systems.

Stable transduction of T cells may be determined by flow cytometry after staining with tag-containing molecules for surface expression of switchable CARs or mabs directed against the fourth domain of switchable CARs from day 3 onwards after final administration of virus supernatant. Switchable CAR transduced T cells can be propagated *in vitro* by culturing them under supply of recombinant cytokines and activating anti-CD3 mabs.

In case the switchable CAR harbors the optional fourth domain, a peptide sequence forming a linear epitope for a mab, immune cells genetically modified to express switchable CARs can be specifically propagated *in vitro* by coating a mab or antibody fragments thereof binding to the fourth switchable CAR domain to the surface of culture dishes or to beads of any kind or a biodegradable polymer matrix, which are added to the cell culture at a defined ratio. The binding of surface-coated mabs to the switchable CAR peptide domain induces cross-linkage of cell-surface expressed switchable CARs and formation of an immune synapse, which leads to the activation of signal pathways specifically triggered by the signal domain of the switchable CAR. Depending on the signal pathways induced, this may lead to enhance proliferation and sustained resistance against activation-induced cell death of the switchable CAR carrying immune cells and therefore enrichment of switchable CAR genetically modified immune cells in a mixed population.

The optional fourth domain, a peptide sequence forming a linear epitope for a mab, can be further utilized to enrich and purify switchable CAR expressing immune cells from mixed populations. Enrichment and purification is performed with the help of a mab or antibody fragment thereof binding to the fourth switchable CAR domain to either mark switchable CAR expressing cells for cell sorting or to transiently link the switchable CAR expressing immune cell to small particles, which can be utilized for cell isolation. In one aspect, switchable CAR engrafted immune cells are incubated with the mab recognizing the fourth domain. Next, magnetic beads are added, which are conjugated with antibodies or fragments thereof directed against the species- and isotype specific heavy and light chains of the mab binding to the optional fourth domain. Thus, switchable CAR expressing immune cells and magnetic beads are linked and are trapped and separated from other immune cells in a magnetic field.

### Design of targeting modules according to the invention

In one example, the PD-L1 and/or PD-L2 binding TM comprises an scFv of Atezolizumab according to SEQ ID No. 57, comprising SEQ IDs No. 6 and 7 connected by a linker and the human La epitope E5B9 according to SEQ ID No. 14 (TM-PD-L1-scFv1).

In further examples, the PD-L1 and/or PD-L2 binding TM comprises an scFv of Durvalumab according to SEQ ID No. 58, comprising SEQ IDs No. 8 and 9 connected by a linker and the human La epitope E5B9 according to SEQ ID No. 14 (TM-PD-L1-scFv2).

In further examples, the PD-L1 and/or PD-L2 binding TM is bivalent and comprises two PD-1 extracellular domains, each of which is a fragment of SEQ ID No. 3 and the human La epitope E5B9 according to SEQ ID No. 14 (TM-tdPD1). TM-tdPD-1 is encoded by SEQ ID No. 59.

### Characterization of the functionality of the targeting modules according to the invention

The functionality of the PD-L1 and/or PD-L2 binding TMs can be confirmed in binding assays to human and murine PD-L1 using surface plasmon resonance as well as in cell-based cytotoxicity assays.

The results of flow-cytometry based in vitro binding assays of three different PD-L1 and/or PD-L2 binding TMs to Nalm-6 cells transduced to express PD-L1 are shown in Fig. 2. Data are presented as mean ± SD of at least four independent experiments.
The results of flow-cytometry based in vitro killing assays of switchable chimeric antigen receptor T cells (SEQ ID No. 60) redirected by three different PD-L1 and/or PD-L2 binding TMs to Nalm-6 cells transduced to express PD-L1 are shown in Fig. 3. Effector to target ratio (E:T) was 1:1. Target cell numbers were determined after incubation for A) 24 h and B) 48 h. Data are presented as mean ± SD of four independent experiments with individual T cell donors.
The results of live-cell microscopy based in vitro killing assays of switchable chimeric antigen receptor T cells (SEQ ID No. 60) redirected by two different anti-PD-L1 TMs to interferon-γ treated PC3-PSCA cells are shown in Fig. 4. Effector to target ratio (E:T) was 1:1. Target cell numbers were determined after incubation for 96 h. Data are presented as mean ± SD of four independent experiments with individual T cell donors.
The results of in vivo treatment of immunodeficient mice subcutaneously transplanted with PC3-PSCA tumors and treated with switchable chimeric antigen receptor T cells (SEQ ID No. 60) alone or in different combinations with an anti-PSCA TM, an anti-PD-L1 TM based on Durvalumab or Durvalumab are shown in Fig. 5. The switchable chimeric antigen receptor T cells were injected intravenously at the indicated time point. The TMs were injected twice daily intraperitoneally and Durvalumab was injected once intraperitoneally during each of the indicated treatment periods (T). Data are presented as mean ± SEM of eleven mice per group.

### Design of a combined switchable CAR and inducible, targeting module expression cassette (Fig. 6)

The lentiviral gene expression plasmid contains an EF1a promoter for continuous polycistronic transcription of rtTA and UniCAR, separated by a Thosea asigna virus derived peptide cleavage site (2pA). In addition, a PD-L1 and/or PD-L2 binding targeting module (TM) is under the control of a TRE3G promoter, which will only be activated by binding of rtTA in presence of doxycycline. This arrangement allows a doxycyclin-dependent inducible and reversible gene-expression.

### Characterization of the functionality of the combined switchable CAR and inducible, targeting module expression cassette

The frequency of gene modified primary human T cells obtained by lentiviral gene transfer is shown in Fig. 7. T cells isolated from voluntary healthy human donors were transduced with lentiviral supernatant transferring genetic information for doxycycline-inducible gene expression of PD-L1 and/or PD-L2 binding targeting modules (TM-PD-L1-scFv-1, TM-PD-L1-scFv2 and TM-tdPD1) under the control of a TetOn-cassette combined with UniCAR. Transduction efficiency at day 14 after transduction was analyzed via immunofluorescence staining of surface displayed UniCAR using a primary mouse a7B6-mAb (10µg/ml) directed against the extracellular E7B6-tag integrated into the UniCAR hinge. An AlexaFluor 647 conjugated goat-a-mouse Ab was used as secondary Ab.

The TM-dependent cytotoxic response of human T cells genetically engineered to express UniCAR and the TM according to the invention under control of a doxycycline-responsive TetOn-cassette is shown in Fig. 8. T cells isolated from voluntary healthy human donors were genetically modified to express UniCAR and a PD-L1 and/or PD-L2 binding targeting module, in particular TM-PD-L1-scFv2, under the control of a TetOn-cassette. The ability of the UniCAR modified cells to mediate TM-dependent cytotoxic response against target cancer cells was evaluated in a flow-cytometry based cytotoxicity assay. Nalm-6-PSCA cells were cultured with human UniCAR expressing T cells at a total T cell to target cell ratio of 1:1 in the presence of increasing concentrations of a PSCA binding TM comprising a PSCA binding domain according to EP 2 990 416 B1; and the human La epitope E5B9 according to SEQ ID No. 14. At indicated time points the number of living target cells was determined. Specific lysis was calculated relative toa control without addition of TM.

The results of the cytotoxicity assay to assess lysis of PD-L1 positive Nalm-6 cells mediated by UniCAR-T modified to secrete PD-L1 specific TM are shown in Fig. 9. Nalm-6-PD-L1 target cells were cultured with genetically modified human T cells in a total T cell to target cell (E:T) ratio of 1:1 in presence of decreasing concentrations of doxycycline ranging from 1,000to 1 ng/ml. T cells were engineered to express UniCAR and a doxycycline-responsive TetOn-cassette to induce expression and secretion of TM-PD-L1-scFv-1, TM-PD-L1-scFv2 or TM-tdPD1 in presence of doxycycline. Specific lysis of target cells was analyzed after A) 48h and B) 120h of incubation and normalized to background lysis without doxycycline.

Fig. 10 shows the results of the cytotoxicity assay of human T cells expressing UniCAR and a PD-L1 specific TM under control of doxycycline-responsive TetOn-cassette against intrinsically PD-L1 up-regulating PC3-PSCA cells. PC3-PSCA cells were treated with 25 ng/ml IFN-γ to induce PD-L1 up-regulation. Subsequently, PD-L1 presenting PC3-PSCA were incubated with genetically modified T cells expressing UniCAR and a PD-L1-specific TM, in particular TM-PD-L1-scFv1, under the control of doxycycline inducible TetOn-cassette in a total T cell to target cell (E:T) ratio of 1:1 for A) 48h and B) 120h. Doxycycline was added in decreasing concentrations from 1000 to 1 ng/ml and specific lysis was calculated relative to lysis of target cells without doxycycline.

The quantification of TM-PD-L1-sFv1 concentrations secreted by gene-engineered human T cells upon doxycycline induction (Fig. 11) was carried out by incubating PD-L1 positive target cells and human T cells engineered to express UniCAR and a TetOn-cassette for doxycycline inducible TM-PD-L1-scFv1 in a total T cell to target cell (E:T) ratio of 1:1 with decreasing concentration of doxycycline for 120 h. Cell-free supernatant was harvested and secreted TM-PD-L1-scFv1 concentrations were analyzed by ELISA. Doxycycline concentration is blotted against TM concentration in ng/ml and resulting molar concentration, respectively.

The kinetics of On/Off switch of doxycycline-inducible TM-PD-L1-scFv1 secretion is shown in Fig. 12. For each indicated time point of the ON-phase 1x10⁵ human T cells genetically engineered to express UniCAR and TM-PD-L1-scFv1 under the control of doxycycline (dox) inducible TetOn-cassette were incubated in 500 µl culture medium in the presence of 1000 ng/ml doxycycline. Likewise, for the OFF-phase a pool of the engineered T cells were first cultured with the same amount of doxycycline for 24 h, washed thoroughly afterwards and cultured as described for the ON-phase, but without addition of doxycycline for additional 4, 6, 8,12 and 24h. For each time point analyzed, cell-free supernatant was harvested and TM-PD-L1-scFv1 levels were quantified using ELISA.

The quantification of UniCAR molecules on the surface in dependence on the activation by doxycycline-induced expression of TM-PD-L1-scFv1 is shown in Fig. 13. The number of UniCAR surface molecules on engineered T cells was quantified using the calibration curve generated with the DAKO QIFIKIT®. Immunofluorescence staining of gene-engineered T cells was performed using anti7B6-mAb for UniCAR detection and AlexaFluor 647 goat-a-mouse Ab as secondary Ab was applied to pre-stained T cells but calibration beads as well. Genetically engineered human T cells expressing UniCAR and TetOn-cassette for doxycycline-inducible TM-PD-L1-scFv1 were cultured in absence or presence of 1,000 ng/ml doxycycline for 24 hours.

Fig. 14 shows that the basal expression of TM-PD-L1-scFv under control of TetOn-cassette is below threshold for cytotoxicity induction. Human T cells genetically modified to express UniCAR and to secret TM-PD-L1-scFv in response to doxycycline were cultured in standard culture medium in absence (SN w/o) or presence of 1,000 ng/ml doxycycline (SN +) for 72 hours. Supernatant was harvested, diluted 1:5, and added to Nalm-6-PD-L1 target cells and human T cells genetically modified to express UniCAR alone. As additional controls, cell mix was incubated with 1 nM purified TM-PD-L1-scFv1 (+TM) or plain cell culture medium (w/o TM) instead. After incubation for 24 h lysis of target cells was analyzed using flow cytometry.

In order to verify stability of TM-PD-L1-scFv1 at 37 °C, purified TM was incubated in culture medium at 37 °C for 72 h and potency was analyzed in a standard flow cytometry-based cytotoxicity assay using human T cells genetically modified to express UniCAR alone and Nalm-6-PD-L1 target cell in a total T cell to target cell (E:T) ratio of 1:1 for 24 h (Fig. 15).

The in vivo efficacy of human T cells genetically engineered to express UniCAR and TM-PD-L1-scFv under control of TetOn-cassette in a xenotransplantation model of prostate cancer (PCa) is shown in Fig. 16. The mice were transplanted subcutaneously with 1x10⁶ tumor cells (PC3-PSCA) (Fig. 16 A). Twenty-five days later 2 million T cells (28.7% transduced) were administered and doxycycline-feed was started the same day. Feeding of doxycycline was continued for two weeks in a row with a break of two days in between. Second treatment cycle was started two weeks later and performed in the same way. The tumor volume was measured once a week with a digital caliper and is blotted as a function of time as mean ± SEM of at least 9 mice (Fig. 16 B).

### Cited non-patent literature

Brudno JN, Kochenderfer JN (2016) Toxicities of chimeric antigen receptor T cells: recognition and management. Blood 127, 3321-3330.
Carpenito C, Milone MC, Hassan R, Simonet JC, Lakhal M, Suhoski, MM, Varela-Rohena A, Haines KM, Heitjan D, Albelda SM, Carrol RG, Riley JL, Pastan, I, June CH (2009) Control of large, established tumor xenografts with genetically retargeted human T cells containing CD28 and CD137 domains. Proc. Natl. Acad. Sci. USA. 106 (9), 3360-3365.
Cartellieri M, Bachmann M, Feldmann A, Bippes C, Stamova S, Wehner R, Temme A, Schmitz M (2010) Chimeric Antigen Receptor-Engineered T Cells for Immunotherapy of Cancer J. Biomed. Biotechnol. Article ID 956304, doi: 10.1155/2010/956304.
Cartellieri M, Feldmann A, Koristka S, Arndt C, Loff S, Ehninger A, von Bonin M, Bejestani EP, Ehninger G, Bachmann MP (2016) Switching CAR T cells on and off: a novel modular platform for retargeting of T cells to AML blasts. Blood cancer J. 6 (8), e458.
Fedorov VD, Themeli M, Sadelain M (2013) PD-1- and CTLA-4-based inhibitory chimeric antigen receptors (iCARs) divert off-target immunotherapy responses. Sci. Transl. Med. 5 (215), 215ra172.
Finney HM, Akbar AN, Lawson AD (2004) Activation of resting human primary T cells with chimeric receptors: costimulation from CD28, inducible costimulator, CD134, and CD137 in series with signals from the TCR zeta chain. J. Immunol. 172 (1), 104-113.
Frigault MJ, Lee J, Basil MC, Carpenito C, Motohashi S, Scholler J, Kawalekar OU, Guedan S, McGettigan SE, Posey AD Jr, Ang S, Cooper LJ, Platt JM, Johnson FB, Paulos CM, Zhao Y, Kalos M, Milone MC, June CH (2015) Identification of chimeric antigen receptors that mediate constitutive or inducible proliferation of T cells. Cancer Immunol. Res. 3 (4), 356-367.
Gade TP, Hassen W, Santos E, Gunset G, Saudemont A, Gong MC, Brentjens R, Zhong XS, Stephan M, Stefanski J, Lyddane C, Osborne JR, Buchanan IM, Hall SJ, Heston WD, Rivière I, Larson SM, Koutcher JA, Sadelain M (2005) Targeted elimination of prostate cancer by genetically directed human T lymphocytes. Cancer Res. 65 (19), 9080-9088.
Gong MC, Latouche JB, Krause A, Heston WDW, Bander NH, Sadelain M (1999) Cancer patient T cells genetically targeted to prostate-specific membrane antigen specifically lyse prostate cancer cells and release cytokines in response to prostate-specific membrane antigen. Neoplasia. 1 (2), 123-127.
Grosser R, Cherkassky L, Chintala N, Adusumilli PS (2019) Combination Immunotherapy with CAR T Cells and Checkpoint Blockade for the Treatment of Solid Tumors. Cancer Cell. 36 (5), 471-482.
Grupp SA, Laos M, Barrett D, Aplenc R, Porter DL, Rheingold SR, Teachey DT, Chew A, Hauck B, Wright JF, Milone MC, Levine, BL, June CH (2013) Chimeric Antigen Receptor-Modified T Cells for Acute Lymphoid Leukemia. N. Engl. J. Med. 368, 1509-1518.
Guedan S, Posey AD, Shaw C, Wing A, Da T, Patel PR, McGettigan SE, Casado-Medrano V, Kawalekar OU, Uribe-Herranz M, Song D, Melenhorst JJ, Lacey SF, Scholler J, Keith B, Young RM, June CH (2018) Enhancing CAR T cell persistence through ICOS and 4-1BB costimulation. JCI Insight. 3(1), 96976.
Guedan S, Madar A, Casado-Medrano V, Shaw CE, Wing A, Liu F, Young RM, June CH, Posey AD (2020) Single residue in CD28-costimulated CAR T cells limits long-term persistence and antitumor durability. J Clin Invest. 133215.
Hombach AA, Abken H (2011) Costimulation by chimeric antigen receptors revisited the T cell antitumor response benefits from combined CD28-OX40 signalling. Int. J. Cancer. 129 (12), 2935-2944.
Hombach AA, Abken H (2013) Of chimeric antigen receptors and antibodies: OX40 and 41BB costimulation sharpen up T cell-based immunotherapy of cancer. Immunotherapy. 5 (7), 677-681.
Hombach AA, Kofler D, Hombach A, Rappl G, Abken H (2007) Effective proliferation of human regulatory T cells requires a strong costimulatory CD28 signal that cannot be substituted by IL-2. J. Immunol. 179 (11), 7924-7931.
Hombach A, Sent D, Schneider C, Heuser C, Koch D, Pohl C, Seliger B, Abken H (2001) T-cell activation by recombinant receptors: CD28 costimulation is required for interleukin 2 secretion and receptor-mediated T-cell proliferation but does not affect receptor-mediated target cell lysis. Cancer Res. 61 (5), 1976-1982.
Kagoya Y, Tanaka S, Guo T, Anczurowski M, Wang CH, Saso K, Butler MO, Minden MD, Hirano N (2018) A novel chimeric antigen receptor containing a JAK-STAT signaling domain mediates superior antitumor effects. Nat Med. 24(3), 352-359.
Kershaw MH, Westwood JA, Parker LL, et al. (2006) A phase I study on adoptive immunotherapy using gene-modified T cells for ovarian cancer. Clin. Cancer Res. 12 (20), 6106-6115.
Lamers CH, Sleijfer S, Willemsen RA, Debets R, Kruit WHJ, Gratama JW, Stoter G (2004) Adoptive immuno-gene therapy of cancer with single chain antibody [scFv(lg)] gene modified T lymphocytes. J. Biol. Regul. Homeost. Agents. 18 (2), 134-140.
Lamers CH, Sleijfer S, Vulto AG, Kruit WH, Kliffen M, Debets R, Gratama JW, Stoter G, Oosterwijk E (2006) Treatment of metastatic renal cell carcinoma with autologous T-lymphocytes genetically retargeted against carbonic anhydrase IX: first clinical experience. J. Clin. Oncol. 24, e20 - e22.
Maher J, Brentjens RJ, Gunset G, Rivière I, Sadelain M (2002) Human T-lymphocyte cytotoxicity and proliferation directed by a single chimeric TCRzeta /CD28 receptor. Nat. Biotechnol. 20 (1), 70-75.
Maude SL, Barrett DM, Teachey DT, Grupp SA (2014) Managing Cytokine Release Syndrome Associated with Novel T Cell-Engaging Therapies. Cancer J. 20 (2), 119-122.
Milone MC, Fish JD, Carpenito C, Carroll RG, Binder GK, Teachey D, Samanta M, Lakhal M, Gloss B, Danet-Desnoyers G, Campana D, Riley JL, Grupp SA, June CH (2009) Chimeric receptors containing CD137 signal transduction domains mediate enhanced survival of T cells and increased antileukemic efficacy in vivo. Mol. Ther. 17 (8), 1453-1464.
Morgan RA, Yang JC, Kitano M, Dudley ME, Laurencot CM, Rosenberg SA (2010) Case Report of a Serious Adverse Event Following the Administration of T Cells Transduced With a Chimeric Antigen Receptor Recognizing ERBB2. Mol. Ther. 18, 843-851.
Pinthus JH, Waks T, Kaufman-Francis K, Schindler DG, Harmelin A, Kanety H, Ramon J, Eshhar Z (2003) Immuno-gene therapy of established prostate tumors using chimeric receptor-redirected human lymphocytes. Cancer Res. 63 (10), 2470-2476.
Pinthus JH, Waks T, Malina V, Kaufman-Francis K, Harmelin A, Aizenberg I, Kanety H, Ramon J, Eshhar Z (2004) Adoptive immunotherapy of prostate cancer bone lesions using redirected effector lymphocytes. J Clin Invest. 114(12), 1774-1781.
Reinke AW, Grant RA, Keating AE (2010) A Synthetic Coiled-Coil Interactome Provides Heterospecific Modules for Molecular Engineering. JACS 132, 6025-6031.
Sotillo E, Barrett DM, Black K., Bagashev A, Oldridge D, Wu G, Sussman R, Lanauze C, Ruella M, Gazzara MR, Martinez NM, Harrington CT, Chung EY, Perazzelli J, Hofmann TJ, Maude SL, Raman P, Barrera A, Gill S, Lacey SF, Melenhorst JJ, Allman D, Jacoby E, Fry T, Mackall C, Barash Y, Lynch KW, Maris JM, Grupp SA, Thomas-Tikhonenko A (2015) Convergence of Acquired Mutations and Alternative Splicing of CD19 Enables Resistance to CART-19 Immunotherapy. Cancer Discov. 5, 1282-1295.
Titov A, Petukhov A, Staliarova A, Motorin D, Bulatov E, Shuvalov O, Soond SM, Piacentini M, Melino G, Zaritskey A, Barlev NA (2018) The biological basis and clinical symptoms of CAR-T therapy-associated toxicites. Cell Death Dis 9, 897.
Töpfer K, Cartellieri M, Michen S, Wiedemuth R, Müller N, Lindemann D, Bachmann M, Füssel M, Schackert G, Temme A (2015) DAP12-based activating chimeric antigen receptor for NK cell tumor immunotherapy. J. Immunol. 194 (7), 3201-3212.
Wang J, Jensen M, Lin Y, Sui X, Chen E, Lindgren CG, Till B, Raubitschek A, Forman SJ, Qian X, James S, Greenberg P, Riddell S, Press OW (2007) Optimizing adoptive polyclonal T cell immunotherapy of lymphomas, using a chimeric T cell receptor possessing CD28 and CD137 costimulatory domains. Hum. Gene Ther. 18 (8), 712-725.
Zhang T, Lemoi BA, Sentman CL (2005) Chimeric NK-receptor-bearing T cells mediate antitumor immunotherapy. Blood. 106 (5), 1544-1551.
Zhang T, Barber A, Sentman CL (2006) Generation of antitumor responses by genetic modification of primary human T cells with a chimeric NKG2D receptor. Cancer Res. 66 (11), 5927-5933.
Zhao Y, Wang QJ, Yang S, Kochenderfer JN, Zheng Z, Zhong X, Sadelain M, Eshhar Z, Rosenberg SA, Morgan RA (2009) A herceptin-based chimeric antigen receptor with modified signaling domains leads to enhanced survival of transduced T lymphocytes and antitumor activity. J. Immunol. 183 (9), 5563-5574.

### Reference signs

- **1**: first domain, a tag-binding domain or tag.
- **2**: second domain, an extracellular hinge and a transmembrane domain.
- **3**: third domain, a signal transduction domain.
- **4**: optional fourth domain, a short peptide linker.

## Claims

1. A targeting module comprising
i) at least one PD-L1 and/or PD-L2 binding domain,
ii) a tag-binding domain or a tag,
for use in a method for stimulating a chimeric antigen receptor mediated immune response in a mammal,
wherein the targeting module is administered in combination with a vector or a cell comprising a nucleotide sequence encoding a switchable chimeric antigen receptor,
wherein the switchable chimeric antigen receptor comprises three domains,
wherein
- the first domain is a tag-binding domain or a tag,
- the second domain is an extracellular hinge and a transmembrane domain and
- the third domain is a signal transduction domain,
wherein the tag-binding domain of the targeting module binds to the tag of the switchable chimeric antigen receptor or the tag of the targeting module binds to the tag-binding domain of the switchable chimeric antigen receptor.

2. The targeting module according to claim 1 is monovalent or bivalent.

3. The targeting module according to claim 1 or 2, wherein the PD-L1 and/or PD-L2 binding domain is BMS-936559, Atezolizumab, Durvalumab, Avelumab, 3F2.1 or a fragment thereof.

4. The targeting module according to one of the claims 1 to 3, wherein the tag is a peptide epitope tag.

5. The targeting module according to claim 4, wherein the peptide epitope tag is a myc-tag, a His-tag, a short linear peptide sequence from yeast transcription factor GCN4, a leucine zipper sequence or a short linear peptide sequence from a human nuclear protein, preferably from the human La protein.

6. The targeting module according to one of the claims 1 to 5 further comprising an effector cell binding domain, wherein the effector cell binding domain specifically binds an epitope on a human CD3 chain, a human T cell receptor (TCR) or human CD16.

7. The targeting module according to one of the claims 1 to 6, wherein the variable regions of the at least one PD-L1 and/or PD-L2 binding domain and/or the effector cell binding domain comprise a humanized amino acid sequence.

8. The targeting module according to one of the claims 1 to 7, wherein the length is in the range of 20 to 1600 amino acids.

9. The targeting module according to one of the claims 1 to 8 for use in a method for stimulating a chimeric antigen receptor mediated immune response in a mammal,
wherein the targeting module is administered in combination with a vector or a cell comprising a nucleotide sequence encoding a switchable chimeric antigen receptor and at least one further targeting module,
wherein the at least one further targeting module comprises at least one target cell binding domain and a tag-binding domain or a tag,
wherein the at least one target cell binding domain is an antibody, antibody fragment, a protein, a peptide or a low molecular weight organic ligand that binds to surface antigens selected from the group comprising CD2, CD3, CD4, CD8, CD10, CD19, CD20, CD22, CD23, CD25, CD30, CD33, CD38, CD44, CD44v6 CD52, CD90, CD99, CD123, CD133, CD150 CD181, CD182, CD184, CD223, CD229, CD269 (BCMA), CD273, CD274, CD276, CD279, CD319, CD366 and CD371, interleukin receptors, especially preferred IL-8Rα (CXCR1), IL-8Rβ (CXCR2), IL-11Ra, IL-11Rβ, IL-13Ra1 and 2, CXCR4, c-Met, mesothelin, members of the epidermal growth factor receptor family and mutants thereof, especially preferred ErbB1, ErbB2, ErbB3, ErbB4 or mutants thereof, members of the tumor necrosis factor receptor superfamily, ephrins, ephrin receptors, especially preferred EphA1-10, EphA5 or EphB1-6, prostate specific antigens prostate stem cell antigen (PSCA) and prostate specific membrane antigen (PSMA), embryonic antigens carcinoembryonic antigen (CEA) and fetal acethylcholine receptor, members of the vascular endothelia growth factor family, epithelia cell adhesion molecule EpCAM, alphafetoprotein AFP, members of the intercellular adhesion molecule family, members of the mucin protein family, follicle stimulating hormone receptor (FSHR), the human high molecular weight-melanoma-associated antigen (HMW-MAA), folate binding protein FBP, folate receptors, somatostatin receptors, ligands of the NKG2D receptor, cytokine receptors, members of the epithelia glycoprotein family, diasialogangliosides, glypicans, G protein-coupled receptors, members of the carbonic anhydrase family, members of the carbohydrate antigen family, Notch ligands, melanoma-associated chondroitin sulfate proteoglycan (MCSP), glycoprotein A33, guanylatecyclase 2C and tumor-specific glycans, including mutants and analogues of the named antibodies, antibody fragments, proteins, peptides or low molecular weight organic ligands,
wherein the targeting module according to one of the claims 1 to 8 and the at least one further targeting module comprise different target cell binding domains, and identical tag-binding domains or a tags.

10. A nucleic acid, a vector or a cell comprising a nucleotide sequence encoding a targeting module according to one of the claims 1 to 9 for use in a method for stimulating a chimeric antigen receptor mediated immune response in a mammal,
wherein the nucleic acid, vector or cell is administered in combination with a vector or a cell comprising a nucleotide sequence encoding a switchable chimeric antigen receptor, wherein the switchable chimeric antigen receptor comprises three domains,
wherein
- the first domain is a tag-binding domain or a tag,
- the second domain is an extracellular hinge and a transmembrane domain and
- the third domain is a signal transduction domain,
wherein the tag-binding domain of the targeting module binds to the tag of the switchable chimeric antigen receptor or the tag of the targeting module binds to the tag-binding domain of the switchable chimeric antigen receptor.

11. The nucleic acid, vector or cell according to claim 10 further comprising an inducible expression system, preferably selected from Tet-On, Tet-On Advanced, Tet-On 3G, T-REx or a promoter responsive to nuclear factor of activated T cells (NFAT).

12. The nucleic acid, vector or cell according to claim 10 or 11 further comprising a nucleotide sequence encoding a switchable chimeric antigen receptor,
wherein the switchable chimeric antigen receptor comprises three domains,
wherein
- the first domain is a tag-binding domain or tag,
- the second domain is an extracellular hinge and a transmembrane domain and
- the third domain is a signal transduction domain,
wherein the tag-binding domain of the targeting module binds to the tag of the switchable chimeric antigen receptor or the tag of the targeting module binds to the tag-binding domain of the switchable chimeric antigen receptor.

13. A nucleic acid, a vector or a cell comprising
- an inducible expression system, preferably selected from Tet-On, Tet-On Advanced, Tet-On 3G, TREx or a promoter responsive to NFAT, and
- a nucleotide sequence encoding a switchable chimeric antigen receptor,
wherein the switchable chimeric antigen receptor comprises three domains, wherein
- the first domain is a tag-binding domain or tag,
- the second domain is an extracellular hinge and a transmembrane domain and
- the third domain is a signal transduction domain, and
- a nucleotide sequence encoding a targeting module according to one of the claims 1 to 9,
wherein the tag-binding domain of the targeting module binds to the tag of the switchable chimeric antigen receptor or the tag of the targeting module binds to the tag-binding domain of the switchable chimeric antigen receptor.

14. A pharmaceutical composition comprising the targeting module according to one of the claims 1 to 9, the nucleic acid, vector or cell according to one of the claims 10 to 13 for use in a method for stimulating a chimeric antigen receptor mediated immune response in a mammal, wherein the pharmaceutical composition is administered in combination with a vector or a cell comprising a nucleotide sequence encoding a switchable chimeric antigen receptor, wherein the switchable chimeric antigen receptor comprises three domains,
wherein
- the first domain is a tag-binding domain or a tag,
- the second domain is an extracellular hinge and a transmembrane domain and
- the third domain is a signal transduction domain,
wherein the tag-binding domain of the targeting module binds to the tag of the switchable chimeric antigen receptor or the tag of the targeting module binds to the tag-binding domain of the switchable chimeric antigen receptor.

15. A kit comprising
a) a targeting module according to one of the claims 1 to 9, the nucleic acid, vector or cell according to one of the claims 10 to 13 and
b) a vector or a cell comprising a nucleotide sequence encoding a switchable chimeric antigen receptor,
wherein the switchable chimeric antigen receptor comprises three domains,
wherein
- the first domain is a tag-binding domain or tag,
- the second domain is an extracellular hinge and a transmembrane domain and
- the third domain is a signal transduction domain,
wherein the tag-binding domain of the targeting module binds to the tag of the switchable chimeric antigen receptor or the tag of the targeting module binds to the tag-binding domain of the switchable chimeric antigen receptor.

16. The kit according to claim 15, wherein the tag is a myc-tag, a His-tag, a short linear peptide sequence from yeast transcription factor GCN4, a leucine zipper sequence or a short linear peptide sequence from a human nuclear protein, preferably from the human La protein.

17. The kit according to claim 15 or 16, wherein the tag-binding domain is an antibody or an antibody fragment binding to a myc-tag, a His-tag, a short linear peptide sequence from yeast transcription factor GCN4, a leucine zipper sequence or a short linear peptide sequence from a human nuclear protein, preferably from a human La protein.

18. The kit according to one of the claims 15 to 17, wherein the extracellular hinge and transmembrane domain is selected from hinge and transmembrane domains of human CD28 molecule, CD8a chain NK cell receptors, preferably natural killer group NKG2D; or parts of the constant region of an antibody and combinations thereof.

19. The kit according to one of the claims 15 to 18, wherein the signal transduction domain is selected from cytoplasmic regions of CD28, CD137 (4-1BB), CD134 (OX40), CD278 (ICOS), DAP10 and CD27, programmed cell death-1 (PD-1), cytotoxic T-lymphocyte antigen 4 (CTLA-4), cytoplasmic regions of CD3 chains, DAP12, CD122 (interleukin-2 receptor β), CD132 (interleukin-2 receptor γ), CD127 (interleukin-7 receptor α), CD360 (interleukin-21 receptor), activating Fc receptors and mutants thereof.

20. The kit according to one of the claims 15 to 19, wherein the targeting module and/or the vector or cell comprising a nucleotide sequence encoding a switchable chimeric antigen receptor are in the form of a pharmaceutical composition.

21. The kit according to one of the claims 15 to 20 for use in a method for stimulating a chimeric antigen receptor mediated immune response in a mammal, preferably for use in the treatment of cancer, infectious disease or autoimmune disease.


SEQUENCE LISTING

<110>  GEMoaB Monoclonals GmbH

<120>  A targeting module comprising PD-L1 and/or PD-L2 for use in a
       method for stimulating a chimeric antigen receptor mediated
       immune response in a mammal

<130>  01281P0022EP

<160>  64

<170>  PatentIn version 3.5

<210>  1
<211>  272
<212>  PRT
<213>  Homo sapiens

<400>  1

Phe Thr Val Thr Val Pro Lys Asp Leu Tyr Val Val Glu Tyr Gly Ser
1               5                   10                  15

Asn Met Thr Ile Glu Cys Lys Phe Pro Val Glu Lys Gln Leu Asp Leu
            20                  25                  30

Ala Ala Leu Ile Val Tyr Trp Glu Met Glu Asp Lys Asn Ile Ile Gln
        35                  40                  45

Phe Val His Gly Glu Glu Asp Leu Lys Val Gln His Ser Ser Tyr Arg
    50                  55                  60

Gln Arg Ala Arg Leu Leu Lys Asp Gln Leu Ser Leu Gly Asn Ala Ala
65                  70                  75                  80

Leu Gln Ile Thr Asp Val Lys Leu Gln Asp Ala Gly Val Tyr Arg Cys
                85                  90                  95

Met Ile Ser Tyr Gly Gly Ala Asp Tyr Lys Arg Ile Thr Val Lys Val
            100                 105                 110

Asn Ala Pro Tyr Asn Lys Ile Asn Gln Arg Ile Leu Val Val Asp Pro
        115                 120                 125

Val Thr Ser Glu His Glu Leu Thr Cys Gln Ala Glu Gly Tyr Pro Lys
    130                 135                 140

Ala Glu Val Ile Trp Thr Ser Ser Asp His Gln Val Leu Ser Gly Lys
145                 150                 155                 160

Thr Thr Thr Thr Asn Ser Lys Arg Glu Glu Lys Leu Phe Asn Val Thr
                165                 170                 175

Ser Thr Leu Arg Ile Asn Thr Thr Thr Asn Glu Ile Phe Tyr Cys Thr
            180                 185                 190

Phe Arg Arg Leu Asp Pro Glu Glu Asn His Thr Ala Glu Leu Val Ile
        195                 200                 205

Pro Glu Leu Pro Leu Ala His Pro Pro Asn Glu Arg Thr His Leu Val
    210                 215                 220

Ile Leu Gly Ala Ile Leu Leu Cys Leu Gly Val Ala Leu Thr Phe Ile
225                 230                 235                 240

Phe Arg Leu Arg Lys Gly Arg Met Met Asp Val Lys Lys Cys Gly Ile
                245                 250                 255

Gln Asp Thr Asn Ser Lys Lys Gln Ser Asp Thr His Leu Glu Glu Thr
            260                 265                 270

<210>  2
<211>  254
<212>  PRT
<213>  Homo sapiens

<400>  2

Leu Phe Thr Val Thr Val Pro Lys Glu Leu Tyr Ile Ile Glu His Gly
1               5                   10                  15

Ser Asn Val Thr Leu Glu Cys Asn Phe Asp Thr Gly Ser His Val Asn
            20                  25                  30

Leu Gly Ala Ile Thr Ala Ser Leu Gln Lys Val Glu Asn Asp Thr Ser
        35                  40                  45

Pro His Arg Glu Arg Ala Thr Leu Leu Glu Glu Gln Leu Pro Leu Gly
    50                  55                  60

Lys Ala Ser Phe His Ile Pro Gln Val Gln Val Arg Asp Glu Gly Gln
65                  70                  75                  80

Tyr Gln Cys Ile Ile Ile Tyr Gly Val Ala Trp Asp Tyr Lys Tyr Leu
                85                  90                  95

Thr Leu Lys Val Lys Ala Ser Tyr Arg Lys Ile Asn Thr His Ile Leu
            100                 105                 110

Lys Val Pro Glu Thr Asp Glu Val Glu Leu Thr Cys Gln Ala Thr Gly
        115                 120                 125

Tyr Pro Leu Ala Glu Val Ser Trp Pro Asn Val Ser Val Pro Ala Asn
    130                 135                 140

Thr Ser His Ser Arg Thr Pro Glu Gly Leu Tyr Gln Val Thr Ser Val
145                 150                 155                 160

Leu Arg Leu Lys Pro Pro Pro Gly Arg Asn Phe Ser Cys Val Phe Trp
                165                 170                 175

Asn Thr His Val Arg Glu Leu Thr Leu Ala Ser Ile Asp Leu Gln Ser
            180                 185                 190

Gln Met Glu Pro Arg Thr His Pro Thr Trp Leu Leu His Ile Phe Ile
        195                 200                 205

Pro Phe Cys Ile Ile Ala Phe Ile Phe Ile Ala Thr Val Ile Ala Leu
    210                 215                 220

Arg Lys Gln Leu Cys Gln Lys Leu Tyr Ser Ser Lys Asp Thr Thr Lys
225                 230                 235                 240

Arg Pro Val Thr Thr Thr Lys Arg Glu Val Asn Ser Ala Ile
                245                 250


<210>  3
<211>  265
<212>  PRT
<213>  Homo sapiens

<400>  3

Phe Leu Asp Ser Pro Asp Arg Pro Trp Asn Pro Pro Thr Phe Ser Pro
1               5                   10                  15

Ala Leu Leu Val Val Thr Glu Gly Asp Asn Ala Thr Phe Thr Cys Ser
            20                  25                  30

Phe Ser Asn Thr Ser Glu Ser Phe Val Leu Asn Trp Tyr Arg Met Ser
        35                  40                  45

Pro Ser Asn Gln Thr Asp Lys Leu Ala Ala Phe Pro Glu Asp Arg Ser
    50                  55                  60

Gln Pro Gly Gln Asp Cys Arg Phe Arg Val Thr Gln Leu Pro Asn Gly
65                  70                  75                  80

Arg Asp Phe His Met Ser Val Val Arg Ala Arg Arg Asn Asp Ser Gly
                85                  90                  95

Thr Tyr Leu Cys Gly Ala Ile Ser Leu Ala Pro Lys Ala Gln Ile Lys
            100                 105                 110

Glu Ser Leu Arg Ala Glu Leu Arg Val Thr Glu Arg Arg Ala Glu Val
        115                 120                 125

Pro Thr Ala His Pro Ser Pro Ser Pro Arg Pro Ala Gly Gln Phe Gln
    130                 135                 140

Thr Leu Val Val Gly Val Val Gly Gly Leu Leu Gly Ser Leu Val Leu
145                 150                 155                 160

Leu Val Trp Val Leu Ala Val Ile Cys Ser Arg Ala Ala Arg Gly Thr
                165                 170                 175

Ile Gly Ala Arg Arg Thr Gly Gln Pro Leu Lys Glu Asp Pro Ser Ala
            180                 185                 190

Val Pro Val Phe Ser Val Asp Tyr Gly Glu Leu Asp Phe Gln Trp Arg
        195                 200                 205

Glu Lys Thr Pro Glu Pro Pro Val Pro Cys Val Pro Glu Gln Thr Glu
    210                 215                 220

Tyr Ala Thr Ile Val Phe Pro Ser Gly Met Gly Thr Ser Ser Pro Ala
225                 230                 235                 240

Arg Arg Gly Ser Ala Asp Gly Pro Arg Ser Ala Gln Pro Leu Arg Pro
                245                 250                 255

Glu Asp Gly His Cys Ser Trp Pro Leu
            260                 265

<210>  4
<211>  123
<212>  PRT
<213>  Artificial Sequence

<220>
<223>  Humanized VH chain

<400>  4

Gln Val Gln Leu Val Gln Ser Gly Ala Glu Val Lys Lys Pro Gly Ser
1               5                   10                  15

Ser Val Lys Val Ser Cys Lys Thr Ser Gly Asp Thr Phe Ser Thr Tyr
            20                  25                  30

Ala Ile Ser Trp Val Arg Gln Ala Pro Gly Gln Gly Leu Glu Trp Met
        35                  40                  45

Gly Gly Ile Ile Pro Ile Phe Gly Lys Ala His Tyr Ala Gln Lys Phe
    50                  55                  60

Gln Gly Arg Val Thr Ile Thr Ala Asp Glu Ser Thr Ser Thr Ala Tyr
65                  70                  75                  80

Met Glu Leu Ser Ser Leu Arg Ser Glu Asp Thr Ala Val Tyr Phe Cys
                85                  90                  95

Ala Arg Lys Phe His Phe Val Ser Gly Ser Pro Phe Gly Met Asp Val
            100                 105                 110

Trp Gly Gln Gly Thr Thr Val Thr Val Ser Ser
        115                 120

<210>  5
<211>  106
<212>  PRT
<213>  Artificial Sequence

<220>
<223>  Humanized VL chain

<400>  5

Glu Ile Val Leu Thr Gln Ser Pro Ala Thr Leu Ser Leu Ser Pro Gly
1               5                   10                  15

Glu Arg Ala Thr Leu Ser Cys Arg Ala Ser Gln Ser Val Ser Ser Tyr
            20                  25                  30

Leu Ala Trp Tyr Gln Gln Lys Pro Gly Gln Ala Pro Arg Leu Leu Ile
        35                  40                  45

Tyr Asp Ala Ser Asn Arg Ala Thr Gly Ile Pro Ala Arg Phe Ser Gly
    50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Gln Arg Ser Asn Trp Pro Thr
                85                  90                  95

Phe Gly Gln Gly Thr Lys Val Glu Ile Lys
            100                 105

<210>  6
<211>  118
<212>  PRT
<213>  Artificial Sequence

<220>
<223>  Humanized VH chain

<400>  6


<210>  7
<211>  107
<212>  PRT
<213>  Artificial Sequence

<220>
<223>  Humanized VL chain

<400>  7

<210>  8
<211>  121
<212>  PRT
<213>  Artificial Sequence

<220>
<223>  Humanized VH chain

<400>  8

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Arg Tyr
            20                  25                  30

Trp Met Ser Trp Val Arg Gln Ala Pro Gly Lys Gly Leu Glu Trp Val
        35                  40                  45

Ala Asn Ile Lys Gln Asp Gly Ser Glu Lys Tyr Tyr Val Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Arg Glu Gly Gly Trp Phe Gly Glu Leu Ala Phe Asp Tyr Trp Gly
            100                 105                 110

Gln Gly Thr Leu Val Thr Val Ser Ser
        115                 120

<210>  9
<211>  108
<212>  PRT
<213>  Artificial Sequence

<220>
<223>  Humanized VL chain

<400>  9

Glu Ile Val Leu Thr Gln Ser Pro Gly Thr Leu Ser Leu Ser Pro Gly
1               5                   10                  15

Glu Arg Ala Thr Leu Ser Cys Arg Ala Ser Gln Arg Val Ser Ser Ser
            20                  25                  30

Tyr Leu Ala Trp Tyr Gln Gln Lys Pro Gly Gln Ala Pro Arg Leu Leu
        35                  40                  45

Ile Tyr Asp Ala Ser Ser Arg Ala Thr Gly Ile Pro Asp Arg Phe Ser
    50                  55                  60

Gly Ser Gly Ser Gly Thr Asp Phe Thr Leu Thr Ile Ser Arg Leu Glu
65                  70                  75                  80

Pro Glu Asp Phe Ala Val Tyr Tyr Cys Gln Gln Tyr Gly Ser Leu Pro
                85                  90                  95

Trp Thr Phe Gly Gln Gly Thr Lys Val Glu Ile Lys
            100                 105

<210>  10
<211>  33
<212>  PRT
<213>  Saccharomyces cerevisiae

<400>  10

Arg Met Lys Gln Leu Glu Pro Lys Val Glu Glu Leu Leu Pro Lys Asn
1               5                   10                  15

Tyr His Leu Glu Asn Glu Val Ala Arg Leu Lys Lys Leu Val Gly Glu
            20                  25                  30

Arg

<210>  11
<211>  14
<212>  PRT
<213>  Saccharomyces cerevisiae

<400>  11

Asn Tyr His Leu Glu Asn Glu Val Ala Arg Leu Lys Lys Leu
1               5                   10

<210>  12
<211>  51
<212>  PRT
<213>  Homo sapiens

<400>  12

Met Asp Pro Asp Leu Glu Ile Arg Ala Ala Phe Leu Arg Gln Arg Asn
1               5                   10                  15

Thr Ala Leu Arg Thr Glu Val Ala Glu Leu Glu Gln Glu Val Gln Arg
            20                  25                  30

Leu Glu Asn Glu Val Ser Gln Tyr Glu Thr Arg Tyr Gly Pro Leu Gly
        35                  40                  45

Gly Gly Lys
    50

<210>  13
<211>  51
<212>  PRT
<213>  Homo sapiens

<400>  13

Met Asp Pro Asp Leu Glu Ile Glu Ala Ala Phe Leu Glu Arg Glu Asn
1               5                   10                  15

Thr Ala Leu Glu Thr Arg Val Ala Glu Leu Arg Gln Arg Val Gln Arg
            20                  25                  30

Leu Arg Asn Arg Val Ser Gln Tyr Arg Thr Arg Tyr Gly Pro Leu Gly
        35                  40                  45

Gly Gly Lys
    50

<210>  14
<211>  10
<212>  PRT
<213>  Homo sapiens

<400>  14

Lys Pro Leu Pro Glu Val Thr Asp Glu Tyr
1               5                   10

<210>  15
<211>  20
<212>  PRT
<213>  Homo sapiens

<220>
<221>  MISC_FEATURE
<222>  (16)..(16)
<223>  Xaa can be alanine or serine

<400>  15

Leu Glu Lys Glu Ala Leu Lys Lys Ile Ile Glu Asp Gln Gln Glu Xaa
1               5                   10                  15

Leu Asn Lys Trp
            20

<210>  16
<211>  20
<212>  PRT
<213>  Homo sapiens

<400>  16

Leu Glu Lys Glu Ala Leu Lys Lys Ile Ile Glu Asp Gln Gln Glu Ala
1               5                   10                  15

Leu Asn Lys Trp
            20

<210>  17
<211>  113
<212>  PRT
<213>  Artificial Sequence

<220>
<223>  Humanized scFv VL chain

<220>
<221>  MISC_FEATURE
<222>  (24)..(24)
<223>  Xaa can be any naturally occurring amino acid

<220>
<221>  MISC_FEATURE
<222>  (35)..(35)
<223>  Xaa can be any naturally occurring amino acid

<220>
<221>  MISC_FEATURE
<222>  (61)..(61)
<223>  Xaa can be any naturally occurring amino acid

<220>
<221>  MISC_FEATURE
<222>  (101)..(101)
<223>  Xaa can be any naturally occurring amino acid

<400>  17

Asp Ile Val Met Thr Gln Ser Pro Asp Ser Leu Ala Val Ser Leu Gly

1                   5                   10                  15

Glu Arg Ala Thr Ile Asn Cys Xaa Ser Ser Gln Ser Leu Leu Asn Ser
            20                  25                  30

Arg Thr Xaa Lys Asn Tyr Leu Ala Trp Tyr Gln Gln Lys Pro Gly Gln
        35                  40                  45

Pro Pro Lys Leu Leu Ile Tyr Trp Ala Ser Thr Arg Xaa Ser Gly Val
    50                  55                  60

Pro Asp Arg Phe Ser Gly Ser Gly Ser Gly Thr Asp Phe Thr Leu Thr
65                  70                  75                  80

Ile Ser Ser Leu Gln Ala Glu Asp Val Ala Val Tyr Tyr Cys Lys Gln
                85                  90                  95

Ser Tyr Asn Leu Xaa Thr Phe Gly Gly Gly Thr Lys Val Glu Ile Leu
            100                 105                 110

Lys

<210>  18
<211>  120
<212>  PRT
<213>  Artificial Sequence

<220>
<223>  Humanized scFv VH chain

<400>  18

Gln Val Gln Leu Val Gln Ser Gly Ala Glu Val Lys Lys Pro Gly Ala
1                   5                   10                  15

Ser Val Lys Val Ser Cys Lys Ala Ser Gly Tyr Thr Phe Thr His Tyr
            20                  25                  30

Tyr Ile Tyr Trp Val Arg Gln Ala Pro Gly Gln Gly Leu Glu Trp Met
        35                  40                  45

Gly Gly Val Asn Pro Ser Asn Gly Gly Thr His Phe Asn Glu Lys Phe
    50                  55                  60

Lys Ser Arg Val Thr Met Thr Arg Asp Thr Ser Ile Ser Thr Ala Tyr
65                  70                  75                  80

Met Glu Leu Ser Arg Leu Arg Ser Asp Asp Thr Ala Val Tyr Tyr Cys
                85                  90                  95

Ala Arg Ser Glu Tyr Asp Tyr Gly Leu Gly Phe Ala Tyr Trp Gly Gln
            100                 105                 110

Gly Thr Leu Val Thr Val Ser Ser
        115                 120

<210>  19
<211>  112
<212>  PRT
<213>  Artificial Sequence

<220>
<223>  Humanized scFv VL chain

<400>  19

Asp Ile Val Met Thr Gln Ser Pro Asp Ser Leu Ala Val Ser Leu Gly
1               5                   10                  15

Glu Arg Ala Thr Ile Asn Cys Lys Ser Ser Gln Ser Leu Leu Asn Ser
            20                  25                  30

Arg Thr Pro Lys Asn Tyr Leu Ala Trp Tyr Gln Gln Lys Pro Gly Gln
        35                  40                  45

Pro Pro Lys Leu Leu Ile Tyr Trp Ala Ser Thr Arg Lys Ser Gly Val
    50                  55                  60

Pro Asp Arg Phe Ser Gly Ser Gly Ser Gly Thr Asp Phe Thr Leu Thr
65                  70                  75                  80

Ile Ser Ser Leu Gln Ala Glu Asp Val Ala Val Tyr Tyr Cys Lys Gln
                85                  90                  95

Ser Tyr Asn Leu Leu Thr Phe Gly Gly Gly Thr Lys Val Glu Ile Lys
            100                 105                 110

<210>  20
<211>  118
<212>  PRT
<213>  Artificial Sequence

<220>
<223>  Humanized scFv VH chain

<400>  20

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Asp Phe

20                  25                  30

Trp Met Asn Trp Val Arg Gln Ala Pro Gly Lys Gly Leu Glu Trp Val
        35                  40                  45

Gly Gln Ile Arg Asn Lys Pro Asn Asn Tyr Glu Thr Tyr Tyr Ser Asp
    50                  55                  60

Ser Leu Lys Gly Arg Phe Thr Ile Ser Arg Asp Asp Ser Lys Ser Ile
65                  70                  75                  80

Thr Tyr Leu Gln Met Asn Ser Leu Arg Ala Glu Asp Thr Ala Val Tyr
                85                  90                  95

Tyr Cys Thr Leu Gly Asn Ser Trp Phe Ala Tyr Trp Gly Gln Gly Thr
            100                 105                 110

Leu Val Thr Val Ser Ser
        115

<210>  21
<211>  112
<212>  PRT
<213>  Artificial Sequence

<220>
<223>  Humanized scFv VL chain

<400>  21

Asp Ile Val Met Thr Gln Ser Pro Asp Ser Leu Ala Val Ser Leu Gly
1               5                   10                  15

Glu Arg Ala Thr Ile Asn Cys Arg Ser Ser Gln Ser Leu Leu Asp Ser
            20                  25                  30

Arg Thr Lys Lys Asn Tyr Leu Ala Trp Tyr Gln Gln Lys Pro Gly Gln
        35                  40                  45

Pro Pro Lys Leu Leu Ile Tyr Trp Ala Ser Thr Arg Glu Ser Gly Val
    50                  55                  60

Pro Asp Arg Phe Ser Gly Ser Gly Ser Gly Thr Asp Phe Thr Leu Thr
65                  70                  75                  80

Ile Ser Ser Leu Gln Ala Glu Asp Val Ala Val Tyr Tyr Cys Lys Gln
                85                  90                  95

Ser Tyr Asn Leu Pro Thr Phe Gly Gly Gly Thr Lys Val Glu Ile Lys
            100                 105                 110

<210>  22
<211>  115
<212>  PRT
<213>  Mus musculus

<400>  22

Asp Ile Val Leu Thr Gln Thr Pro Ala Ile Met Ser Ala Ser Pro Gly
1               5                   10                  15

Glu Lys Val Thr Met Thr Cys Ser Ala Ser Ser Ser Val Ser Tyr Met
            20                  25                  30

His Trp Tyr Gln Gln Lys Ser Gly Thr Ser Pro Lys Arg Trp Ile Tyr
        35                  40                  45

Asp Thr Ser Lys Leu Ala Ser Gly Val Pro Thr Arg Phe Ser Gly Ser
    50                  55                  60

Gly Ser Gly Thr Ser Tyr Ser Leu Thr Ile Ser Ser Met Glu Ala Glu
65                  70                  75                  80

Asp Ala Ala Thr Tyr Tyr Cys Gln Gln Trp Ser Ser Asn Pro Leu Thr
                85                  90                  95

Phe Gly Ala Gly Thr Lys Leu Glu Leu Lys Arg Ala Asp Ala Ala Pro
            100                 105                 110

Thr Val Ser
        115


<210>  23
<211>  117
<212>  PRT
<213>  Mus musculus

<400>  23

Glu Val Lys Leu Gln Glu Ser Gly Pro Glu Leu Val Lys Ser Gly Ala
1               5                   10                  15

Ser Val Lys Met Ser Cys Lys Ala Ser Gly Tyr Thr Phe Thr Lys Tyr
            20                  25                  30

Val Ile His Trp Val Lys Gln Lys Pro Gly Gln Gly Leu Glu Trp Ile
        35                  40                  45

Gly Tyr Ile Asn Pro Tyr Asn Asp Val Ser Lys Tyr Asn Glu Lys Phe
    50                  55                  60

Arg Gly Lys Ala Thr Leu Thr Ser Asp Lys Ser Ser Ser Thr Ala Tyr
65                  70                  75                  80

Met Glu Leu Ser Ser Leu Thr Ser Glu Asp Ser Ala Val Tyr Phe Cys
                85                  90                  95

Ser Arg Asn Lys Asp Tyr Tyr Pro Met Asp Tyr Trp Gly Gln Gly Thr
            100                 105                 110

Ser Val Thr Val Ser
        115

<210>  24
<211>  105
<212>  PRT
<213>  Artificial Sequence

<220>
<223>  Humanized scFv VL chain

<400>  24

Glu Ile Val Leu Thr Gln Ser Pro Ala Thr Leu Ser Leu Ser Pro Gly
1               5                   10                  15

Glu Arg Ala Thr Leu Ser Cys Ser Ala Ser Ser Ser Val Ser Tyr Met
            20                  25                  30

His Trp Tyr Gln Gln Lys Pro Gly Gln Ala Pro Arg Leu Leu Ile Tyr
        35                  40                  45

Asp Thr Ser Lys Leu Ala Ser Gly Ile Pro Ala Arg Phe Ser Gly Ser
    50                  55                  60

Gly Ser Gly Thr Asp Phe Thr Leu Thr Ile Ser Ser Leu Glu Pro Glu
65                  70                  75                  80

Asp Phe Ala Val Tyr Tyr Cys Gln Gln Trp Ser Ser Asn Pro Leu Thr
                85                  90                  95

Phe Gly Gly Gly Thr Lys Val Glu Ile
            100                 105

<210>  25
<211>  117
<212>  PRT
<213>  Artificial Sequence

<220>
<223>  Humanized scFv VH chain

<400>  25

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Lys Tyr
            20                  25                  30

Val Ile His Trp Val Arg Gln Ala Pro Gly Gln Gly Leu Glu Trp Met
        35                  40                  45

Gly Tyr Ile Asn Pro Tyr Asn Asp Val Ser Lys Tyr Asn Glu Lys Phe
    50                  55                  60

Arg Gly Arg Val Thr Met Thr Thr Asp Thr Ser Thr Ser Thr Ala Tyr
65                  70                  75                  80

Met Glu Leu Arg Ser Leu Arg Ser Asp Asp Thr Ala Val Tyr Tyr Cys
                85                  90                  95

Ala Arg Asn Lys Asp Tyr Tyr Pro Met Asp Tyr Trp Gly Gln Gly Thr
            100                 105                 110

Thr Val Thr Val Ser
        115


<210>  26
<211>  108
<212>  PRT
<213>  Artificial Sequence

<220>
<223>  Humanized antibody VL chain

<400>  26

Tyr Glu Leu Ile Gln Pro Ser Ser Ala Ser Val Thr Val Gly Glu Thr
1               5                   10                  15

Val Lys Ile Thr Cys Ser Gly Asp Gln Leu Pro Lys Asn Phe Ala Tyr
            20                  25                  30

Trp Phe Gln Gln Lys Ser Asp Lys Asn Ile Leu Leu Leu Ile Tyr Met
        35                  40                  45

Asp Asn Lys Arg Pro Ser Gly Ile Pro Glu Arg Phe Ser Gly Ser Thr
    50                  55                  60

Ser Gly Thr Thr Ala Thr Leu Thr Ile Ser Gly Ala Gln Pro Glu Asp
65                  70                  75                  80

Glu Ala Ala Tyr Tyr Cys Leu Ser Ser Tyr Gly Asp Asn Asn Asp Leu
                85                  90                  95

Val Phe Gly Ser Gly Thr Gln Leu Thr Val Leu Arg
            100                 105

<210>  27
<211>  121
<212>  PRT
<213>  Artificial Sequence

<220>
<223>  Humanized antibody VH chain

<400>  27

Glu Val Tyr Leu Val Glu Ser Gly Gly Asp Leu Val Gln Pro Gly Ser
1               5                   10                  15

Ser Leu Lys Val Ser Cys Ala Ala Ser Gly Phe Thr Phe Ser Asp Phe
            20                  25                  30

Trp Met Tyr Trp Val Arg Gln Ala Pro Gly Lys Gly Leu Glu Trp Val
        35                  40                  45

Gly Arg Ile Lys Asn Ile Pro Asn Asn Tyr Ala Thr Glu Tyr Ala Asp
    50                  55                  60

Ser Val Arg Gly Arg Phe Thr Ile Ser Arg Asp Asp Ser Arg Asn Ser
65                  70                  75                  80

Ile Tyr Leu Gln Met Asn Arg Leu Arg Val Asp Asp Thr Ala Ile Tyr
                85                  90                  95

Tyr Cys Thr Arg Ala Gly Arg Phe Asp His Phe Asp Tyr Trp Gly Gln
            100                 105                 110

Gly Thr Met Val Thr Val Ser Ser Ala
        115                 120

<210>  28
<211>  106
<212>  PRT
<213>  Artificial Sequence

<220>
<223>  Humanized antibody VL chain

<400>  28

Ser Tyr Val Leu Thr Gln Pro Ser Ser Val Ser Val Ala Pro Gly Gln
1               5                   10                  15

Thr Ala Thr Ile Ser Cys Gly Gly His Asn Ile Gly Ser Lys Asn Val
            20                  25                  30

His Trp Tyr Gln Gln Arg Pro Gly Gln Ser Pro Val Leu Val Ile Tyr
        35                  40                  45

Gln Asp Asn Lys Arg Pro Ser Gly Ile Pro Glu Arg Phe Ser Gly Ser
    50                  55                  60

Asn Ser Gly Asn Thr Ala Thr Leu Thr Ile Ser Gly Thr Gln Ala Met
65                  70                  75                  80

Asp Glu Ala Asp Tyr Tyr Cys Gln Val Trp Asp Asn Tyr Ser Val Leu
                85                  90                  95

Phe Gly Gly Gly Thr Lys Leu Thr Val Leu
            100                 105

<210>  29
<211>  120
<212>  PRT
<213>  Artificial Sequence

<220>
<223>  Humanized antibody VH chain

<400>  29

Gln Val Gln Leu Val Gln Ser Gly Ala Glu Val Lys Lys Pro Gly Glu
1               5                   10                  15

Ser Leu Lys Val Ser Cys Lys Ala Ser Gly Tyr Thr Phe Thr Ser Tyr
            20                  25                  30

Tyr Met His Trp Val Arg Gln Ala Pro Gly Gln Gly Leu Glu Trp Met
        35                  40                  45

Gly Ile Ile Asn Pro Ser Gly Gly Ser Thr Ser Tyr Ala Gln Lys Phe
    50                  55                  60

Gln Gly Arg Val Thr Met Thr Arg Asp Thr Ser Thr Ser Thr Val Tyr
65                  70                  75                  80

Met Glu Leu Ser Ser Leu Arg Ser Glu Asp Thr Ala Val Tyr Tyr Cys
                85                  90                  95

Ala Arg Gly Ser Ala Tyr Tyr Tyr Asp Phe Ala Asp Tyr Trp Gly Gln
            100                 105                 110

Gly Thr Leu Val Thr Val Ser Ser
        115                 120

<210>  30
<211>  335
<212>  PRT
<213>  Artificial Sequence

<220>
<223>  Fusion protein Escherichia coli and herpes simplex virus

<400>  30

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Ile Glu Met Leu Asp Arg His His
    50                  55                  60

Thr His Phe Cys Pro Leu Lys Gly Glu Ser Trp Gln Asp Phe Leu Arg
65                  70                  75                  80

Asn Asn Ala Lys Ser Phe Arg Cys Ala Leu Leu Ser His Arg Asn Gly
                85                  90                  95

Ala Lys Val His Ser Asp Thr Arg Pro Thr Glu Lys Gln Tyr Glu Thr
            100                 105                 110

Leu Glu Asn Gln Leu Ala Phe Leu Cys Gln Gln Gly Phe Ser Leu Glu
        115                 120                 125

Asn Ala Leu Tyr Ala Leu Ser Ala Val Gly His Phe Thr Leu Gly Cys
    130                 135                 140

Val Leu Glu Asp Gln Glu His Gln Val Ala Lys Glu Glu Arg Glu Thr
145                 150                 155                 160

Pro Thr Thr Asp Ser Met Pro Pro Leu Leu Arg Gln Ala Ile Glu Leu
                165                 170                 175

Phe Asp His Gln Gly Ala Glu Pro Ala Phe Leu Phe Gly Leu Glu Leu
            180                 185                 190

Ile Ile Cys Gly Leu Glu Lys Gln Leu Lys Cys Glu Ser Gly Ser Ala
        195                 200                 205

Tyr Ser Arg Ala Arg Thr Lys Asn Asn Tyr Gly Ser Thr Ile Glu Gly
    210                 215                 220

Leu Leu Asp Leu Pro Asp Asp Asp Ala Pro Glu Glu Ala Gly Leu Ala
225                 230                 235                 240

Ala Pro Arg Leu Ser Phe Leu Pro Ala Gly His Thr Arg Arg Leu Ser
                245                 250                 255

Thr Ala Pro Pro Thr Asp Val Ser Leu Gly Asp Glu Leu His Leu Asp
            260                 265                 270

Gly Glu Asp Val Ala Met Ala His Ala Asp Ala Leu Asp Asp Phe Asp
        275                 280                 285

Leu Asp Met Leu Gly Asp Gly Asp Ser Pro Gly Pro Gly Phe Thr Pro
    290                 295                 300

His Asp Ser Ala Pro Tyr Gly Ala Leu Asp Met Ala Asp Phe Glu Phe
305                 310                 315                 320

Glu Gln Met Phe Thr Asp Ala Leu Gly Ile Asp Glu Tyr Gly Gly
                325                 330                 335

<210>  31
<211>  248
<212>  PRT
<213>  Artificial Sequence

<220>
<223>  Fusion protein Escherichia coli and herpes simplex virus

<400>  31

Met Ser Arg Leu Asp Lys Ser Lys Val Ile Asn Gly Ala Leu Glu Leu
1               5                   10                  15

Leu Asn Gly Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Pro Ile Glu Met Leu Asp Arg His His
    50                  55                  60

Thr His Phe Cys Pro Leu Glu Gly Glu Ser Trp Gln Asp Phe Leu Arg

65                  70                  75                  80

Asn Asn Ala Lys Ser Phe Arg Cys Ala Leu Leu Ser His Arg Asp Gly
                85                  90                  95

Ala Lys Val His Leu Gly Thr Arg Pro Thr Glu Lys Gln Tyr Glu Thr
            100                 105                 110

Leu Glu Asn Gln Leu Ala Phe Leu Cys Gln Gln Gly Phe Ser Leu Glu
        115                 120                 125

Asn Ala Leu Tyr Ala Leu Ser Ala Val Gly His Phe Thr Leu Gly Cys
    130                 135                 140

Val Leu Glu Glu Gln Glu His Gln Val Ala Lys Glu Glu Arg Glu Thr
145                 150                 155                 160

Pro Thr Thr Asp Ser Met Pro Pro Leu Leu Arg Gln Ala Ile Glu Leu
                165                 170                 175

Phe Asp Arg Gln Gly Ala Glu Pro Ala Phe Leu Phe Gly Leu Glu Leu
            180                 185                 190

Ile Ile Cys Gly Leu Glu Lys Gln Leu Lys Cys Glu Ser Gly Gly Pro
        195                 200                 205

Ala Asp Ala Leu Asp Asp Phe Asp Leu Asp Met Leu Pro Ala Asp Ala
    210                 215                 220

Leu Asp Asp Phe Asp Leu Asp Met Leu Pro Ala Asp Ala Leu Asp Asp
225                 230                 235                 240

Phe Asp Leu Asp Met Leu Pro Gly
                245

<210>  32
<211>  248
<212>  PRT
<213>  Artificial Sequence

<220>
<223>  Fusion protein Escherichia coli and herpes simplex virus

<400>  32

Met Ser Arg Leu Asp Lys Ser Lys Val Ile Asn Ser Ala Leu Glu Leu
1               5                   10                  15

Leu Asn Gly Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Pro Ile Glu Met Leu Asp Arg His His
    50                  55                  60

Thr His Ser Cys Pro Leu Glu Gly Glu Ser Trp Gln Asp Phe Leu Arg
65                  70                  75                  80

Asn Asn Ala Lys Ser Tyr Arg Cys Ala Leu Leu Ser His Arg Asp Gly
                85                  90                  95

Ala Lys Val His Leu Gly Thr Arg Pro Thr Glu Lys Gln Tyr Glu Thr
            100                 105                 110

Leu Glu Asn Gln Leu Ala Phe Leu Cys Gln Gln Gly Phe Ser Leu Glu
        115                 120                 125

Asn Ala Leu Tyr Ala Leu Ser Ala Val Gly His Phe Thr Leu Gly Cys
    130                 135                 140

Val Leu Glu Glu Gln Glu His Gln Val Ala Lys Glu Glu Arg Glu Thr
145                 150                 155                 160

Pro Thr Thr Asp Ser Met Pro Pro Leu Leu Lys Gln Ala Ile Glu Leu
                165                 170                 175

Phe Asp Arg Gln Gly Ala Glu Pro Ala Phe Leu Phe Gly Leu Glu Leu
            180                 185                 190

Ile Ile Cys Gly Leu Glu Lys Gln Leu Lys Cys Glu Ser Gly Gly Pro
        195                 200                 205

Thr Asp Ala Leu Asp Asp Phe Asp Leu Asp Met Leu Pro Ala Asp Ala
    210                 215                 220

Leu Asp Asp Phe Asp Leu Asp Met Leu Pro Ala Asp Ala Leu Asp Asp
225                 230                 235                 240

Phe Asp Leu Asp Met Leu Pro Gly
                245

<210>  33
<211>  19
<212>  DNA
<213>  Escherichia coli

<400>  33
tccctatcag tgatagaga                                                    19


<210>  34
<211>  16
<212>  DNA
<213>  Artificial Sequence

<220>
<223>  Spacer sequence

<400>  34
acgatgtcga gtttac                                                       16


<210>  35
<211>  379
<212>  DNA
<213>  Artificial Sequence

<220>
<223>  TRE sequence

<400>  35
gagtttactc cctatcagtg atagagaacg tatgaagagt ttactcccta tcagtgatag        60

agaacgtatg cagactttac tccctatcag tgatagagaa cgtataagga gtttactccc       120

tatcagtgat agagaacgta tgaccagttt actccctatc agtgatagag aacgtatcta       180

cagtttactc cctatcagtg atagagaacg tatatccagt ttactcccta tcagtgatag       240

agaacgtata agctttaggc gtgtacggtg ggcgcctata aaagcagagc tcgtttagtg       300

aaccgtcaga tcgcctggag caattccaca acacttttgt cttataccaa ctttccgtac       360

cacttcctac cctcgtaaa                                                    379


<210>  36
<211>  30
<212>  DNA
<213>  Homo sapiens

<400>  36
ggaggaaaaa ctgtttcata cagaaggcgt                                        30


<210>  37
<211>  52
<212>  DNA
<213>  Homo sapiens

<400>  37
cagaattaac agtataaatt gcatctcttg ttcaagagtt ccctatcact ct               52


<210>  38
<211>  110
<212>  DNA
<213>  Homo sapiens

<400> 38
acattttgac acccccataa tatttttcca gaattaacag tataaattgc atctcttgtt 60

caagagttcc ctatcactct ctttaatcac tactcacagt aacctcaact 110


<210> 39
<211> 232
<212> DNA
<213> Artificial Sequence

<220>
<223> Fusion of human DNA sequences

<400> 39
ggaggaaaaa ctgtttcata cagaaggcgt ggaggaaaaa ctgtttcata cagaaggcgt 60

ggaggaaaaa ctgtttcata cagaaggcgt ggaggaaaaa ctgtttcata cagaaggcgt 120

ggaggaaaaa ctgtttcata cagaaggcgt ggaggaaaaa ctgtttcata cagaaggcgt 180

cagaattaac agtataaatt gcatctcttg ttcaagagtt ccctatcact ct 232


<210> 40
<211> 296
<212> DNA
<213> Artificial Sequence

<220>
<223> Fusion of human DNA sequences

<400> 40
aggaggaaaa actgtttcat acagaaggcg taggaggaaa aactgtttca tacagaaggc 60

gtaggaggaa aaactgtttc atacagaagg cgtaggagga aaaactgttt catacagaag 120

gcgtaggagg aaaaactgtt tcatacagaa ggcgtaggag gaaaaactgt ttcatacaga 180

aggcgtacat tttgacaccc ccataatatt tttccagaat taacagtata aattgcatct 240

cttgttcaag agttccctat cactctcttt aatcactact cacagtaacc tcaact 296


<210> 41
<211> 627
<212> PRT
<213> Artificial Sequence

<220>
<223> humanized homo sapiens/mus musculus chimera

<400> 41
   Met Arg Arg Met Gln Leu Leu Leu Leu Ile Ala Leu Ser Leu Ala Leu
   1               5                   10                  15
   Val Thr Asn Ser Glu Phe Gln Val Gln Leu Val Gln Ser Gly Ala Glu
               20                  25                  30
   Val Lys Lys Pro Gly Ala Ser Val Lys Val Ser Cys Lys Ala Ser Gly
   35                  40                  45
   Tyr Thr Phe Thr His Tyr Tyr Ile Tyr Trp Val Arg Gln Ala Pro Gly
       50                  55                  60
   Gln Gly Leu Glu Trp Met Gly Gly Val Asn Pro Ser Asn Gly Gly Thr
   65                  70                  75                  80
   His Phe Asn Glu Lys Phe Lys Ser Arg Val Thr Met Thr Arg Asp Thr
                   85                  90                  95
   Ser Ile Ser Thr Ala Tyr Met Glu Leu Ser Arg Leu Arg Ser Asp Asp
               100                 105                 110
   Thr Ala Val Tyr Tyr Cys Ala Arg Ser Glu Tyr Asp Tyr Gly Leu Gly
           115                 120                 125
   Phe Ala Tyr Trp Gly Gln Gly Thr Leu Val Thr Val Ser Ser Gly Gly
       130                 135                 140
   Gly Gly Ser Gly Gly Gly Gly Ser Gly Gly Gly Gly Ser Asp Ile Val
   145                 150                 155                 160
   Met Thr Gln Ser Pro Asp Ser Leu Ala Val Ser Leu Gly Glu Arg Ala
                   165                 170                 175
   Thr Ile Asn Cys Lys Ser Ser Gln Ser Leu Leu Asn Ser Arg Thr Pro
               180                 185                 190
   Lys Asn Tyr Leu Ala Trp Tyr Gln Gln Lys Pro Gly Gln Pro Pro Lys
           195                 200                 205
   Leu Leu Ile Tyr Trp Ala Ser Thr Arg Lys Ser Gly Val Pro Asp Arg
       210                 215                 220
   Phe Ser Gly Ser Gly Ser Gly Thr Asp Phe Thr Leu Thr Ile Ser Ser
   225                 230                 235                 240
   Leu Gln Ala Glu Asp Val Ala Val Tyr Tyr Cys Lys Gln Ser Tyr Asn
                   245                 250                 255
   Leu Leu Thr Phe Gly Gly Gly Thr Lys Val Glu Ile Lys Ala Ala Ala
               260                 265                 270
   Leu Glu Lys Glu Ala Leu Lys Lys Ile Ile Glu Asp Gln Gln Glu Ala
           275                 280                 285
   Leu Asn Lys Trp Ala Ala Ala Gly Pro Gly Gly Gly Gly Ser Lys Ile
       290                 295                 300
   Leu Val Lys Gln Ser Pro Met Leu Val Ala Tyr Asp Asn Ala Val Asn
   305                 310                 315                 320
   Leu Ser Cys Lys Tyr Ser Tyr Asn Leu Phe Ser Arg Glu Phe Arg Ala
                   325                 330                 335
   Ser Leu His Lys Gly Leu Asp Ser Ala Val Glu Val Cys Val Val Tyr
               340                 345                 350
   Gly Asn Tyr Ser Gln Gln Leu Gln Val Tyr Ser Lys Thr Gly Phe Asn
           355                 360                 365
   Cys Asp Gly Lys Leu Gly Asn Glu Ser Val Thr Phe Tyr Leu Gln Asn
       370                 375                 380
   Leu Tyr Val Asn Gln Thr Asp Ile Tyr Phe Cys Lys Ile Glu Val Met
   385                 390                 395                 400
   Ala Pro Pro Pro Ala Leu Asp Asn Glu Lys Ser Asn Gly Thr Ile Ile
                   405                 410                 415
   His Val Lys Gly Lys His Leu Cys Pro Ser Pro Leu Phe Pro Gly Pro
               420                 425                 430
   Ser Lys Pro Phe Trp Val Leu Val Val Val Gly Gly Val Leu Ala Cys
           435                 440                 445
   Tyr Ser Leu Leu Val Thr Val Ala Phe Ile Ile Phe Trp Val Arg Ser
       450                 455                 460
   Lys Arg Ser Arg Gly Gly His Ser Asp Tyr Met Asn Met Thr Pro Arg
   465                 470                 475                 480
   Arg Pro Gly Pro Thr Arg Lys His Tyr Gln Pro Tyr Ala Pro Pro Arg
                   485                 490                 495
   Asp Phe Ala Ala Tyr Arg Ser Gly Gly Gly Gly Ser Gly Gly Gly Ala
               500                 505                 510
   Ser Gly Gly Gly Gly Ser Phe Ser Arg Ser Ala Asp Ala Pro Ala Tyr
           515                 520                 525
   Gln Gln Gly Gln Asn Gln Leu Tyr Asn Glu Leu Asn Leu Gly Arg Arg
       530                 535                 540
   Glu Glu Tyr Asp Val Leu Asp Lys Arg Arg Gly Arg Asp Pro Glu Met
   545                 550                 555                 560
   Gly Gly Lys Pro Arg Arg Lys Asn Pro Gln Glu Gly Leu Tyr Asn Glu
                   565                 570                 575
   Leu Gln Lys Asp Lys Met Ala Glu Ala Tyr Ser Glu Ile Gly Met Lys
               580                 585                 590
   Gly Glu Arg Arg Arg Gly Lys Gly His Asp Gly Leu Tyr Gln Gly Leu
           595                 600                 605
   Ser Thr Ala Thr Lys Asp Thr Tyr Gly Ala Leu His Met Gln Ala Leu
       610                 615                 620
   Pro Pro Arg
   625

<210>  42
<211>  532
<212>  PRT
<213>  Artificial Sequence

<220>
<223>  humanized homo sapiens/mus musculus chimera

<400>  42

Met Arg Arg Met Gln Leu Leu Leu Leu Ile Ala Leu Ser Leu Ala Leu
1               5                   10                  15

Val Thr Asn Ser Glu Phe Gln Val Gln Leu Val Gln Ser Gly Ala Glu
            20                  25                  30

Val Lys Lys Pro Gly Ala Ser Val Lys Val Ser Cys Lys Ala Ser Gly
        35                  40                  45

Tyr Thr Phe Thr His Tyr Tyr Ile Tyr Trp Val Arg Gln Ala Pro Gly
    50                  55                  60

Gln Gly Leu Glu Trp Met Gly Gly Val Asn Pro Ser Asn Gly Gly Thr
65                  70                  75                  80

His Phe Asn Glu Lys Phe Lys Ser Arg Val Thr Met Thr Arg Asp Thr
                85                  90                  95

Ser Ile Ser Thr Ala Tyr Met Glu Leu Ser Arg Leu Arg Ser Asp Asp
            100                 105                 110

Thr Ala Val Tyr Tyr Cys Ala Arg Ser Glu Tyr Asp Tyr Gly Leu Gly
        115                 120                 125

Phe Ala Tyr Trp Gly Gln Gly Thr Leu Val Thr Val Ser Ser Gly Gly
    130                 135                 140

Gly Gly Ser Gly Gly Gly Gly Ser Gly Gly Gly Gly Ser Asp Ile Val
145                 150                 155                 160

Met Thr Gln Ser Pro Asp Ser Leu Ala Val Ser Leu Gly Glu Arg Ala
                165                 170                 175

Thr Ile Asn Cys Lys Ser Ser Gln Ser Leu Leu Asn Ser Arg Thr Pro
            180                 185                 190

Lys Asn Tyr Leu Ala Trp Tyr Gln Gln Lys Pro Gly Gln Pro Pro Lys
        195                 200                 205

Leu Leu Ile Tyr Trp Ala Ser Thr Arg Lys Ser Gly Val Pro Asp Arg
    210                 215                 220

Phe Ser Gly Ser Gly Ser Gly Thr Asp Phe Thr Leu Thr Ile Ser Ser
225                 230                 235                 240

Leu Gln Ala Glu Asp Val Ala Val Tyr Tyr Cys Lys Gln Ser Tyr Asn
                245                 250                 255

Leu Leu Thr Phe Gly Gly Gly Thr Lys Val Glu Ile Lys Ala Ala Ala
            260                 265                 270

Leu Glu Lys Glu Ala Leu Lys Lys Ile Ile Glu Asp Gln Gln Glu Ala
        275                 280                 285

Leu Asn Lys Trp Ala Ala Ala Gly Pro Gly Gly Gly Gly Ser Thr Thr
    290                 295                 300

Thr Pro Ala Pro Arg Pro Pro Thr Pro Ala Pro Thr Ile Ala Ser Gln
305                 310                 315                 320

Pro Leu Ser Leu Arg Pro Glu Ala Cys Arg Pro Ala Ala Gly Gly Ala
                325                 330                 335

Val His Thr Arg Gly Leu Asp Phe Ala Cys Asp Ile Tyr Ile Trp Ala
            340                 345                 350

Pro Leu Ala Gly Thr Cys Gly Val Leu Leu Leu Ser Leu Val Ile Thr
        355                 360                 365

Leu Tyr Cys Gly Gly Gly Gly Ser Lys Arg Gly Arg Lys Lys Leu Leu
    370                 375                 380

Tyr Ile Phe Lys Gln Pro Phe Met Arg Pro Val Gln Thr Thr Gln Glu
385                 390                 395                 400

Glu Asp Gly Cys Ser Cys Arg Phe Pro Glu Glu Glu Glu Gly Gly Cys
                405                 410                 415

Glu Leu Gly Gly Gly Gly Ser Phe Ser Arg Ser Ala Asp Ala Pro Ala
            420                 425                 430

Tyr Lys Gln Gly Gln Asn Gln Leu Tyr Asn Glu Leu Asn Leu Gly Arg
        435                 440                 445

Arg Glu Glu Tyr Asp Val Leu Asp Lys Arg Arg Gly Arg Asp Pro Glu
    450                 455                 460

Met Gly Gly Lys Pro Arg Arg Lys Asn Pro Gln Glu Gly Leu Tyr Asn
465                 470                 475                 480

Glu Leu Gln Lys Asp Lys Met Ala Glu Ala Tyr Ser Glu Ile Gly Met
                485                 490                 495

Lys Gly Glu Arg Arg Arg Gly Lys Gly His Asp Gly Leu Tyr Gln Gly
            500                 505                 510

Leu Ser Thr Ala Thr Lys Asp Thr Tyr Asp Ala Leu His Met Gln Ala
        515                 520                 525

Leu Pro Pro Arg
    530

<210>  43
<211>  615
<212>  PRT
<213>  Artificial Sequence

<220>
<223>  humanized homo sapiens/mus musculus chimera

<400>  43

Met Arg Arg Met Gln Leu Leu Leu Leu Ile Ala Leu Ser Leu Ala Leu
1               5                   10                  15

Val Thr Asn Ser Glu Phe Glu Val Gln Leu Val Glu Ser Gly Gly Gly
            20                  25                  30

Leu Val Gln Pro Gly Gly Ser Leu Arg Leu Ser Cys Ala Ala Ser Gly
        35                  40                  45

Phe Thr Phe Ser Asp Phe Trp Met Asn Trp Val Arg Gln Ala Pro Gly
    50                  55                  60

Lys Gly Leu Glu Trp Val Gly Gln Ile Arg Asn Lys Pro Asn Asn Tyr
65                  70                  75                  80

Glu Thr Tyr Tyr Ser Asp Ser Leu Lys Gly Arg Phe Thr Ile Ser Arg
                85                  90                  95

Asp Asp Ser Lys Ser Ile Thr Tyr Leu Gln Met Asn Ser Leu Arg Ala
            100                 105                 110

Glu Asp Thr Ala Val Tyr Tyr Cys Thr Leu Gly Asn Ser Trp Phe Ala
        115                 120                 125

Tyr Trp Gly Gln Gly Thr Leu Val Thr Val Ser Ser Gly Gly Gly Gly
    130                 135                 140

Ser Gly Gly Gly Gly Ser Gly Gly Gly Gly Ser Asp Ile Val Met Thr
145                 150                 155                 160

Gln Ser Pro Asp Ser Leu Ala Val Ser Leu Gly Glu Arg Ala Thr Ile
                165                 170                 175

Asn Cys Arg Ser Ser Gln Ser Leu Leu Asp Ser Arg Thr Lys Lys Asn
            180                 185                 190

Tyr Leu Ala Trp Tyr Gln Gln Lys Pro Gly Gln Pro Pro Lys Leu Leu
        195                 200                 205

Ile Tyr Trp Ala Ser Thr Arg Glu Ser Gly Val Pro Asp Arg Phe Ser
    210                 215                 220

Gly Ser Gly Ser Gly Thr Asp Phe Thr Leu Thr Ile Ser Ser Leu Gln
225                 230                 235                 240

Ala Glu Asp Val Ala Val Tyr Tyr Cys Lys Gln Ser Tyr Asn Leu Pro
                245                 250                 255

Thr Phe Gly Gly Gly Thr Lys Val Glu Ile Lys Ala Ala Ala Lys Pro
            260                 265                 270

Leu Pro Glu Val Thr Asp Glu Tyr Ala Ala Ala Gly Pro Gly Gly Gly
        275                 280                 285

Gly Ser Lys Ile Leu Val Lys Gln Ser Pro Met Leu Val Ala Tyr Asp
    290                 295                 300

Asn Ala Val Asn Leu Ser Cys Lys Tyr Ser Tyr Asn Leu Phe Ser Arg
305                 310                 315                 320

Glu Phe Arg Ala Ser Leu His Lys Gly Leu Asp Ser Ala Val Glu Val
                325                 330                 335

Cys Val Val Tyr Gly Asn Tyr Ser Gln Gln Leu Gln Val Tyr Ser Lys
            340                 345                 350

Thr Gly Phe Asn Cys Asp Gly Lys Leu Gly Asn Glu Ser Val Thr Phe
        355                 360                 365

Tyr Leu Gln Asn Leu Tyr Val Asn Gln Thr Asp Ile Tyr Phe Cys Lys
    370                 375                 380

Ile Glu Val Met Ala Pro Pro Pro Ala Leu Asp Asn Glu Lys Ser Asn
385                 390                 395                 400

Gly Thr Ile Ile His Val Lys Gly Lys His Leu Cys Pro Ser Pro Leu
                405                 410                 415

Phe Pro Gly Pro Ser Lys Pro Phe Trp Val Leu Val Val Val Gly Gly
            420                 425                 430

Val Leu Ala Cys Tyr Ser Leu Leu Val Thr Val Ala Phe Ile Ile Phe
        435                 440                 445

Trp Val Arg Ser Lys Arg Ser Arg Gly Gly His Ser Asp Tyr Met Asn
    450                 455                 460

Met Thr Pro Arg Arg Pro Gly Pro Thr Arg Lys His Tyr Gln Pro Tyr
465                 470                 475                 480

Ala Pro Pro Arg Asp Phe Ala Ala Tyr Arg Ser Gly Gly Gly Gly Ser
                485                 490                 495

Gly Gly Gly Ala Ser Gly Gly Gly Gly Ser Phe Ser Arg Ser Ala Asp
            500                 505                 510

Ala Pro Ala Tyr Gln Gln Gly Gln Asn Gln Leu Tyr Asn Glu Leu Asn
        515                 520                 525

Leu Gly Arg Arg Glu Glu Tyr Asp Val Leu Asp Lys Arg Arg Gly Arg
    530                 535                 540

Asp Pro Glu Met Gly Gly Lys Pro Arg Arg Lys Asn Pro Gln Glu Gly
545                 550                 555                 560

Leu Tyr Asn Glu Leu Gln Lys Asp Lys Met Ala Glu Ala Tyr Ser Glu
                565                 570                 575

Ile Gly Met Lys Gly Glu Arg Arg Arg Gly Lys Gly His Asp Gly Leu
            580                 585                 590

Tyr Gln Gly Leu Ser Thr Ala Thr Lys Asp Thr Tyr Gly Ala Leu His
        595                 600                 605

Met Gln Ala Leu Pro Pro Arg
    610                 615

<210>  44
<211>  520
<212>  PRT
<213>  Artificial Sequence

<220>
<223>  humanized homo sapiens/mus musculus chimera

<400>  44

Met Arg Arg Met Gln Leu Leu Leu Leu Ile Ala Leu Ser Leu Ala Leu
1               5                   10                  15

Val Thr Asn Ser Glu Phe Glu Val Gln Leu Val Glu Ser Gly Gly Gly
            20                  25                  30

Leu Val Gln Pro Gly Gly Ser Leu Arg Leu Ser Cys Ala Ala Ser Gly
        35                  40                  45

Phe Thr Phe Ser Asp Phe Trp Met Asn Trp Val Arg Gln Ala Pro Gly
    50                  55                  60

Lys Gly Leu Glu Trp Val Gly Gln Ile Arg Asn Lys Pro Asn Asn Tyr
65                  70                  75                  80

Glu Thr Tyr Tyr Ser Asp Ser Leu Lys Gly Arg Phe Thr Ile Ser Arg
                85                  90                  95

Asp Asp Ser Lys Ser Ile Thr Tyr Leu Gln Met Asn Ser Leu Arg Ala
            100                 105                 110

Glu Asp Thr Ala Val Tyr Tyr Cys Thr Leu Gly Asn Ser Trp Phe Ala
        115                 120                 125

Tyr Trp Gly Gln Gly Thr Leu Val Thr Val Ser Ser Gly Gly Gly Gly
    130                 135                 140

Ser Gly Gly Gly Gly Ser Gly Gly Gly Gly Ser Asp Ile Val Met Thr
145                 150                 155                 160

Gln Ser Pro Asp Ser Leu Ala Val Ser Leu Gly Glu Arg Ala Thr Ile
                165                 170                 175

Asn Cys Arg Ser Ser Gln Ser Leu Leu Asp Ser Arg Thr Lys Lys Asn
            180                 185                 190

Tyr Leu Ala Trp Tyr Gln Gln Lys Pro Gly Gln Pro Pro Lys Leu Leu
        195                 200                 205

Ile Tyr Trp Ala Ser Thr Arg Glu Ser Gly Val Pro Asp Arg Phe Ser
    210                 215                 220

Gly Ser Gly Ser Gly Thr Asp Phe Thr Leu Thr Ile Ser Ser Leu Gln
225                 230                 235                 240

Ala Glu Asp Val Ala Val Tyr Tyr Cys Lys Gln Ser Tyr Asn Leu Pro
                245                 250                 255

Thr Phe Gly Gly Gly Thr Lys Val Glu Ile Lys Ala Ala Ala Lys Pro
            260                 265                 270

Leu Pro Glu Val Thr Asp Glu Tyr Ala Ala Ala Gly Pro Gly Gly Gly
        275                 280                 285

Gly Ser Thr Thr Thr Pro Ala Pro Arg Pro Pro Thr Pro Ala Pro Thr
    290                 295                 300

Ile Ala Ser Gln Pro Leu Ser Leu Arg Pro Glu Ala Cys Arg Pro Ala
305                 310                 315                 320

Ala Gly Gly Ala Val His Thr Arg Gly Leu Asp Phe Ala Cys Asp Ile
                325                 330                 335

Tyr Ile Trp Ala Pro Leu Ala Gly Thr Cys Gly Val Leu Leu Leu Ser
            340                 345                 350

Leu Val Ile Thr Leu Tyr Cys Gly Gly Gly Gly Ser Lys Arg Gly Arg
        355                 360                 365

Lys Lys Leu Leu Tyr Ile Phe Lys Gln Pro Phe Met Arg Pro Val Gln
    370                 375                 380

Thr Thr Gln Glu Glu Asp Gly Cys Ser Cys Arg Phe Pro Glu Glu Glu
385                 390                 395                 400

Glu Gly Gly Cys Glu Leu Gly Gly Gly Gly Ser Phe Ser Arg Ser Ala
                405                 410                 415

Asp Ala Pro Ala Tyr Lys Gln Gly Gln Asn Gln Leu Tyr Asn Glu Leu
            420                 425                 430

Asn Leu Gly Arg Arg Glu Glu Tyr Asp Val Leu Asp Lys Arg Arg Gly
        435                 440                 445

Arg Asp Pro Glu Met Gly Gly Lys Pro Arg Arg Lys Asn Pro Gln Glu
    450                 455                 460

Gly Leu Tyr Asn Glu Leu Gln Lys Asp Lys Met Ala Glu Ala Tyr Ser
465                 470                 475                 480

Glu Ile Gly Met Lys Gly Glu Arg Arg Arg Gly Lys Gly His Asp Gly
                485                 490                 495

Leu Tyr Gln Gly Leu Ser Thr Ala Thr Lys Asp Thr Tyr Asp Ala Leu
            500                 505                 510

His Met Gln Ala Leu Pro Pro Arg
        515                 520

<210>  45
<211>  367
<212>  PRT
<213>  Artificial Sequence

<220>
<223>  humanized homo sapiens/mus musculus chimera

<400>  45

Met Arg Arg Met Gln Leu Leu Leu Leu Ile Ala Leu Ser Leu Ala Leu
1               5                   10                  15

Val Thr Asn Ser Glu Phe Lys Pro Leu Pro Glu Val Thr Asp Glu Tyr
            20                  25                  30

Ala Ala Ala Gly Pro Gly Gly Gly Gly Ser Lys Ile Leu Val Lys Gln
        35                  40                  45

Ser Pro Met Leu Val Ala Tyr Asp Asn Ala Val Asn Leu Ser Cys Lys
    50                  55                  60

Tyr Ser Tyr Asn Leu Phe Ser Arg Glu Phe Arg Ala Ser Leu His Lys
65                  70                  75                  80

Gly Leu Asp Ser Ala Val Glu Val Cys Val Val Tyr Gly Asn Tyr Ser
                85                  90                  95

Gln Gln Leu Gln Val Tyr Ser Lys Thr Gly Phe Asn Cys Asp Gly Lys
            100                 105                 110

Leu Gly Asn Glu Ser Val Thr Phe Tyr Leu Gln Asn Leu Tyr Val Asn
        115                 120                 125

Gln Thr Asp Ile Tyr Phe Cys Lys Ile Glu Val Met Ala Pro Pro Pro
    130                 135                 140

Ala Leu Asp Asn Glu Lys Ser Asn Gly Thr Ile Ile His Val Lys Gly
145                 150                 155                 160

Lys His Leu Cys Pro Ser Pro Leu Phe Pro Gly Pro Ser Lys Pro Phe
                165                 170                 175

Trp Val Leu Val Val Val Gly Gly Val Leu Ala Cys Tyr Ser Leu Leu
            180                 185                 190

Val Thr Val Ala Phe Ile Ile Phe Trp Val Arg Ser Lys Arg Ser Arg
        195                 200                 205

Gly Gly His Ser Asp Tyr Met Asn Met Thr Pro Arg Arg Pro Gly Pro
    210                 215                 220

Thr Arg Lys His Tyr Gln Pro Tyr Ala Pro Pro Arg Asp Phe Ala Ala
225                 230                 235                 240

Tyr Arg Ser Gly Gly Gly Gly Ser Gly Gly Gly Ala Ser Gly Gly Gly
                245                 250                 255

Gly Ser Phe Ser Arg Ser Ala Asp Ala Pro Ala Tyr Gln Gln Gly Gln
            260                 265                 270

Asn Gln Leu Tyr Asn Glu Leu Asn Leu Gly Arg Arg Glu Glu Tyr Asp
        275                 280                 285

Val Leu Asp Lys Arg Arg Gly Arg Asp Pro Glu Met Gly Gly Lys Pro
    290                 295                 300

Arg Arg Lys Asn Pro Gln Glu Gly Leu Tyr Asn Glu Leu Gln Lys Asp
305                 310                 315                 320

Lys Met Ala Glu Ala Tyr Ser Glu Ile Gly Met Lys Gly Glu Arg Arg
                325                 330                 335

Arg Gly Lys Gly His Asp Gly Leu Tyr Gln Gly Leu Ser Thr Ala Thr
            340                 345                 350

Lys Asp Thr Tyr Gly Ala Leu His Met Gln Ala Leu Pro Pro Arg
        355                 360                 365

<210>  46
<211>  379
<212>  PRT
<213>  Artificial Sequence

<220>
<223>  humanized homo sapiens/mus musculus chimera

<400>  46

Met Arg Arg Met Gln Leu Leu Leu Leu Ile Ala Leu Ser Leu Ala Leu
1               5                   10                  15

Val Thr Asn Ser Glu Phe Lys Pro Leu Pro Glu Val Thr Asp Glu Tyr
            20                  25                  30

Ala Ala Ala Gly Pro Gly Gly Gly Gly Ser Lys Ile Leu Val Lys Gln
        35                  40                  45

Ser Pro Met Leu Val Ala Tyr Asp Asn Ala Val Asn Leu Ser Cys Lys
    50                  55                  60

Tyr Ser Tyr Asn Leu Phe Ser Arg Glu Phe Arg Ala Ser Leu His Lys
65                  70                  75                  80

Gly Leu Asp Ser Ala Val Glu Val Cys Val Val Tyr Gly Asn Tyr Ser
                85                  90                  95

Gln Gln Leu Gln Val Tyr Ser Lys Thr Gly Phe Asn Cys Asp Gly Lys
            100                 105                 110

Leu Gly Asn Glu Ser Val Thr Phe Tyr Leu Gln Asn Leu Tyr Val Asn
        115                 120                 125

Gln Thr Asp Ile Tyr Phe Cys Lys Ile Glu Val Met Ala Pro Pro Pro
    130                 135                 140

Ala Leu Asp Asn Glu Lys Ser Asn Gly Thr Ile Ile His Val Lys Gly
145                 150                 155                 160

Lys His Leu Cys Pro Ser Pro Leu Phe Pro Gly Pro Ser Lys Pro Phe
                165                 170                 175

Trp Val Leu Val Val Val Gly Gly Val Leu Ala Cys Tyr Ser Leu Leu
            180                 185                 190

Val Thr Val Ala Phe Ile Ile Phe Trp Val Arg Ser Lys Arg Ser Arg
        195                 200                 205

Gly Gly Gly Gly Ser Lys Arg Gly Arg Lys Lys Leu Leu Tyr Ile Phe
    210                 215                 220

Lys Gln Pro Phe Met Arg Pro Val Gln Thr Thr Gln Glu Glu Asp Gly
225                 230                 235                 240

Cys Ser Cys Arg Phe Pro Glu Glu Glu Glu Gly Gly Cys Glu Leu Gly
                245                 250                 255

Gly Gly Gly Ser Gly Gly Gly Ala Ser Gly Gly Gly Gly Ser Phe Ser
            260                 265                 270

Arg Ser Ala Asp Ala Pro Ala Tyr Gln Gln Gly Gln Asn Gln Leu Tyr
        275                 280                 285

Asn Glu Leu Asn Leu Gly Arg Arg Glu Glu Tyr Asp Val Leu Asp Lys
    290                 295                 300

Arg Arg Gly Arg Asp Pro Glu Met Gly Gly Lys Pro Arg Arg Lys Asn
305                 310                 315                 320

Pro Gln Glu Gly Leu Tyr Asn Glu Leu Gln Lys Asp Lys Met Ala Glu
                325                 330                 335

Ala Tyr Ser Glu Ile Gly Met Lys Gly Glu Arg Arg Arg Gly Lys Gly
            340                 345                 350

His Asp Gly Leu Tyr Gln Gly Leu Ser Thr Ala Thr Lys Asp Thr Tyr
        355                 360                 365

Gly Ala Leu His Met Gln Ala Leu Pro Pro Arg
    370                 375

<210>  47
<211>  375
<212>  PRT
<213>  Artificial Sequence

<220>

<223>  humanized homo sapiens/mus musculus chimera

<400>  47

Met Arg Arg Met Gln Leu Leu Leu Leu Ile Ala Leu Ser Leu Ala Leu
1               5                   10                  15

Val Thr Asn Ser Glu Phe Glu Lys Glu Ala Leu Lys Lys Ile Ile Glu
            20                  25                  30

Asp Gln Gln Glu Ser Leu Asn Lys Ala Ala Ala Gly Pro Gly Gly Gly
        35                  40                  45

Gly Ser Lys Ile Leu Val Lys Gln Ser Pro Met Leu Val Ala Tyr Asp
    50                  55                  60

Asn Ala Val Asn Leu Ser Cys Lys Tyr Ser Tyr Asn Leu Phe Ser Arg
65                  70                  75                  80

Glu Phe Arg Ala Ser Leu His Lys Gly Leu Asp Ser Ala Val Glu Val
                85                  90                  95

Cys Val Val Tyr Gly Asn Tyr Ser Gln Gln Leu Gln Val Tyr Ser Lys
            100                 105                 110

Thr Gly Phe Asn Cys Asp Gly Lys Leu Gly Asn Glu Ser Val Thr Phe
        115                 120                 125

Tyr Leu Gln Asn Leu Tyr Val Asn Gln Thr Asp Ile Tyr Phe Cys Lys
    130                 135                 140

Ile Glu Val Met Ala Pro Pro Pro Ala Leu Asp Asn Glu Lys Ser Asn
145                 150                 155                 160

Gly Thr Ile Ile His Val Lys Gly Lys His Leu Cys Pro Ser Pro Leu
                165                 170                 175

Phe Pro Gly Pro Ser Lys Pro Phe Trp Val Leu Val Val Val Gly Gly
            180                 185                 190

Val Leu Ala Cys Tyr Ser Leu Leu Val Thr Val Ala Phe Ile Ile Phe
        195                 200                 205

Trp Val Arg Ser Lys Arg Ser Arg Gly Gly His Ser Asp Tyr Met Asn
    210                 215                 220

Met Thr Pro Arg Arg Pro Gly Pro Thr Arg Lys His Tyr Gln Pro Tyr
225                 230                 235                 240

Ala Pro Pro Arg Asp Phe Ala Ala Tyr Arg Ser Gly Gly Gly Gly Ser
                245                 250                 255

Gly Gly Gly Ala Ser Gly Gly Gly Gly Ser Phe Ser Arg Ser Ala Asp
            260                 265                 270

Ala Pro Ala Tyr Gln Gln Gly Gln Asn Gln Leu Tyr Asn Glu Leu Asn
        275                 280                 285

Leu Gly Arg Arg Glu Glu Tyr Asp Val Leu Asp Lys Arg Arg Gly Arg
    290                 295                 300

Asp Pro Glu Met Gly Gly Lys Pro Arg Arg Lys Asn Pro Gln Glu Gly
305                 310                 315                 320

Leu Tyr Asn Glu Leu Gln Lys Asp Lys Met Ala Glu Ala Tyr Ser Glu
                325                 330                 335

Ile Gly Met Lys Gly Glu Arg Arg Arg Gly Lys Gly His Asp Gly Leu
            340                 345                 350

Tyr Gln Gly Leu Ser Thr Ala Thr Lys Asp Thr Tyr Gly Ala Leu His
        355                 360                 365

Met Gln Ala Leu Pro Pro Arg
    370                 375

<210>  48
<211>  387
<212>  PRT
<213>  Artificial Sequence

<220>
<223>  humanized homo sapiens/mus musculus chimera

<400>  48

Met Arg Arg Met Gln Leu Leu Leu Leu Ile Ala Leu Ser Leu Ala Leu
1               5                   10                  15

Val Thr Asn Ser Glu Phe Glu Lys Glu Ala Leu Lys Lys Ile Ile Glu
            20                  25                  30

Asp Gln Gln Glu Ser Leu Asn Lys Ala Ala Ala Gly Pro Gly Gly Gly
        35                  40                  45

Gly Ser Lys Ile Leu Val Lys Gln Ser Pro Met Leu Val Ala Tyr Asp
    50                  55                  60

Asn Ala Val Asn Leu Ser Cys Lys Tyr Ser Tyr Asn Leu Phe Ser Arg
65                  70                  75                  80

Glu Phe Arg Ala Ser Leu His Lys Gly Leu Asp Ser Ala Val Glu Val
                85                  90                  95

Cys Val Val Tyr Gly Asn Tyr Ser Gln Gln Leu Gln Val Tyr Ser Lys
            100                 105                 110

Thr Gly Phe Asn Cys Asp Gly Lys Leu Gly Asn Glu Ser Val Thr Phe
        115                 120                 125

Tyr Leu Gln Asn Leu Tyr Val Asn Gln Thr Asp Ile Tyr Phe Cys Lys
    130                 135                 140

Ile Glu Val Met Ala Pro Pro Pro Ala Leu Asp Asn Glu Lys Ser Asn
145                 150                 155                 160

Gly Thr Ile Ile His Val Lys Gly Lys His Leu Cys Pro Ser Pro Leu
                165                 170                 175

Phe Pro Gly Pro Ser Lys Pro Phe Trp Val Leu Val Val Val Gly Gly
            180                 185                 190

Val Leu Ala Cys Tyr Ser Leu Leu Val Thr Val Ala Phe Ile Ile Phe
        195                 200                 205

Trp Val Arg Ser Lys Arg Ser Arg Gly Gly Gly Gly Ser Lys Arg Gly
    210                 215                 220

Arg Lys Lys Leu Leu Tyr Ile Phe Lys Gln Pro Phe Met Arg Pro Val
225                 230                 235                 240

Gln Thr Thr Gln Glu Glu Asp Gly Cys Ser Cys Arg Phe Pro Glu Glu
                245                 250                 255

Glu Glu Gly Gly Cys Glu Leu Gly Gly Gly Gly Ser Gly Gly Gly Ala
            260                 265                 270

Ser Gly Gly Gly Gly Ser Phe Ser Arg Ser Ala Asp Ala Pro Ala Tyr
        275                 280                 285

Gln Gln Gly Gln Asn Gln Leu Tyr Asn Glu Leu Asn Leu Gly Arg Arg
    290                 295                 300

Glu Glu Tyr Asp Val Leu Asp Lys Arg Arg Gly Arg Asp Pro Glu Met
305                 310                 315                 320

Gly Gly Lys Pro Arg Arg Lys Asn Pro Gln Glu Gly Leu Tyr Asn Glu
                325                 330                 335

Leu Gln Lys Asp Lys Met Ala Glu Ala Tyr Ser Glu Ile Gly Met Lys
            340                 345                 350

Gly Glu Arg Arg Arg Gly Lys Gly His Asp Gly Leu Tyr Gln Gly Leu
        355                 360                 365

Ser Thr Ala Thr Lys Asp Thr Tyr Gly Ala Leu His Met Gln Ala Leu
    370                 375                 380

Pro Pro Arg
385

<210>  49
<211>  1881
<212>  DNA
<213>  Artificial Sequence

<220>
<223>  humanized homo sapiens/mus musculus chimera

<400>  49
atgcgccgca tgcagctgct gcttctgatc gctctgagcc tggctcttgt gaccaactct      60

gaattccagg tgcagctggt gcagagcgga gccgaggtga agaagcctgg agcctctgtg     120

aaggtgagct gcaaggcttc tggctacacc ttcacccact actacatcta ctgggtgaga     180

caggctcccg gacagggcct ggagtggatg ggaggcgtga accccagcaa cggaggcacc     240

cacttcaacg agaagttcaa gtctcgcgtg accatgaccc gcgacaccag catctctacc     300

gcttacatgg agctgagccg cctgcgctct gatgataccg ctgtgtacta ctgcgctcgc     360

agcgagtacg attacggact gggcttcgcc tactggggcc agggaaccct ggtgaccgtg     420

agctctggag gcggaggcag cggaggcggc ggatctggag gcggaggaag cgatatcgtg     480

atgacccagt ctcctgatag cctggctgtg agcctgggcg agagagctac catcaactgc     540

aagagcagcc agagcctgct gaactctcgc acccctaaga actaccttgc ttggtaccag     600

cagaagcctg gacagccccc taagctgctg atctactggg cttctacccg caagagcggc     660

gtgcccgaca gattctctgg cagcggaagc ggcaccgatt tcaccctgac catcagcagc     720

ctgcaggctg aggacgtggc cgtgtactac tgcaagcagt cttacaacct gctgaccttc     780

ggaggcggaa ccaaggtgga gatcaaggct gccgctctgg agaaggaggc cctgaagaag     840

atcatcgagg atcagcagga ggctctgaac aagtgggctg ccgctgggcc cggaggaggc     900

ggcagcaaga tcctggtcaa acagtcccct atgctggtcg cttacgacaa cgccgttaat     960

ctgagttgca aatatagtta caacctgttt agccgggaat ttcgcgcatc tctccacaag    1020

ggactggatt ctgcggttga ggtttgtgtg gtctatggca attatagcca gcaactgcaa 1080

gtgtacagca aaacaggctt taactgcgac gggaaactcg ggaacgaatc agtgaccttc 1140

tatctgcaga acctgtacgt taaccaaaca gatatttact tctgcaagat agaggtgatg 1200

gctccaccgc cagcactgga taacgagaag tccaatggaa ccatcattca cgtcaagggg 1260

aagcatctgt gtccttcccc gttgttccct gggccgagca aacccttttg ggtgcttgtg 1320

gtagttggcg gggtattggc ctgctattcc cttctcgtaa ctgtggcctt catcatcttc 1380

tgggtcagat ctaagaggtc taggggcggg catagcgact acatgaacat gacacccagg 1440

cggcctggcc ccactcgcaa acactaccag ccatacgcac caccaagaga ctttgccgca 1500

tatcggagtg gtggcggcgg gtcaggaggt ggagctagcg gtggaggagg ttccttctct 1560

aggtcagctg atgctcccgc ctatcagcaa ggtcagaacc agctctacaa tgagctgaat 1620

ctgggacgtc gggaggagta cgacgtgctg gataaacgaa gaggacgcga tcccgagatg 1680

ggtgggaagc ctaggcgcaa gaatccccag gaaggcctct acaatgaact gcagaaagac 1740

aagatggccg aagcctacag cgagattggc atgaaagggg agcgacggag aggaaaggga 1800

catgacgggt tgtatcaggg tctttccact gcgacaaagg atacctatgg ggctctgcac 1860

atgcaagcac tgccacctag a 1881

<210> 50
<211> 1596
<212> DNA
<213> Artificial Sequence

<220>
<223> humanized homo sapiens/mus musculus chimera

<400> 50
atgcgccgca tgcagctgct gcttctgatc gctctgagcc tggctcttgt gaccaactct 60

gaattccagg tgcagctggt gcagagcgga gccgaggtga agaagcctgg agcctctgtg 120

aaggtgagct gcaaggcttc tggctacacc ttcacccact actacatcta ctgggtgaga 180

caggctcccg gacagggcct ggagtggatg ggaggcgtga accccagcaa cggaggcacc 240

cacttcaacg agaagttcaa gtctcgcgtg accatgaccc gcgacaccag catctctacc 300

gcttacatgg agctgagccg cctgcgctct gatgataccg ctgtgtacta ctgcgctcgc 360

agcgagtacg attacggact gggcttcgcc tactggggcc agggaaccct ggtgaccgtg 420

agctctggag gcggaggcag cggaggcggc ggatctggag gcggaggaag cgatatcgtg 480

atgacccagt ctcctgatag cctggctgtg agcctgggcg agagagctac catcaactgc 540

aagagcagcc agagcctgct gaactctcgc acccctaaga actaccttgc ttggtaccag 600

cagaagcctg gacagccccc taagctgctg atctactggg cttctacccg caagagcggc 660

gtgcccgaca gattctctgg cagcggaagc ggcaccgatt tcaccctgac catcagcagc 720

ctgcaggctg aggacgtggc cgtgtactac tgcaagcagt cttacaacct gctgaccttc 780

ggaggcggaa ccaaggtgga gatcaaggct gccgctctgg agaaggaggc cctgaagaag 840

atcatcgagg atcagcagga ggctctgaac aagtgggctg ccgctgggcc cggaggaggc 900

gggtcaacca ctacccctgc tcccaggcct cctacacccg cgccaacgat tgccagtcaa 960

cctctgtccc tgaggcctga agcctgcaga ccagctgcag gcggtgctgt gcatacacgg 1020

ggcttggact ttgcctgcga tatctacatc tgggcacctc ttgccgggac ttgtggcgtt 1080

ctcctgcttt ctctggtcat taccctgtac tgtggaggcg gcggtagcaa acgcggacgg 1140

aagaaactgc tgtacatctt caagcagccc ttcatgcgcc ccgtgcagac aacacaggaa 1200

gaggacggtt gcagctgccg atttcccgaa gaggaggagg gaggctgtga attgggcgga 1260

ggaggctcct tctctcgcag tgcagatgcc ccagcgtaca agcaaggcca gaaccagctg 1320

tacaacgagc tcaatctggg gcgtagagag gagtatgacg tgctggacaa gaggcgagga 1380

agggaccccg agatgggtgg caaaccgcgg cgtaagaatc cgcaggaagg cctctacaac 1440

gagctgcaga aagacaagat ggccgaagcc tattccgaga tagggatgaa aggggaaaga 1500

cggcgcggta aagggcacga tgggctctat cagggtctga gcactgcaac caaggacacg 1560

tatgatgcct tgcacatgca agctcttcca ccaaga 1596

<210> 51
<211> 1845
<212> DNA
<213> Artificial Sequence

<220>
<223> humanized homo sapiens/mus musculus chimera

<400> 51
atgcgccgca tgcagctgct gcttctgatc gctctgagcc tggctcttgt gaccaactct 60

gaattcgagg tgcaactggt cgaaagtggc ggtggtttag ttcagcctgg tggaagtcta 120

cggcttagct gcgcagcatc cggtttcacc tttagcgact tttggatgaa ctgggttcgg 180

caggctccgg gcaaaggact ggagtgggtt gggcaaatcc gcaacaaacc gaataactac 240

gaaacttatt actcagatag cctgaagggt cgattcacca tcagcaggga tgattcaaag 300

tcaatcactt acctacagat gaactcatta agagcggagg atactgcggt gtattactgt 360

acactaggta actcctggtt cgcgtattgg ggacagggca cccttgtaac cgtctccagc 420

ggaggcggag gcagcggagg cggcggatct ggaggcggag gaagcgacat tgttatgacc 480

cagagcccgg actctctcgc tgttagtctt ggtgagcgag cgactattaa ctgccggagc 540

agtcagagtt tgttggactc tcggacgaaa aagaactacc tggcatggta ccagcagaag 600

ccgggccaac cacctaaatt actgatatat tgggcgtcga ctcgtgagtc aggggtaccg 660

gacaggtttt ctggaagcgg atcaggaaca gacttcactt tgacgatctc ttcgcttcaa 720

gccgaggacg ttgcggttta ttattgtaag caaagctata atctgccgac atttggtggc 780

ggcaccaagg ttgaaattaa ggctgccgct aagcctctgc ctgaagtgac cgatgagtat 840

gctgccgctg ggcccggagg aggcggcagc aagatcctgg tcaaacagtc ccctatgctg 900

gtcgcttacg acaacgccgt taatctgagt tgcaaatata gttacaacct gtttagccgg 960

gaatttcgcg catctctcca caagggactg gattctgcgg ttgaggtttg tgtggtctat 1020

ggcaattata gccagcaact gcaagtgtac agcaaaacag gctttaactg cgacgggaaa 1080

ctcgggaacg aatcagtgac cttctatctg cagaacctgt acgttaacca aacagatatt 1140

tacttctgca agatagaggt gatggctcca ccgccagcac tggataacga gaagtccaat 1200

ggaaccatca ttcacgtcaa ggggaagcat ctgtgtcctt ccccgttgtt ccctgggccg 1260

agcaaaccct tttgggtgct tgtggtagtt ggcggggtat tggcctgcta ttcccttctc 1320

gtaactgtgg ccttcatcat cttctgggtc agatctaaga ggtctagggg cgggcatagc 1380

gactacatga acatgacacc caggcggcct ggccccactc gcaaacacta ccagccatac 1440

gcaccaccaa gagactttgc cgcatatcgg agtggtggcg gcgggtcagg aggtggagct 1500

agcggtggag gaggttcctt ctctaggtca gctgatgctc ccgcctatca gcaaggtcag 1560

aaccagctct acaatgagct gaatctggga cgtcgggagg agtacgacgt gctggataaa 1620

cgaagaggac gcgatcccga gatgggtggg aagcctaggc gcaagaatcc ccaggaaggc 1680

ctctacaatg aactgcagaa agacaagatg gccgaagcct acagcgagat tggcatgaaa 1740

ggggagcgac ggagaggaaa gggacatgac gggttgtatc agggtctttc cactgcgaca 1800

aaggatacct atggggctct gcacatgcaa gcactgccac ctaga 1845

<210> 52
<211> 1563
<212> DNA
<213> Artificial Sequence

<220>
<223> humanized homo sapiens/mus musculus chimera

<400> 52
atgcgccgca tgcagctgct gcttctgatc gctctgagcc tggctcttgt gaccaactct 60

gaattcgagg tgcaactggt cgaaagtggc ggtggtttag ttcagcctgg tggaagtcta 120

cggcttagct gcgcagcatc cggtttcacc tttagcgact tttggatgaa ctgggttcgg 180

caggctccgg gcaaaggact ggagtgggtt gggcaaatcc gcaacaaacc gaataactac 240

gaaacttatt actcagatag cctgaagggt cgattcacca tcagcaggga tgattcaaag 300

tcaatcactt acctacagat gaactcatta agagcggagg atactgcggt gtattactgt 360

acactaggta actcctggtt cgcgtattgg ggacagggca cccttgtaac cgtctccagc 420

ggaggcggag gcagcggagg cggcggatct ggaggcggag gaagcgacat tgttatgacc 480

cagagcccgg actctctcgc tgttagtctt ggtgagcgag cgactattaa ctgccggagc 540

agtcagagtt tgttggactc tcggacgaaa aagaactacc tggcatggta ccagcagaag 600

ccgggccaac cacctaaatt actgatatat tgggcgtcga ctcgtgagtc aggggtaccg 660

gacaggtttt ctggaagcgg atcaggaaca gacttcactt tgacgatctc ttcgcttcaa 720

gccgaggacg ttgcggttta ttattgtaag caaagctata atctgccgac atttggtggc 780

ggcaccaagg ttgaaattaa ggctgccgct aagcctctgc ctgaagtgac cgatgagtat 840

gctgccgctg ggcccggagg aggcggcagc accactaccc ctgctcccag gcctcctaca 900

cccgcgccaa cgattgccag tcaacctctg tccctgaggc ctgaagcctg cagaccagct 960

gcaggcggtg ctgtgcatac acggggcttg gactttgcct gcgatatcta catctgggca 1020

cctcttgccg ggacttgtgg cgttctcctg ctttctctgg tcattaccct gtactgtgga 1080

ggcggcggta gcaaacgcgg acggaagaaa ctgctgtaca tcttcaagca gcccttcatg 1140

cgccccgtgc agacaacaca ggaagaggac ggttgcagct gccgatttcc cgaagaggag 1200

gagggaggct gtgaattggg cggaggaggc tccttctctc gcagtgcaga tgccccagcg 1260

tacaagcaag gccagaacca gctgtacaac gagctcaatc tggggcgtag agaggagtat 1320

gacgtgctgg acaagaggcg aggaagggac cccgagatgg gtggcaaacc gcggcgtaag 1380

aatccgcagg aaggcctcta caacgagctg cagaaagaca agatggccga agcctattcc 1440

gagataggga tgaaagggga aagacggcgc ggtaaaggcc acgatggcct ctatcagggt 1500

ctgagcactg caaccaagga cacgtatgat gccttgcaca tgcaagctct tccaccaaga 1560

taa 1563

<210> 53
<211> 1101
<212> DNA
<213> Artificial Sequence

<220>
<223> humanized homo sapiens/mus musculus chimera

<400> 53
atgcgccgca tgcagctgct gcttctgatc gctctgagcc tggctcttgt gaccaactct 60

gaattcaaac ccctacctga agtgactgat gagtatgctg ccgctgggcc cggaggaggc 120

ggcagcaaga tcctggtcaa acagtcccct atgctggtcg cttacgacaa cgccgttaat 180

ctgagttgca aatatagtta caacctgttt agccgggaat ttcgcgcatc tctccacaag 240

ggactggatt ctgcggttga ggtttgtgtg gtctatggca attatagcca gcaactgcaa 300

gtgtacagca aaacaggctt taactgcgac gggaaactcg ggaacgaatc agtgaccttc 360

tatctgcaga acctgtacgt taaccaaaca gatatttact tctgcaagat agaggtgatg 420

gctccaccgc cagcactgga taacgagaag tccaatggaa ccatcattca cgtcaagggg 480

aagcatctgt gtccttcccc gttgttccct gggccgagca aacccttttg ggtgcttgtg 540

gtagttggcg gggtattggc ctgctattcc cttctcgtaa ctgtggcctt catcatcttc 600

tgggtcagat ctaagaggtc taggggcggg catagcgact acatgaacat gacacccagg 660

cggcctggcc ccactcgcaa acactaccag ccatacgcac caccaagaga ctttgccgca 720

tatcggagtg gtggcggcgg gtcaggaggt ggagctagcg gtggaggagg ttccttctct 780

aggtcagctg atgctcccgc ctatcagcaa ggtcagaacc agctctacaa tgagctgaat 840

ctgggacgtc gggaggagta cgacgtgctg gataaacgaa gaggacgcga tcccgagatg 900

ggtgggaagc ctaggcgcaa gaatccccag gaaggcctct acaatgaact gcagaaagac 960

aagatggccg aagcctacag cgagattggc atgaaagggg agcgacggag aggaaaggga 1020

catgacgggt tgtatcaggg tctttccact gcgacaaagg atacctatgg ggctctgcac 1080

atgcaagcac tgccacctag a 1101

<210> 54
<211> 1137
<212> DNA
<213> Artificial Sequence

<220>
<223> humanized homo sapiens/mus musculus chimera

<400> 54
atgcgccgca tgcagctgct gcttctgatc gctctgagcc tggctcttgt gaccaactct 60

gaattcaaac ccctacctga agtgactgat gagtatgctg ccgctgggcc cggaggaggc 120

ggcagcaaga tcctggtcaa acagtcccct atgctggtcg cttacgacaa cgccgttaat 180

ctgagttgca aatatagtta caacctgttt agccgggaat ttcgcgcatc tctccacaag 240

ggactggatt ctgcggttga ggtttgtgtg gtctatggca attatagcca gcaactgcaa 300

gtgtacagca aaacaggctt taactgcgac gggaaactcg ggaacgaatc agtgaccttc 360

tatctgcaga acctgtacgt taaccaaaca gatatttact tctgcaagat agaggtgatg 420

gctccaccgc cagcactgga taacgagaag tccaatggaa ccatcattca cgtcaagggg 480

aagcatctgt gtccttcccc gttgttccct gggccgagca aacccttttg ggtgcttgtg 540

gtagttggcg gggtattggc ctgctattcc cttctcgtaa ctgtggcctt catcatcttc 600

tgggtcagat ctaagaggtc taggggcggg ggcgggtcaa aacgcggacg gaagaaactg 660

ctgtacatct tcaagcagcc cttcatgcgc cccgtgcaga caacacagga agaggacggt 720

tgcagctgcc gatttcccga agaggaggag ggaggctgtg aattgggtgg cggcgggtca 780

ggaggtggag ctagcggtgg aggaggttcc ttctctaggt cagctgatgc tcccgcctat 840

cagcaaggtc agaaccagct ctacaatgag ctgaatctgg gacgtcggga ggagtacgac 900

gtgctggata aacgaagagg acgcgatccc gagatgggtg ggaagcctag gcgcaagaat 960

ccccaggaag gcctctacaa tgaactgcag aaagacaaga tggccgaagc ctacagcgag 1020

attggcatga aaggggagcg acggagagga aagggacatg acgggttgta tcagggtctt 1080

tccactgcga caaaggatac ctatggggct ctgcacatgc aagcactgcc acctaga 1137

<210> 55
<211> 1125
<212> DNA
<213> Artificial Sequence

<220>
<223> humanized homo sapiens/mus musculus chimera

<400> 55
atgcgccgca tgcagctgct gcttctgatc gctctgagcc tggctcttgt gaccaactct 60

gaattcgaga aagaagcact gaagaaaata atagaagacc aacaagaatc cctaaacaaa 120

gctgccgctg ggcccggagg aggcggcagc aagatcctgg tcaaacagtc ccctatgctg 180

gtcgcttacg acaacgccgt taatctgagt tgcaaatata gttacaacct gtttagccgg 240

gaatttcgcg catctctcca caagggactg gattctgcgg ttgaggtttg tgtggtctat 300

ggcaattata gccagcaact gcaagtgtac agcaaaacag gctttaactg cgacgggaaa 360

ctcgggaacg aatcagtgac cttctatctg cagaacctgt acgttaacca aacagatatt 420

tacttctgca agatagaggt gatggctcca ccgccagcac tggataacga gaagtccaat 480

ggaaccatca ttcacgtcaa ggggaagcat ctgtgtcctt ccccgttgtt ccctgggccg 540

agcaaaccct tttgggtgct tgtggtagtt ggcggggtat tggcctgcta ttcccttctc 600

gtaactgtgg ccttcatcat cttctgggtc agatctaaga ggtctagggg cgggcatagc 660

gactacatga acatgacacc caggcggcct ggccccactc gcaaacacta ccagccatac 720

gcaccaccaa gagactttgc cgcatatcgg agtggtggcg gcgggtcagg aggtggagct 780

agcggtggag gaggttcctt ctctaggtca gctgatgctc ccgcctatca gcaaggtcag 840

aaccagctct acaatgagct gaatctggga cgtcgggagg agtacgacgt gctggataaa 900

cgaagaggac gcgatcccga gatgggtggg aagcctaggc gcaagaatcc ccaggaaggc 960

ctctacaatg aactgcagaa agacaagatg gccgaagcct acagcgagat tggcatgaaa 1020

ggggagcgac ggagaggaaa gggacatgac gggttgtatc agggtctttc cactgcgaca 1080

aaggatacct atggggctct gcacatgcaa gcactgccac ctaga 1125

<210> 56
<211> 1161

<212>  DNA
<213>  Artificial Sequence

<220>
<223>  humanized homo sapiens/mus musculus chimera

<400>  56
atgcgccgca tgcagctgct gcttctgatc gctctgagcc tggctcttgt gaccaactct      60

gaattcgaga aagaagcact gaagaaaata atagaagacc aacaagaatc cctaaacaaa     120

gctgccgctg ggcccggagg aggcggcagc aagatcctgg tcaaacagtc ccctatgctg     180

gtcgcttacg acaacgccgt taatctgagt tgcaaatata gttacaacct gtttagccgg     240

gaatttcgcg catctctcca caagggactg gattctgcgg ttgaggtttg tgtggtctat     300

ggcaattata gccagcaact gcaagtgtac agcaaaacag gctttaactg cgacgggaaa     360

ctcgggaacg aatcagtgac cttctatctg cagaacctgt acgttaacca aacagatatt     420

tacttctgca agatagaggt gatggctcca ccgccagcac tggataacga gaagtccaat     480

ggaaccatca ttcacgtcaa ggggaagcat ctgtgtcctt ccccgttgtt ccctgggccg     540

agcaaaccct tttgggtgct tgtggtagtt ggcggggtat tggcctgcta ttcccttctc     600

gtaactgtgg ccttcatcat cttctgggtc agatctaaga ggtctagggg cgggggcggg     660

tcaaaacgcg gacggaagaa actgctgtac atcttcaagc agcccttcat gcgccccgtg     720

cagacaacac aggaagagga cggttgcagc tgccgatttc ccgaagagga ggagggaggc     780

tgtgaattgg gtggcggcgg gtcaggaggt ggagctagcg gtggaggagg ttccttctct     840

aggtcagctg atgctcccgc ctatcagcaa ggtcagaacc agctctacaa tgagctgaat     900

ctgggacgtc gggaggagta cgacgtgctg gataaacgaa gaggacgcga tcccgagatg     960

ggtgggaagc ctaggcgcaa gaatccccag gaaggcctct acaatgaact gcagaaagac    1020

aagatggccg aagcctacag cgagattggc atgaaagggg agcgacggag aggaaaggga    1080

catgacgggt tgtatcaggg tctttccact gcgacaaagg atacctatgg ggctctgcac    1140

atgcaagcac tgccacctag a                                              1161


<210>  57
<211>  274
<212>  PRT
<213>  Artificial Sequence

<220>
<223>  Tagged humanized scFv

<400>  57

Glu Val Gln Leu Val Glu Ser Gly Gly Gly Leu Val Gln Pro Gly Gly
1               5                   10                  15


Ser Leu Arg Leu Ser Cys Ala Ala Ser Gly Phe Thr Phe Ser Asp Ser

20                  25                  30

Trp Ile His Trp Val Arg Gln Ala Pro Gly Lys Gly Leu Glu Trp Val
        35                  40                  45

Ala Trp Ile Ser Pro Tyr Gly Gly Ser Thr Tyr Tyr Ala Asp Ser Val
    50                  55                  60

Lys Gly Arg Phe Thr Ile Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ala Arg Arg His Trp Pro Gly Gly Phe Asp Tyr Trp Gly Gln Gly Thr
            100                 105                 110

Leu Val Thr Val Ser Ser Gly Gly Gly Gly Ser Gly Gly Gly Gly Ser
        115                 120                 125

Gly Gly Gly Gly Ser Gly Gly Gly Gly Ser Asp Ile Gln Met Thr Gln
    130                 135                 140

Ser Pro Ser Ser Leu Ser Ala Ser Val Gly Asp Arg Val Thr Ile Thr
145                 150                 155                 160

Cys Arg Ala Ser Gln Asp Val Ser Thr Ala Val Ala Trp Tyr Gln Gln
                165                 170                 175

Lys Pro Gly Lys Ala Pro Lys Leu Leu Ile Tyr Ser Ala Ser Phe Leu
            180                 185                 190

Tyr Ser Gly Val Pro Ser Arg Phe Ser Gly Ser Gly Ser Gly Thr Asp
        195                 200                 205

Phe Thr Leu Thr Ile Ser Ser Leu Gln Pro Glu Asp Phe Ala Thr Tyr
    210                 215                 220

Tyr Cys Gln Gln Tyr Leu Tyr His Pro Ala Thr Phe Gly Gln Gly Thr
225                 230                 235                 240

Lys Val Glu Ile Lys Ala Ala Ala Lys Pro Leu Pro Glu Val Thr Asp
                245                 250                 255

Glu Tyr Ala Arg Gly Gly Pro Gly Gly Gly Gly Ser His His His His
            260                 265                 270

His His

<210> 58
<211> 278
<212> PRT
<213> Artificial Sequence

<220>
<223> Tagged humanized scFv

<400> 58

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Arg Tyr
            20                  25                  30

Trp Met Ser Trp Val Arg Gln Ala Pro Gly Lys Gly Leu Glu Trp Val
        35                  40                  45

Ala Asn Ile Lys Gln Asp Gly Ser Glu Lys Tyr Tyr Val Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Arg Glu Gly Gly Trp Phe Gly Glu Leu Ala Phe Asp Tyr Trp Gly
            100                 105                 110

Gln Gly Thr Leu Val Thr Val Ser Ser Gly Gly Gly Gly Ser Gly Gly
        115                 120                 125

Gly Gly Ser Gly Gly Gly Gly Ser Gly Gly Gly Gly Ser Glu Ile Val
    130                 135                 140

Leu Thr Gln Ser Pro Gly Thr Leu Ser Leu Ser Pro Gly Glu Arg Ala
145                 150                 155                 160

Thr Leu Ser Cys Arg Ala Ser Gln Arg Val Ser Ser Ser Tyr Leu Ala
                165                 170                 175

Trp Tyr Gln Gln Lys Pro Gly Gln Ala Pro Arg Leu Leu Ile Tyr Asp
            180                 185                 190

Ala Ser Ser Arg Ala Thr Gly Ile Pro Asp Arg Phe Ser Gly Ser Gly

195                 200                 205

Ser Gly Thr Asp Phe Thr Leu Thr Ile Ser Arg Leu Glu Pro Glu Asp
    210                 215                 220

Phe Ala Val Tyr Tyr Cys Gln Gln Tyr Gly Ser Leu Pro Trp Thr Phe
225                 230                 235                 240

Gly Gln Gly Thr Lys Val Glu Ile Lys Ala Ala Ala Lys Pro Leu Pro
                245                 250                 255

Glu Val Thr Asp Glu Tyr Ala Arg Gly Gly Pro Gly Gly Gly Gly Ser
            260                 265                 270

His His His His His His
        275

<210>  59
<211>  302
<212>  PRT
<213>  Artificial Sequence

<220>
<223>  Tagged human sequences

<400>  59

Glu Phe Pro Gly Trp Phe Leu Asp Ser Pro Asp Arg Pro Trp Asn Pro
1               5                   10                  15

Pro Thr Phe Ser Pro Ala Leu Leu Val Val Thr Glu Gly Asp Asn Ala
            20                  25                  30

Thr Phe Thr Cys Ser Phe Ser Asn Thr Ser Glu Ser Phe Val Leu Asn
        35                  40                  45

Trp Tyr Arg Met Ser Pro Ser Asn Gln Thr Asp Lys Leu Ala Ala Phe
    50                  55                  60

Pro Glu Asp Arg Ser Gln Pro Gly Gln Asp Cys Arg Phe Arg Val Thr
65                  70                  75                  80

Gln Leu Pro Asn Gly Arg Asp Phe His Met Ser Val Val Arg Ala Arg
                85                  90                  95

Arg Asn Asp Ser Gly Thr Tyr Leu Cys Gly Ala Ile Ser Leu Ala Pro
            100                 105                 110

Lys Ala Gln Ile Lys Glu Ser Leu Arg Ala Glu Leu Arg Val Thr Glu
        115                 120                 125

Arg Arg Ala Glu Val Pro Thr Ala His Ala Arg Gly Lys Pro Leu Pro
    130                 135                 140

Glu Val Thr Asp Glu Tyr Ala Ala Ala Pro Gly Trp Phe Leu Asp Ser
145                 150                 155                 160

Pro Asp Arg Pro Trp Asn Pro Pro Thr Phe Ser Pro Ala Leu Leu Val
                165                 170                 175

Val Thr Glu Gly Asp Asn Ala Thr Phe Thr Cys Ser Phe Ser Asn Thr
            180                 185                 190

Ser Glu Ser Phe Val Leu Asn Trp Tyr Arg Met Ser Pro Ser Asn Gln
        195                 200                 205

Thr Asp Lys Leu Ala Ala Phe Pro Glu Asp Arg Ser Gln Pro Gly Gln
    210                 215                 220

Asp Cys Arg Phe Arg Val Thr Gln Leu Pro Asn Gly Arg Asp Phe His
225                 230                 235                 240

Met Ser Val Val Arg Ala Arg Arg Asn Asp Ser Gly Thr Tyr Leu Cys
                245                 250                 255

Gly Ala Ile Ser Leu Ala Pro Lys Ala Gln Ile Lys Glu Ser Leu Arg
            260                 265                 270

Ala Glu Leu Arg Val Thr Glu Arg Arg Ala Glu Val Pro Thr Ala His
        275                 280                 285

Ala Ala Ala Gly Gly Gly Gly Ser His His His His His His
    290                 295                 300

<210>  60
<211>  532
<212>  PRT
<213>  Artificial Sequence

<220>
<223>  humanized homo sapiens/mus musculus chimera

<400>  60

Met Arg Arg Met Gln Leu Leu Leu Leu Ile Ala Leu Ser Leu Ala Leu
1               5                   10                  15

Val Thr Asn Ser Glu Phe Gln Val Gln Leu Val Gln Ser Gly Ala Glu
            20                  25                  30

Val Lys Lys Pro Gly Ala Ser Val Lys Val Ser Cys Lys Ala Ser Gly
        35                  40                  45

Tyr Thr Phe Thr His Tyr Tyr Ile Tyr Trp Val Arg Gln Ala Pro Gly
    50                  55                  60

Gln Gly Leu Glu Trp Met Gly Gly Val Asn Pro Ser Asn Gly Gly Thr
65                  70                  75                  80

His Phe Asn Glu Lys Phe Lys Ser Arg Val Thr Met Thr Arg Asp Thr
                85                  90                  95

Ser Ile Ser Thr Ala Tyr Met Glu Leu Ser Arg Leu Arg Ser Asp Asp
            100                 105                 110

Thr Ala Val Tyr Tyr Cys Ala Arg Ser Glu Tyr Asp Tyr Gly Leu Gly
        115                 120                 125

Phe Ala Tyr Trp Gly Gln Gly Thr Leu Val Thr Val Ser Ser Gly Gly
    130                 135                 140

Gly Gly Ser Gly Gly Gly Gly Ser Gly Gly Gly Gly Ser Asp Ile Val
145                 150                 155                 160

Met Thr Gln Ser Pro Asp Ser Leu Ala Val Ser Leu Gly Glu Arg Ala
                165                 170                 175

Thr Ile Asn Cys Lys Ser Ser Gln Ser Leu Leu Asn Ser Arg Thr Pro
            180                 185                 190

Lys Asn Tyr Leu Ala Trp Tyr Gln Gln Lys Pro Gly Gln Pro Pro Lys
        195                 200                 205

Leu Leu Ile Tyr Trp Ala Ser Thr Arg Lys Ser Gly Val Pro Asp Arg
    210                 215                 220

Phe Ser Gly Ser Gly Ser Gly Thr Asp Phe Thr Leu Thr Ile Ser Ser
225                 230                 235                 240

Leu Gln Ala Glu Asp Val Ala Val Tyr Tyr Cys Lys Gln Ser Tyr Asn
                245                 250                 255

Leu Leu Thr Phe Gly Gly Gly Thr Lys Val Glu Ile Lys Ala Ala Ala
            260                 265                 270

Leu Glu Lys Glu Ala Leu Lys Lys Ile Ile Glu Asp Gln Gln Glu Ala
        275                 280                 285

Leu Asn Lys Trp Ala Ala Ala Gly Pro Gly Gly Gly Gly Ser Thr Thr
    290                 295                 300

Thr Pro Ala Pro Arg Pro Pro Thr Pro Ala Pro Thr Ile Ala Ser Gln
305                 310                 315                 320

Pro Leu Ser Leu Arg Pro Glu Ala Cys Arg Pro Ala Ala Gly Gly Ala
                325                 330                 335

Val His Thr Arg Gly Leu Asp Phe Ala Cys Asp Ile Tyr Ile Trp Ala
            340                 345                 350

Pro Leu Ala Gly Thr Cys Gly Val Leu Leu Leu Ser Leu Val Ile Thr
        355                 360                 365

Leu Tyr Cys Gly Gly Gly Gly Ser Lys Arg Gly Arg Lys Lys Leu Leu
    370                 375                 380

Tyr Ile Phe Lys Gln Pro Phe Met Arg Pro Val Gln Thr Thr Gln Glu
385                 390                 395                 400

Glu Asp Gly Cys Ser Cys Arg Phe Pro Glu Glu Glu Glu Gly Gly Cys
                405                 410                 415

Glu Leu Gly Gly Gly Gly Ser Phe Ser Arg Ser Ala Asp Ala Pro Ala
            420                 425                 430

Tyr Lys Gln Gly Gln Asn Gln Leu Tyr Asn Glu Leu Asn Leu Gly Arg
        435                 440                 445

Arg Glu Glu Tyr Asp Val Leu Asp Lys Arg Arg Gly Arg Asp Pro Glu
    450                 455                 460

Met Gly Gly Lys Pro Arg Arg Lys Asn Pro Gln Glu Gly Leu Tyr Asn
465                 470                 475                 480

Glu Leu Gln Lys Asp Lys Met Ala Glu Ala Tyr Ser Glu Ile Gly Met
                485                 490                 495

Lys Gly Glu Arg Arg Arg Gly Lys Gly His Asp Gly Leu Tyr Gln Gly
            500                 505                 510

Leu Ser Thr Ala Thr Lys Asp Thr Tyr Asp Ala Leu His Met Gln Ala
        515                 520                 525

Leu Pro Pro Arg

    530

<210>  61
<211>  450
<212>  PRT
<213>  Artificial Sequence

<220>
<223>  Humanized VH chain

<400>  61

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ile Met Met Trp Val Arg Gln Ala Pro Gly Lys Gly Leu Glu Trp Val
        35                  40                  45

Ser Ser Ile Tyr Pro Ser Gly Gly Ile Thr Phe Tyr Ala Asp Thr Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Ile Lys Leu Gly Thr Val Thr Thr Val Asp Tyr Trp Gly Gln
            100                 105                 110

Gly Thr Leu Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Lys Val Glu Pro Lys Ser Cys Asp
    210                 215                 220

Lys Thr His Thr Cys Pro Pro Cys Pro Ala Pro Glu Leu Leu Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Pro Ala Pro Ile Glu
                325                 330                 335

Lys Thr Ile Ser Lys Ala Lys Gly Gln Pro Arg Glu Pro Gln Val Tyr
            340                 345                 350

Thr Leu Pro Pro Ser Arg Asp Glu Leu Thr Lys Asn Gln Val Ser Leu
        355                 360                 365

Thr Cys Leu Val Lys Gly Phe Tyr Pro Ser Asp Ile Ala Val Glu Trp
    370                 375                 380

Glu Ser Asn Gly Gln Pro Glu Asn Asn Tyr Lys Thr Thr Pro Pro Val
385                 390                 395                 400

Leu Asp Ser Asp Gly Ser Phe Phe Leu Tyr Ser Lys Leu Thr Val Asp
                405                 410                 415

Lys Ser Arg Trp Gln Gln Gly Asn Val Phe Ser Cys Ser Val Met His
            420                 425                 430

Glu Ala Leu His Asn His Tyr Thr Gln Lys Ser Leu Ser Leu Ser Pro
        435                 440                 445

Gly Lys

    450

<210>  62
<211>  216
<212>  PRT
<213>  Artificial Sequence

<220>
<223>  Humanized VL chain

<400>  62

Gln Ser Ala Leu Thr Gln Pro Ala Ser Val Ser Gly Ser Pro Gly Gln
1               5                   10                  15

Ser Ile Thr Ile Ser Cys Thr Gly Thr Ser Ser Asp Val Gly Gly Tyr
            20                  25                  30

Asn Tyr Val Ser Trp Tyr Gln Gln His Pro Gly Lys Ala Pro Lys Leu
        35                  40                  45

Met Ile Tyr Asp Val Ser Asn Arg Pro Ser Gly Val Ser Asn Arg Phe
    50                  55                  60

Ser Gly Ser Lys Ser Gly Asn Thr Ala Ser Leu Thr Ile Ser Gly Leu
65                  70                  75                  80

Gln Ala Glu Asp Glu Ala Asp Tyr Tyr Cys Ser Ser Tyr Thr Ser Ser
                85                  90                  95

Ser Thr Arg Val Phe Gly Thr Gly Thr Lys Val Thr Val Leu Gly Gln
            100                 105                 110

Pro Lys Ala Asn Pro Thr Val Thr Leu Phe Pro Pro Ser Ser Glu Glu
        115                 120                 125

Leu Gln Ala Asn Lys Ala Thr Leu Val Cys Leu Ile Ser Asp Phe Tyr
    130                 135                 140

Pro Gly Ala Val Thr Val Ala Trp Lys Ala Asp Gly Ser Pro Val Lys
145                 150                 155                 160

Ala Gly Val Glu Thr Thr Lys Pro Ser Lys Gln Ser Asn Asn Lys Tyr
                165                 170                 175

Ala Ala Ser Ser Tyr Leu Ser Leu Thr Pro Glu Gln Trp Lys Ser His
            180                 185                 190

Arg Ser Tyr Ser Cys Gln Val Thr His Glu Gly Ser Thr Val Glu Lys
        195                 200                 205

Thr Val Ala Pro Thr Glu Cys Ser
    210                 215

<210>  63
<211>  123
<212>  PRT
<213>  Artificial Sequence

<220>
<223>  Humanized VH chain

<400>  63

Glu Val Gln Leu Val Glu Ser Gly Gly Gly Leu Val Gln Pro Gly Arg
1               5                   10                  15

Ser Leu Arg Leu Ser Cys Ala Ala Ser Gly Phe Thr Phe Asp Asp Tyr
            20                  25                  30

Ala Met His Trp Val Arg Gln Val Pro Gly Lys Gly Leu Glu Trp Val
        35                  40                  45

Ser Gly Ile Ser Trp Ile Arg Thr Gly Ile Gly Tyr Ala Asp Ser Val
    50                  55                  60

Lys Gly Arg Phe Thr Ile Phe Arg Asp Asn Ala Lys Asn Ser Leu Tyr
65                  70                  75                  80

Leu Gln Met Asn Ser Leu Arg Ala Glu Asp Thr Ala Leu Tyr Tyr Cys
                85                  90                  95

Ala Lys Asp Met Lys Gly Ser Gly Thr Tyr Gly Gly Trp Phe Asp Thr
            100                 105                 110

Trp Gly Gln Gly Thr Leu Val Thr Val Ser Ser
        115                 120


<210>  64
<211>  107
<212>  PRT
<213>  Artificial Sequence

<220>
<223>  Humanized VL chain

<400>  64

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Ser Ile Ser Ser Tyr

20                  25                  30

Leu Asn Trp Tyr Gln Gln Lys Pro Gly Lys Ala Pro Lys Leu Leu Ile
        35                  40                  45

Tyr Val Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Ser Tyr Ser Thr Pro Ile
                85                  90                  95

Thr Phe Gly Gln Gly Thr Arg Leu Glu Ile Lys
            100                 105